# EUROPEAN PATENT APPLICATION

(11) **EP 2 612 669 A2**
(43) Date of publication of application: **10.07.2013**
(21) Application number: 11822140.7
(22) Date of filing: 31.08.2011
(51) Int. Cl.: A61K 31/426, A61K 31/427, A61P 3/04, A61P 3/10

(54) **USE OF THE FETAL REPROGRAMMING OF A PPAR AGONIST**

(30) Priority: 31.08.2010 KR 20100085085
(71) Applicant: SNU R & DB Foundation, Gwanak-gu, Seoul 151-015 (KR)
(72) Inventor: KANG, Heonjoong, Seoul 137-060 (KR); HWANG, Hoo-Sang, Seoul 138-782 (KR); CHIN, Jungwook, Seoul 138-791 (KR)
(74) Representative: Zinnecker, Armin
(86) International application number: PCT/KR2011/006467
(87) International publication number: WO 2012/030165

(57) **Abstract**

The present invention relates to a novel use of a PPAR δ agonist, and more particularly, to a fetal reprogramming effect of a PPAR δ agonist. According to the present invention, a PPAR δ agonist adjusts calcium ion during embryo genesis and a early fetal development period to increase slow muscle fiber and to thus improve muscle endurance, thereby improving lipid and glucose metabolism and reprogramming the metabolism of the entire body, thus preventing/inhibiting the occurrence of metabolic diseases, such as obesity and diabetes in an adult body caused by a high-fat diet and a lack of exercise, and improving memory for an adult. In addition, fetal reprogramming using a PPAR δ agonist prevents/inhibits the occurrence of diabetes in a mouse model for diabetes. Therefore, the PPAR δ agonist may be used in a pharmaceutical composition for enhancing the endurance of a human and an animal by embryonic/fetal reprogramming, preventing/inhibiting metabolic diseases such as obesity, diabetes, arteriosclerosis and fatty liver, and enhancing memory. The PPAR δ agonist may also be used in a nutritional supplement for pregnant women, in food additives, in a functional food supplement or functional beverage composition, in pharmaceutical compositions for animals, in an endurance enhancer for animals, in dry milk and baby formula compositions, in an animal feed composition, etc.

## Description

### [Techmical Field]

The present invention relates to a fetal reprogramming effect of a PPAR δ agonist, and more particularly, to a new use of a PPAR δ agonist, which adjusts calcium ions during embryo genesis and a early fetal development period to increase slow muscle fibers and thus enhance muscle endurance, thereby improving lipid and glucose metabolism and reprogramming the metabolism of the entire body and thus preventing/inhibiting the occurrence of metabolic diseases, such as obesity and diabetes in an adult or adult body caused by high-fat diet and lack of exercise, and improving memory. According to the present invention, the PPAR δ agonist prevents/inhibits the occurrence of diabetes even in a mouse with congenital diabetes as well as controls fetal reprogramming in a normal mouse, and thus is effective in the prevention of metabolic diseases such as obesity, diabetes, and the like, and treatment of premature children through the fetal reprogramming in human beings and animals and also is effective in enhancing endurance and improving memory of human beings and animals.

### [Background Art]

Skeletal muscles may be divided into a slow muscle fiber and a fast muscle fiber based on the muscle contraction rate and metabolism characteristics (Schiaffino & Reggiani Physiol. Rev., 1996, 76, 371-423). The fast muscle fiber, which is Type II muscle fiber, uses glucose as an energy source, and is commonly present in the muscles needed for fast movement such as sprinting. On the other hand, the slow muscle fiber uses lipid as a main energy source, and are commonly present in the muscles needed for continuous movement. The slow muscle fiber has more mitochondria than the fast muscle fiber and thus appears redder than the fast muscle fiber, and has resistivity against fatigue due to lactic acid at the time of exercise and thus is appropriate in the exercise requiring endurance (Costill, DL. et al., J. Appl. Pysiol., 1976, 40, 149-154; Saltin B et al., Ann. N. Y. Acad. Sci., 1977, 301, 3-44). In addition, since the slow muscle fiber is greater than the fast muscle fiber in view of fatty acid oxidative capacity and insulin sensitivity, metabolic diseases such as obesity and diabetes were remarkably suppressed in spite of the intake of high-fat feedstuff (35% V/V lipid) (Wang, YX. et al., PlosBiol., 2004, 2, e294; Vihang A. Narkar et al, Cell, 2008, 134, 405-415).

It has been known that there are Calcineurin (Serrano, AL. et al., Proc. Natl. Acad. Sci. USA., 2001, 98, 13108-13113), Calmodulin-dependentkinase (Wu, H. et al., Science., 2002, 296, 349-352), PGC-1α (Lin, J. et al., Nature., 2002, 418, 797-801), AMPK (Vihang A. Narkar et al, Cell, 2008, 134: 405-415), Sirt1 and Adiponectin (Masato Iwabu et al., Nature, 2010, 464, 1313-1319), and PPAR δ (Wang, YX. et al., PlosBiol., 2004, 2, e294) in genes associated with fast-to-slow muscle fiber transformation. Of these, PPAR δ has been found to be a very important modulator in the fast-to-slow muscle fiber transformation through studies on overexpression or selective removal thereof. PPAR δ is a ligand-dependent transcription factor that is commonly expressed in the slow muscle fiber. It has been reported that muscle-specific PPAR δ transgenic mice showed a significant increase in generation of slow muscle fibers, and thus 67% and 92% increases in the running time and distance, respectively (Wang, YX. et al., PlosBiol., 2004, 2. e294). Whereas, it has been reported that mice of which PPAR δ gene was selectively removed showed a 62% decrease in the running time and also a 66% decrease in the running distance as compared with normal mice (Wang, YX. et al .,PlosBiol., 2004, 2. e294). Therefore, it may be seen that PPAR δ is an important gene in generating the slow muscle fiber and controlling exercise capacity.

However, it has been reported that study results using PPAR δ agonist were completely different from the anticipation from the existing studies. GW501516 is the most effective in PPAR δ agonists as reported in documents until now (William R. Oliver, Jr. et al., Proc. Natl. Acad. Sci. USA., 2001, 98, 5306-5311). It has been reported that experiment results using this material were different from those using the existing PPAR δ transgenic mice. In other words, administration of GW501516 alone to an adult did not show improvement in slow muscle fibers and endurance, but only the simultaneous combination of exercise and administration of GW501516 generated slow muscle fibers and enhanced endurance (Vihang A. Narkar et al, Cell, 2008, 134, 405-415). Accordingly, few cases have been known until now where the PPAR δ agonist alone enhances endurance.

Fetal programming is a theory where the environment to which the fetus is exposed during a gestation period determines health of a fetus and an adult, which was first discussed in Southampton University and Kings College, UK, and is currently used with the term "developmental programming" and "fetal developmental programming" More specifically, this means that the environment to which the fetus is exposed during a development period influences generation of organs and viscera of the fetus, which then influences the whole life (Mc-Millen and Robinson, Physiol Rev., 2005, 85, 571-633; Plagemann, Horm Res., 2006, 65, 8389; Taylor and Poston, Exp Physiol, 2007, 92, 287298). Epidemiological investigation results reported that an excessive supply or an insufficient supply of nutrient during a fetal programming period causes an increase in the occurrence of serious diseases such as obesity, cardiovascular disease, diabetes, hypertension, arteriosclerosis, and cancer, as an adult. For this reason, various studies have been conducted to prevent them, and a representative example therefor is taurine or the like. It has been known that the intake of taurine during the development period suppresses the occurrence of diabetes (Cherif et al., J. Endocrinol., 1998, 159, 341-348), and it has been known that arginine improves diabetes and cardiovascular disorder (Wu et al., J Nutr., 2004, 134, 2169-2172). In addition, it has been known that folic acid which pregnant women take a lot in order to suppress miscarriage is also effective in preventing the diabetes (Lillycrop et al., J Nutr., 2005, 135, 1382-1386). Very interestingly, it has been reported that administration of exedin-4, which is a GLP-1 agonist and has a diabetes treatment effect, during a development period of a rat, can decrease oxidative stress and thus prevent fetal diabetes (Stoffers DA et al., Diabetes. 2003, 52, 734-740; Raab EL et al., Am. J. Physiol. Regul. Integr. Comp. Physiol., 2009, 297, 1785-1794).

The present inventors validated a fetal reprogramming effect of a PPAR δ agonist, and then completed the present invention. The present invention confirmed the effect of PPAR δ by using a method of administrating an agonist to a mother during a pregnant period and a lactancy period (maternal treatment). As a result, the PPAR δ agonist alone adjusted calcium ions and thus increased generation of slow muscle fibers. Mouse pups subjected to fetal reprogramming with the PPAR δ agonist had significantly enhanced endurance even though they were not administered with the PPAR δ agonist or did not exercise after growing to adults. Further, the occurrence of metabolic diseases such as obesity and diabetes, caused by the intake of high-fat and lack of exercise, was significantly prevented and suppressed, and new effects of improving memory was validated. Moreover, it was validated that the PPAR δ agonist alone prevents/suppresses the occurrence of diabetes from birth to adulthood even in the case of db/db mice, which is a congenital diabetes disease model, as well as, normal mice.

### [Disclosure]

### [Technical Problem]

The present invention is directed to a fetal reprogramming effect of a PPAR δ agonist, and an object of the present invention is to increase activity of PPAR δ protein by using a compound during the development period of a human being and an animal and thus increase slow muscle fibers and enhance endurance, thereby fundamentally preventing or reducing the occurrence of metabolic diseases, and improving memory. In the present invention, the PPAR δ agonist alone was used without exercise, to increase slow muscle fibers of a human being or an animal and thus significantly enhance endurance; to reprogram the metabolism of the entire body and thus fundamentally prevent or suppress the occurrence of metabolic diseases; and to improve the memory, which are completely different from as reported by the existing inventions (WO 2009/086526, WO2008/083330). More progressive is that the fetal reprogramming effect of the PPAR δ is consistent with mice born with a congenital diabetes disease as well as normal mice.

In other words, an object of the present invention is to provide a composition for fetal reprogramming of a mammal, containing a peroxisome proliferator activated receptor δ (PPAR δ) agonist as an effective component.

Another object of the present invention is to provide a fetal reprogramming method of a mammal excluding a human being, including administering a PPAR δ agonist to the mammal except the human being.

Still another object of the present invention is to provide a composition for a food additive, a functional food supplement, or a functional beverage, for enhancing muscle endurance, preventing metabolic disease, or improving memory, of an object produced by a fetal reprogramming method of a mammal including a human being, containing a PPAR δ agonist.

Still another object of the present invention is to provide a composition for feedstuff for enhancing muscle endurance, preventing metabolic disease, or improving memory, of an object produced by a fetal reprogramming method of a mammal, containing a PPAR δ agonist as an additive.

Still another object of the present invention is to provide a composition for dry milk or baby food for enhancing muscle endurance, preventing metabolic disease, or improving memory, of an object produced by a fetal reprogramming method of a mammal, containing a PPAR δ agonist as an additive.

Still another object of the present invention is to provide an endurance enhancer of an object produced by a fetal reprogramming method of a mammal including a human being, containing a PPAR δ agonist as an effective component.

Still another object of the present invention is to provide an endurance enhancer of an object produced by a fetal reprogramming method of a mammal excluding a human being, containing a PPAR δ agonist as an effective component.

Another object of the present invention is to provide a noble PPAR δ agonist, or a hydrate, solvate, stereoisomer, or pharmaceutically acceptable salt thereof, and still another object of the present invention is to provide a composition for medicine for treating and preventing atherosclerosis or hyperlipidemia, treating and preventing hypercholesterolemia, treating and preventing fatty liver, treating and preventing diabetes, treating and preventing obesity, strengthening muscle, treating and preventing muscular diseases, enhancing endurance, improving memory, or treating and preventing dementia or Parkinson's disease, containing the noble PPAR δ agonist as an effective component.

Still another object of the present invention is to provide a composition for a functional food supplement, a functional beverage, a food additive, and a feedstuff for animal, containing the noble PPAR δ agonist as an effective component.

Still another object of the present invention is to provide a composition for functional cosmetics for preventing and improving obesity, preventing and improving fatty liver, enhance the muscle, and preventing and improving muscular diseases, and enhancing endurance, containing the noble PPAR δ agonist as an effective component.

### [Technical Solution]

Hereinafter, the present invention will be described in detail.

The present invention is directed to a fetal reprogramming effect of a PPAR δ agonist during a development period of a mammal, and the present invention is directed to effects of enhancing muscle endurance, preventing and suppressing metabolic diseases such as obesity, diabetes, atherosclerosis, and fatty liver, and improving memory, through fetal reprogramming of the PPAR δ agonist during development periods of all animals including a human being.

The present inventors used a method of administering medicine to a pregnant mother (maternal treatment) and a method of administering medicine to a lactating mother. However, the present invention is not limited to the two methods, and includes PPAR δ fetal reprogramming methods through other pathways except for the administration methods used in the present invention. The present invention includes, for example, a method of increasing expression and activity of PPAR δ in a fetus through a mother animal, and a method of directly increasing expression and activity of PPAR δ in a fetus by using a form of dry milk, baby food, and the like. In addition, the present invention includes an injection, a taker, a skin permeation ointment for a pregnant animal mother and a pregnant woman, containing agonists used in the present invention [CMDD 1111, 1112, 1226] as well as a PPAR δ agonist as an effective component, and fetal reprogramming method of PPAR δ using beverage and food containing a PPAR δ agonist.

According to the existing study result, the PPAR δ agonist was orally administered to the adult and then it was observed whether or not the muscle fiber was changed, but the generation of slow muscle fibers was not increased (Vihang A. Narkar et al, Cell, 2008, 134, 405-415). In the present invention, slow muscle fibers of a born fetus were significantly increased even though the PPAR δ agonist was administered to only a pregnant mother or a lactating mother. Therefore, it may be seen that the fetal reprogramming results by PPAR δ mainly results in an increase in slow muscle fibers and the most important acting time thereof is a myogenesis period.

The slow muscle fiber is characterized by oxidation of fatty acid, biosynthesis of mitochondria, and an increase in protein associated with slow myosin heavy chain and slow contraction. In the mice subjected to fetal reprogrammed by PPAR δ, all the gene groups were increasingly expressed. Further, it may be confirmed from the results of external shape comparison and tissue staining of the muscle that the slow muscle fibers were increasingly generated by the PPAR δ agonist. Taken together of the above two experiments, it may be seen that the fetal reprogramming by PPAR δ agonist can promote generation of the slow muscle.

Calcium has been known as an important regulator that promotes fast-to-slow muscle fiber transformation. According to results of a study by the present inventors, as the result of fetal reprogramming by the PPAR δ agonist, expression of genes such as RYR, ATP2A2, and PLN, which regulate concentration of cytosolic calcium, was remarkably increased in the mouse muscle whereby the retention time of cytosolic calcium ions was significantly increased. This increase in cytosolic calcium ions in the cell activated calcium signals and thus increased generation of slow muscle fibers.

In the present invention, mice with increased slow muscle fibers had 2.9 fold the exercise time as compared with control mice. In order to establish a mechanism thereof, a gene analysis (microarray) experiment using skeleton muscle tissues was conducted. As a result, it was confirmed that expression of genes such as RYR2, ATP2A2, PLN, and MYBPC3, which have been known to be commonly expressed in the heart muscle, was remarkably increased for an administered mice group. To sum the above results up, the fetal reprogramming by the PPAR δ agonist improved muscle functions as well as heart functions in the mice.

The largest meaning of the present invention is that the fetal reprogramming by the PPAR δ agonist compound can improve health and life quality of human beings or animals after growth. As for the mice subjected to fetal reprogramming by PPAR δ, even though the PPAR δ agonist compound was not administered or exercise was not made as adults, the generation of slow muscle fibers and the enhancement of endurance were remarkably increased when the mice became adults, and the occurrence of metabolic syndromes such as obesity and diabetes caused by intake of high-fat and lack of exercise was significantly suppressed.

Obesity is a direct cause of metabolic diseases such as hyperlipidemia, arteriosclerosis, and myocardial infarction as well as diabetes. Therefore, according to the present invention, mice having increased lipid metabolizing capacity through the fetal reprogramming by PPAR δ agonist can have an effect of preventing all metabolic syndromes including obesity.

The slow muscle fiber contains more mitochondria than the fast muscle fiber. The mitochondria are characterized by having maternal inheritance. Therefore, the mice having increased mitochondria through the fetal reprogramming by the PPAR δ agonist can influence mitochondria formation in the next generation, and in advance, enhance endurance and prevent the occurrence of metabolic syndromes of the next generation to which the PPAR δ agonist is never administered.

That is, the present invention provides a composition for fetal reprogramming of a mammal, containing a peroxisome proliferator activated receptor δ (PPAR δ) agonist as an effective component. Further, the present invention provides a fetal reprogramming method of a mammal excluding a human being, including administering a PPAR δ agonist to the mammal excluding the human being. Further, the present invention provides a composition for a food additive, a functional food supplement, or a functional beverage for enhancing muscle endurance, preventing metabolic diseases, or improving memory, of an object produced by a fetal reprogramming method of a mammal including a human being, containing a PPAR δ agonist. Further, the present invention provides a composition for feedstuff for enhancing muscle endurance, preventing metabolic disease, or improving memory, of an object produced by a fetal reprogramming method of a mammal, containing a PPAR δ agonist as an additive. Further, the present invention provides a composition for dry milk or baby food for enhancing muscle endurance, preventing metabolic disease, or improving memory, of an object produced by a fetal reprogramming method of a mammal, containing a PPAR δ agonist as an additive. Further, the present invention provides an endurance enhancer of an object produced by a fetal reprogramming method of a mammal including a human being, containing a PPAR δ agonist as an effective component. Further, the present invention provides an endurance enhancer of an object produced by a fetal reprogramming method of a mammal excluding a human being, containing a PPAR δ agonist as an effective component.

Examples of the PPAR δ agonist in the present invention are as follows. However, the present invention is not limited to the following examples, and include all the PPAR δ agonists [Examples of PPAR δ agonist: WO 2010/039789, WO 2010/028761, WO 2010/023572, WO 2010/009215, WO 2010/009212, WO 2010/009210, WO 2010/008299, WO 2009/155709, WO 2009/140342, WO 2009/136290, WO 2009/114173, WO 2009/098000, WO 2009/097999, WO 2009/097998, WO 2009/097997, WO 2009/097995, WO 2009/091550, WO 2009/078981, WO 2009/039944, WO 2009/039943, WO 2009/039942, WO 2009/027785, WO 2009/024287, WO 2009/016216, WO 2009/006483, WO 2008/144925, WO 2008/135141, WO 2008/122014, WO 2008/115574, WO 2008/104557, WO 2008/089892, WO 2008/085316, WO 2008/079293, WO 2008/070150, WO 2008/060476, WO 2008/058631, WO 2008/058630, WO 2008/058629, WO 2008/058628, WO 2008/052658, WO 2008/017381, WO 2008/000484, WO 2007/131622, WO 2007/131621, WO 2007/131620, WO 2007/131619, WO 2007/130075, WO 2007/121432, WO 2007/110216, WO 2007/110215, WO 2007/107869, WO 2007/105050, WO 2007/105049, WO 2007/089857, WO 2007/089667, WO 2007/087448, WO 2007/087204, WO 2007/059871, WO 2007/056496, WO 2007/056210, WO 2007/047432, WO 2007/033231, WO 2007/016538, WO 2006/137796, WO 2006/137795, WO 2006/124490, WO 2006/123185, WO 2006/123184, WO 2006/123183, WO 2006/123182, WO 2006/113919, WO 2006/111321, WO 2006/111261, WO 2006/104826, WO 2006/102067, WO 2006/098912, WO 2006/097220, WO 2006/092507, WO 2006/091506, WO 2006/087630, WO 2006/084176, WO 2006/083645, WO 2006/078605, WO 2006/078575, WO 2006/076681, WO 2006/056854, WO 2006/055187, WO/2006/041197, WO 2006/040002, WO 2006/034128, WO 2006/033004, WO 2006/033062, WO 2006/032987, WO 2006/032384, WO 2006/032023, WO 2006/031969, WO 2006/018174, WO 2006/017055, WO 2005/115983, WO 2005/113506, WO 2005/105754, WO 2005/105736, WO 2005/105726, WO 2005/103022, WO 2005/097784, WO 2005/092317, WO 2005/092316, WO 2005/091857, WO 2005/077925, WO 2005/066136, WO 2005/065683, WO 2005/063762, WO 2005/063761, WO 2005/060958, WO 2005/041959, WO 2005/037763, WO 2005/037199, WO 2005/030694, WO 2005/007628, WO 2005/007111, WO 2004/101752, WO 2004/096137, WO 2004/093799, WO 2004/092117, WO 2004/073606, WO 2004/067482, WO 2004/066929, WO 2004/063184, WO 2004/063166, WO 2004/063165, WO 2004/063155, WO 2004/033438, WO 2004/033416, WO 2004/018421, WO 2004/007430, WO 2003/094845, WO 2003/074495, WO 2003/072100, WO 2003/048108, WO 2002/102813, WO 2002/092590, WO 2001/070754, WO 2001/070753, WO 1998/049555, WO 1998/27974, WO 1997/28149, WO 1997/28115, WO 1997/27857, WO 1997/27847, WO 1997/28137, WO 1996/001317 etc.].

Some of the PPAR δ agonists are represented by Chemical Formula I below.

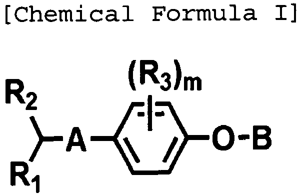

[In Chemical Formula I, A is oxygen (O), nitrogen (NH), sulfur (S), or selenium (Se); B is

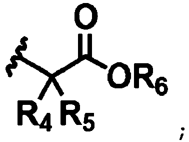

R₁ is selected from the following structures:

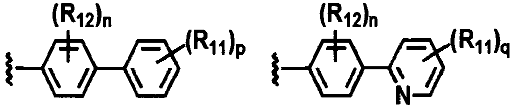

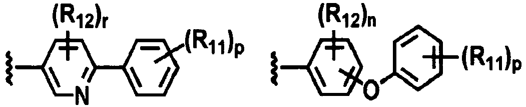

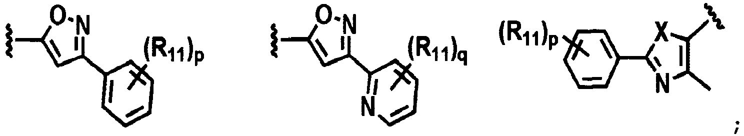

R₂ is selected from hydrogen, C₁-C₅ alkyl, 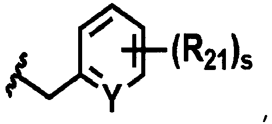 or the following structures:

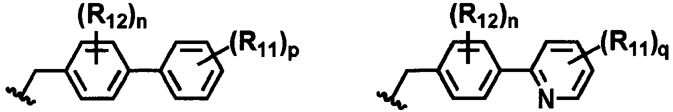

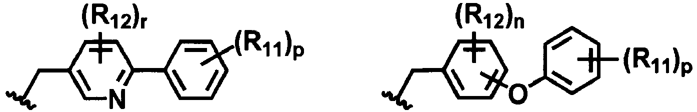

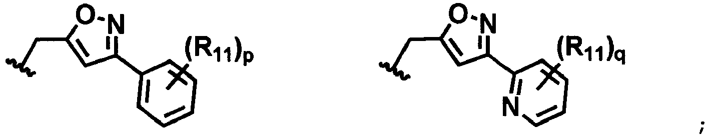

X is sulfur (S) or selenium (Se);

Y is carbon (CH) or nitrogen (N);

R₃ is hydrogen, C₁-C₈ alkyl, or halogen;

R₄ and R₅ each are independently hydrogen, halogen, or C₁-C₈ alkyl;

R₆ is hydrogen, C₁-C₈ alkyl, C₂-C₇ alkenyl, alkali metal, alkali earth metal, or organic acid;

R₁₁ and R₁₂ each are independently hydrogen, halogen, C₁-C₈ alkyl, or C₁-C₈ alkoxy;

R₂₁ is hydrogen, halogen, C₁-C₇ alkyl, a heterocyclic group, C₁-C₇ alkoxy, C₁-C₅ alkyl substituted with halogen, or phenyl substituted with halogen;

m and n each are independently an integer of 1~4;

p is an integer of 1~5;

q is an integer of 1~4;

r is an integer of 1~3;

s is an integer of 1~5; and

the alkyl and alkoxy of R₂, R₃, R₄, R₅, R₆, R₁₁, R₁₂ and R₂₁ may be further substituted with at least one halogen or C₁-C₅ alkylamine.]

Some of the PPAR δ agonists are represented by Chemical Formula II below.

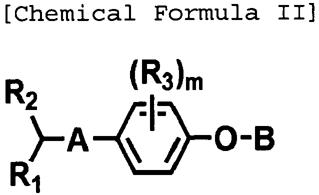

[In Chemical Formula I, A is sulfur (S) or selenium (Se); B is

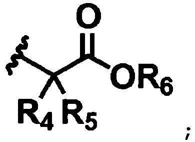

R₁ is selected from the following structures:

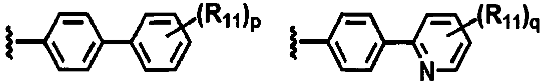

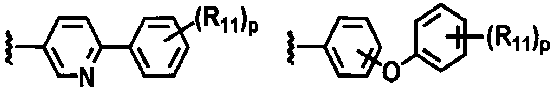

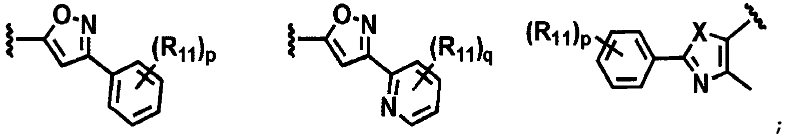

R₂ is selected from hydrogen, C₁-C₅ alkyl, 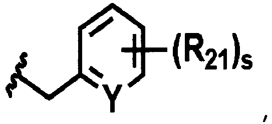 or the following structures:

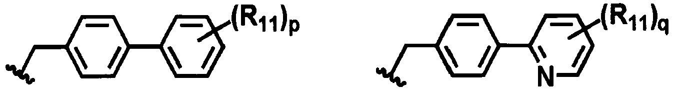

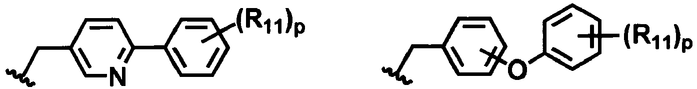

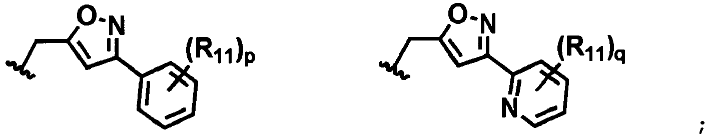

X is sulfur (S) or selenium (Se);

Y is carbon (CH) or nitrogen (N);

R₃ is hydrogen, C₁-C₅ alkyl or halogen;

R₄ and R₅ each are independently hydrogen or C₁-C₅ alkyl;

R₆ is hydrogen, C₁-C₅ alkyl, C₂-C₅ alkenyl, alkali metal, or alkali earth metal;

R₁₁ is hydrogen, halogen, C₁-C₅ alkyl, or C₁-C₅ alkoxy;

R₂₁ is hydrogen, halogen, C₁-C₅ alkyl, a heterocyclic group, C₁-C₅ alkoxy, C₁-C₅ alkyl substituted with halogen, or phenyl substituted with halogen;

m is an integer of 1~4;

p is an integer of 1~5;

q is an integer of 1~4;

s is an integer of 1~5; and

the alkyl and alkoxy of R₂, R₃, R₄, R₅, R₆, R₁₁, and R₂₁ may be further substituted with at least one halogen or C₁-C₅ alkylamine.]

Some of the PPAR δ agonists are represented by Chemical Formula III below:

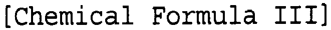

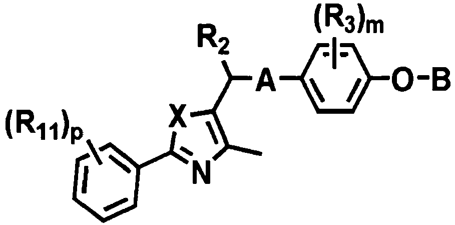

[In Chemical Formula III, A is independently sulfur (S) or selenium (Se); B is

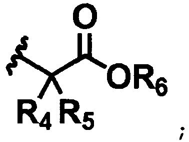

R₂ is selected from hydrogen, C₁-C₅ alkyl, 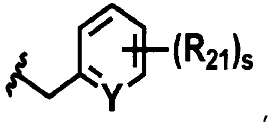 or the following structures:

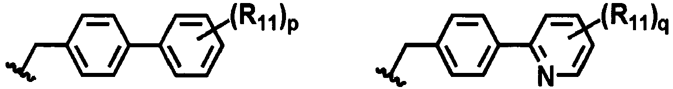

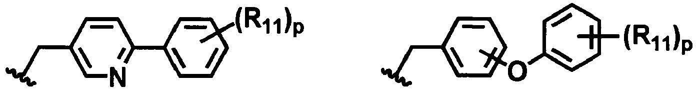

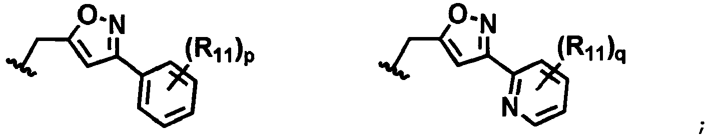

X is sulfur (S) or selenium (Se);

Y is carbon (CH) or nitrogen (N);

R₃ is hydrogen, C₁-C₅ alkyl, or halogen;

R₄ and R₅ each are independently hydrogen or C₁-C₅ alkyl;

R₆ is hydrogen, C₁-C₅ alkyl, C₂-C₅ alkenyl, alkali metal, or alkali earth metal;

R₁₁ is hydrogen, halogen, C₁-C₅ alkyl, or C₁-C₅ alkoxy;

R₂₁ is hydrogen, halogen, C₁-C₅ alkyl, a heterocyclic group, C₁-C₅ alkoxy, C₁-C₅ alkyl substituted with halogen, or phenyl substituted with halogen;

m is an integer of 1~4;

p is an integer of 1~5;

q is an integer of 1~4;

s is an integer of 1~5; and

the alkyl and alkoxy of R₂, R₃, R₄, R₅, R₆, R₁₁, and R₂₁ may be further substituted with at least one halogen or C₁-C₅ alkylamine.]

Some of the PPAR α agonists are represented by Chemical Formula IV below.

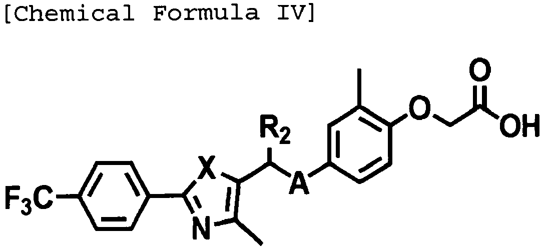

[In Chemical Formula IV, A is independently sulfur (S) or selenium (Se);

R₂ is selected from hydrogen, C₁-C₅ alkyl, 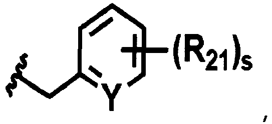 or the following structures:

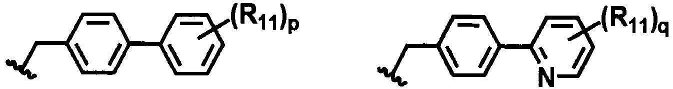

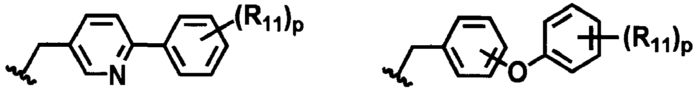

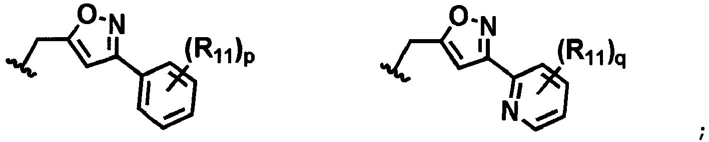

X is sulfur (S) or selenium (Se);

Y is carbon (CH) or nitrogen (N);

R₁₁ is hydrogen, halogen, C₁-C₅ alkyl, or C₁-C₅ alkoxy;

R₂₁ is hydrogen, halogen, C₁-C₅ alkyl, a heterocyclic group, C₁-C₅ alkoxy, C₁-C₅ alkyl substituted with halogen, or phenyl substituted with halogen;

p is an integer of 1~5;

q is an integer of 1~4;

s is an integer of 1~5; and

the alkyl and alkoxy of R₁₁ and R₂₁ may be further substituted with at least one halogen or C₁-C₅ alkylamine.]

A novel compound of the PPAR δ agonists is represented by Chemical Formula V below.

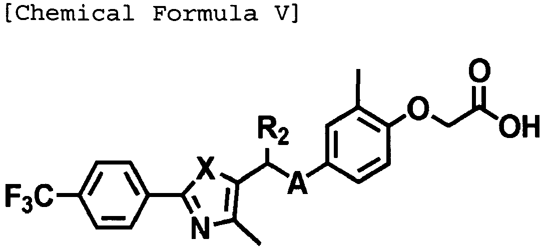

[In Chemical Formula V, A is independently sulfur (S) or selenium (Se);

R₂ is selected from the following structures:

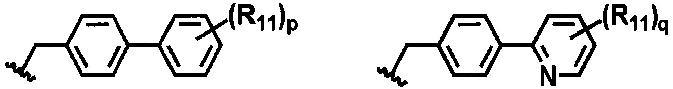

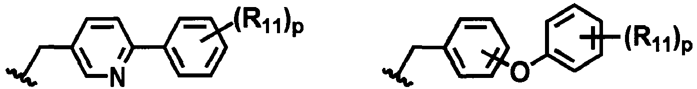

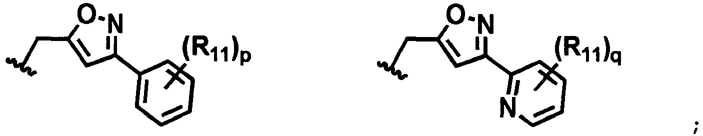

X is sulfur (S) or selenium (Se);

R₁₁ is hydrogen, halogen, C₁-C₅ alkyl, or C₁-C₅ alkoxy;

p is an integer of 1~5;

q is an integer of 1~4;

the alkyl and alkoxy of R₁₁ may be further substituted with at least one halogen or C₁-C₅ alkylamine.]

More specifically, a novel material of the PPAR δ agonists is represented by Chemical Formula VI below:

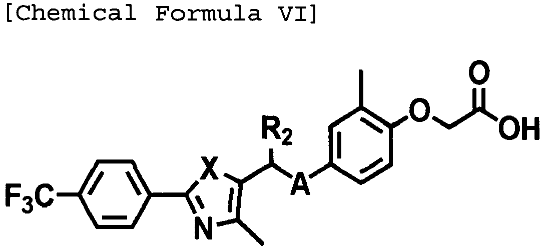

[In Chemical Formula VI, A is independently sulfur (S) or selenium (Se);

R₂ is selected from the following structures:

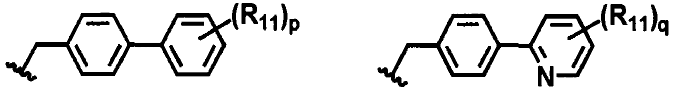

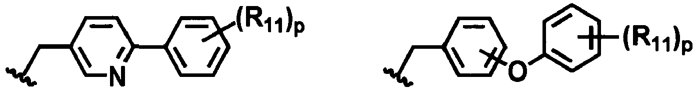

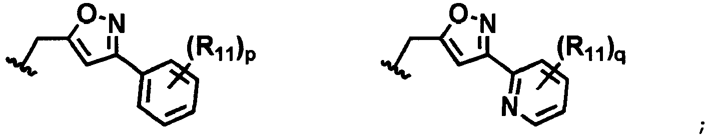

X is sulfur (S) or selenium (Se);

R₁₁ is hydrogen, C₁-C₃ alkyl or halogen;

p is an integer of 1-5;

q is an integer of 1-4;

the alkyl of R₁₁ may be further substituted with at least one halogen or C₁-C₅ alkylamine.]

More specifically, the novel material among the PPAR δ agonists is as follows:

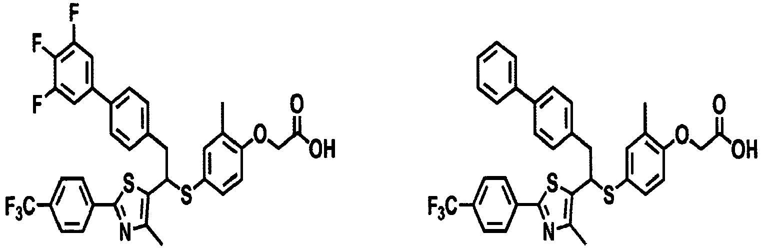

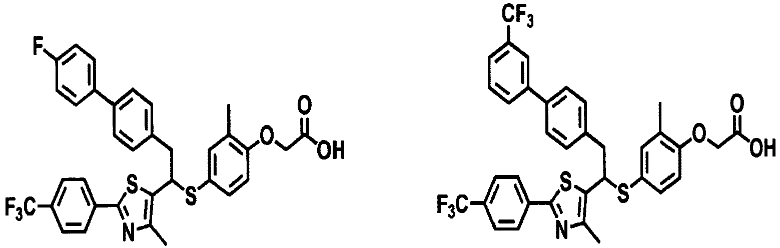

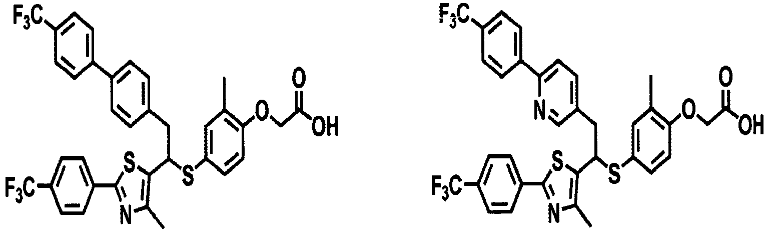

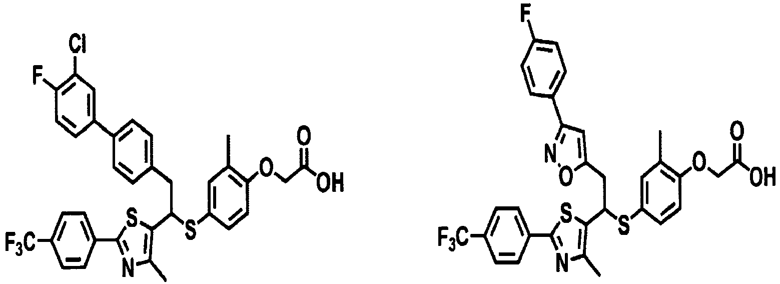

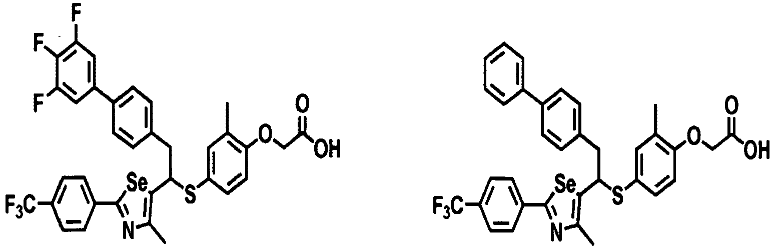

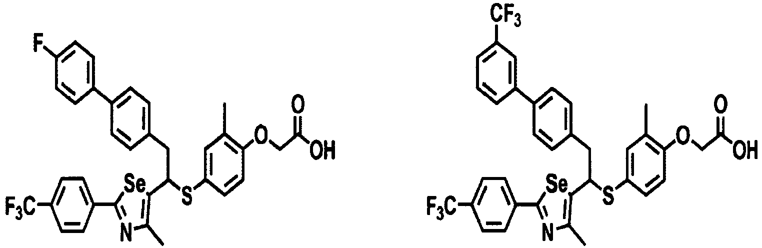

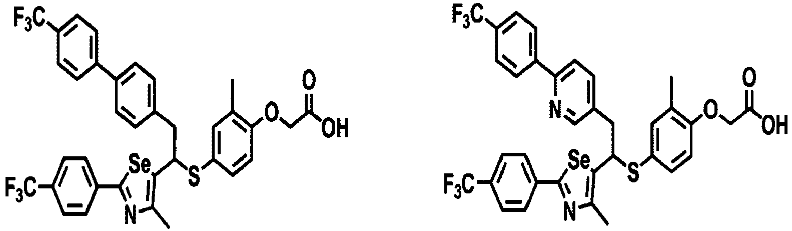

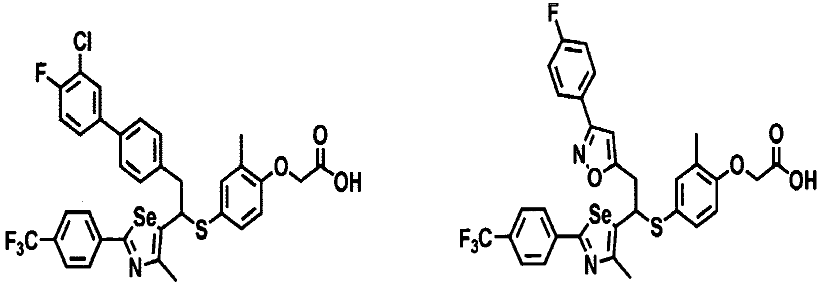

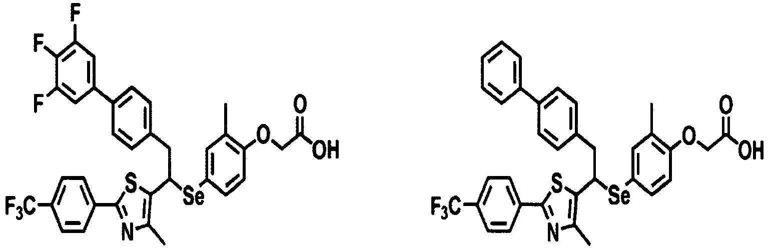

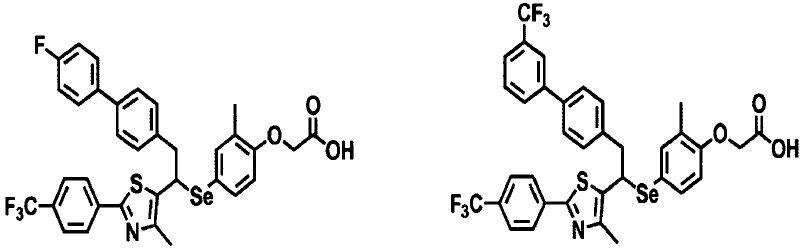

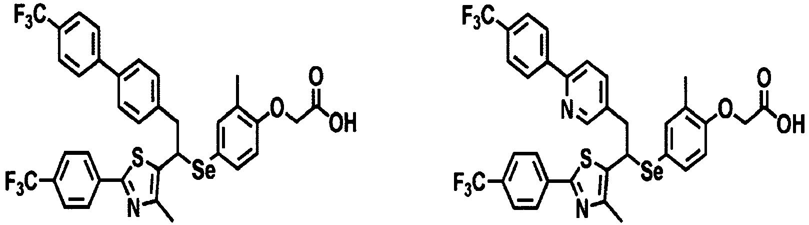

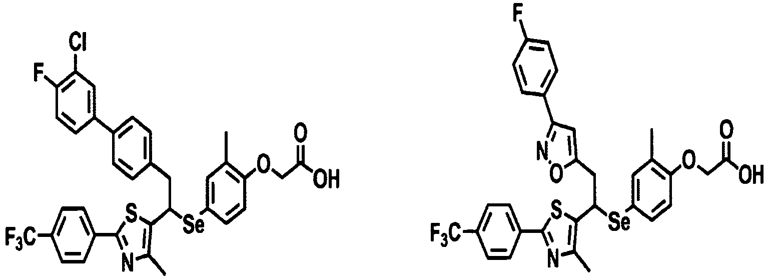

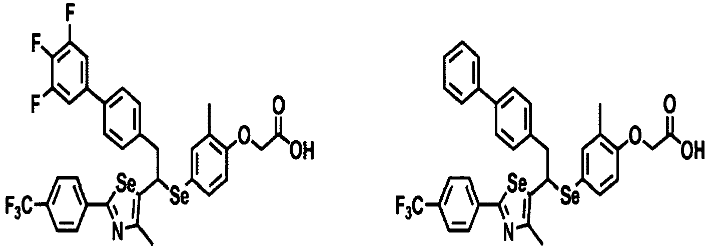

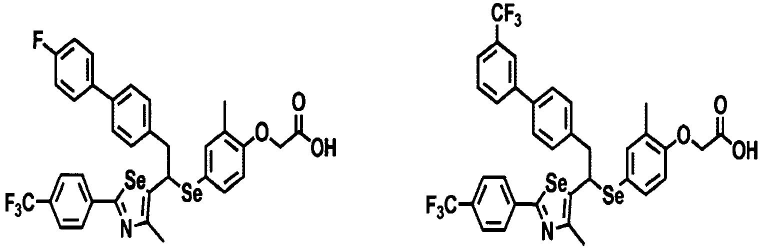

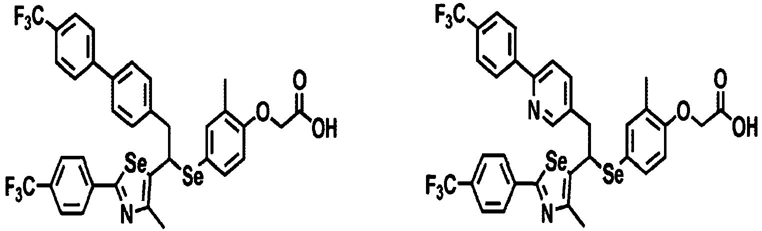

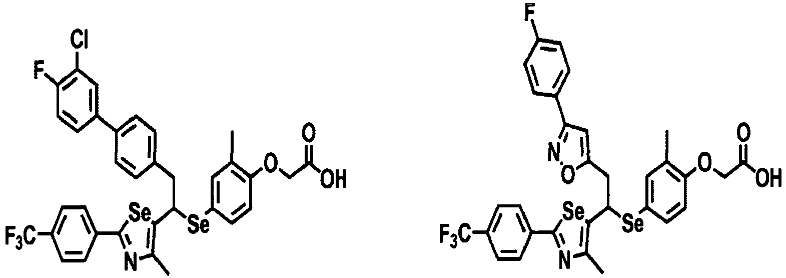

In addition, the present invention provides a composition for medicine for treating and preventing atherosclerosis or hyperlipidemia, treating and preventing hypercholesterolemia, treating and preventing fatty liver, treating and preventing diabetes, treating and preventing obesity, strengthening muscle, treating and preventing muscular diseases, enhancing endurance, improving memory, or treating and preventing dementia or Parkinson's disease, containing the PPAR δ agonist represented by Chemical Formula V above as an effective component. Further, the present invention provides a composition for a functional food supplement, a functional beverage, a food additive, and a feedstuff for animal, containing the noble PPAR δ agonist as an effective component. Further, the present invention provides a composition for functional cosmetics for preventing and improving obesity, preventing and improving fatty liver, strengthening muscle, preventing and improving muscular diseases, and enhancing endurance, containing the noble PPAR δ agonist as an effective component.

The amount of PPAR δ agonist used to achieve therapeutic effects according to the present invention is varied depending on the specific compound, administration method, object to be treated, and disease to be treated, but depends on a conventional medicine administration amount. More preferably, the PPAR δ agonist may be administered in the range of an effective input amount of 1-100 mg/kg (body weight)/1day. In addition, the PPAR δ agonist is administered one per day or several times per day within the range of an effective input amount. In addition, oral administration or topical administration may be possible depending on the kind of dosage form. The pharmaceutical composition according to the present invention may be formulated into all of the existing various forms in the case of oral administration, and may be in various forms such as tablet, powder, dry syrup, chewable tablet, granule, chewing tablet, capsule, soft capsule, pill, drink, sublingual tablet, and the like. The tablet according to the present invention may be administered to a patient in an effective amount through any bio-available form or manner, that is, an oral pathway. An appropriate form or manner may be easily selected depending on characteristics of disease status to be treated or prevented, stage of the disease, and other related matter. If the composition according to the present invention is in a tablet form, it may further at least one pharmaceutically acceptable vehicle, and the ratio of properties of the vehicle may be determined by dissolution and chemical properties of the selected tablet, the selected administration pathway, and the standard pharmaceutical practice.

### [Advantageous Effects]

According to the present invention, the PPAR δ agonist adjusts calcium ion during embryo genesis and an early fetal development period to increase slow muscle fiber and thus improve muscle endurance, thereby improving lipid and glucose metabolism and reprogramming the metabolism of the entire body, thus preventing/inhibiting the occurrence of metabolic diseases, such as obesity and diabetes as an adult or in an adult body, caused by a high-fat diet and a lack of exercise, and improving memory. Therefore, the PPAR δ agonist can be used for a composition for medicine, a nutritional supplement for pregnant women, a composition for medicine for animals, an endurance enhancer for animals, a composition for dry milk and baby food, and a composition for feedstuff for animal, for enhancing endurance of a human being and an animal preventing/inhibiting metabolic diseases such as obesity, diabetes, arteriosclerosis and fatty liver, and improving memory, through fetal reprogramming.

### [Description of Drawings]

FIG. 1 is an experiment procedure schematic diagram of Example 1 [a co-administration during both of gestation and lactation periods, b. administration during a gestation period, and c. administration during a lactation period].

FIG. 2 shows the results of a pharmacokinetic experiment conducted in order to check whether or not a PPAR δ agonist administered to a pregnant mother passes through the placenta.

FIG. 3 shows the results of body weight measurement immediately after birth of fetal mice subjected to fetal reprogramming by the PPAR δ agonist, in the experiment conducted according to the method of FIG. 1 (n=8).

FIG. 4 shows the results of body weight changes of fetuses through 2-week observation in the experiment conducted according to the method of FIG. 1 (n=8).

FIG. 5 shows the results of comparison of body weight when the fetuses grew to 6 weeks in age in the experiment conducted according to the method of FIG. 1 (n=8).

FIG. 6 is an image showing the results of fetal skeleton staining to check normality or abnormality of skeleton development in the experiment conducted according to the method of FIG. 1.

FIG. 7 shows the results of muscular fiber type analysis after growth of the fetuses subjected to fetal reprogramming in the experiment conducted according to the method of FIG. 1. (A and B) - muscle change observation results after the skin of the dorsal region and forelegs were resected. (C and D) - slow muscle fiber change results in the gastrocnemius muscle through metachromatic staining and immunohistochemistry, after the hind legs were resected.

FIG. 8 is a table summarizing micro-array results in the experiment conducted according to the method of FIG. 1.

FIG. 9 shows the comparison results of expressions of genes, which are important for lipid oxidation and thermogenesis in the muscle, by using the real-time PCR analysis method, in the experiment conducted according to the method of FIG. 1 (**, P < 0.01; ***, *P* < 0.001).

FIG. 10 shows the comparison results of expressions of genes, which are important for slow muscle fiber development by using the real-time PCR analysis method, in the experiment conducted according to the method of FIG. 1 (*, *P* < 0.05; **, *P* < 0.01; ***, *P* < 0.001).

FIG. 11 is a chart showing comparison results of expressions of genes, which are important for slow type muscular function by using the real-time PCR analysis method, in the experiment conducted according to the method of FIG. 1 (*, *P* < 0.05; ***, *P* < 0.001).

FIG. 12 shows analysis results of expressions of genes in the PPAR δ-deficient muscular cell by using the real-time PCR in order to verify that the results of the fetal reprogramming experiment conducted by the method of FIG. 1 are PPAR δ-dependent (*, P < 0.05; **, *P* < 0.01; ***, *P* < 0.001).

FIG. 13 shows the results of the experiment using a confocal microscope in order to confirm quantitative change in cytosolic calcium ions due to PPAR δ in the fetal reprogramming experiment conducted by the method of FIG. 1 (*, P < 0.05).

FIG. 14 shows the results of measurement of endurance of male mice having increased slow muscle fibers by PPAR δ, using a treadmill, in the fetal reprogramming experiment conducted by the method of FIG. 1 (***, P < 0.001,n = 4-5).

FIG. 15 shows the results of measurement of endurance of female mice having increased slow muscle fibers by PPAR δ, using a treadmill, in the fetal reprogramming experiment conducted by the method of FIG. 1 (***, *P* < 0.001, n = 4-5).

FIG. 16 shows the results of measurement of respiratory exchange ratios, using Oximax, in the experiment conducted by the method of FIG. 1 (**, P < 0.01, n = 4).

FIG. 17 shows the comparison results of conversion of the results of FIG. 16 into area values (**, P < 0.01, n = 4).

FIG. 18 shows the record results of body weight increase change with the intake of high-fat feedstuff having 35% fat content in the fetal reprogramming experiment conducted by the method of FIG. 1 (*, *P* < 0.05, n = 5).

FIG. 19 shows the results of a glucose tolerance test of mice having induced diabetes due to the intake of high-fat diet in the fetal reprogramming experiment conducted by the method of FIG. 1 (**, *P* < 0.01, n = 6).

FIG. 20 shows the results of an insulin tolerance test of mice having induced diabetes due to the intake of high-fat diet in the fetal reprogramming experiment conducted by the method of FIG. 1 (**, *P* < 0.001, n = 6).

FIG. 21 shows the result verifying CMDD1226 effect in the fetal reprogramming experiment conducted by the method of FIG. 1 (**, *P* < 0.01, n=4).

FIG. 22 shows the experiment results of comparison of the difference in endurance enhancement effect depending on the administration period of GW501516 in the fetal reprogramming experiment conducted by the method of FIG. 1 (1: Vehicle treatment of pregnant mice during gestation and lactation period; 2: GW501516 treatment of pregnant mice during lactation; 3: GW501516 treatment of pregnant mice during gestation; 4: GW501516 treatment of pregnant mice during gestation and lactation period) (**, *P* < 0.01; ***, *p* < 0.001, n=4).

FIG. 23 shows the difference in endurance enhancement effect depending on the administration concentration of CMDD1112 and the gender (male) in the fetal reprogramming experiment conducted by the method of FIG. 1 (***, *P* < 0.001, n=4).

FIG. 24 shows the difference in endurance enhancement effect depending on the administration concentration of CMDD1112 and the gender (female) in the fetal reprogramming experiment conducted by the method of FIG. 1 (***, *P* < 0.001,n=4).

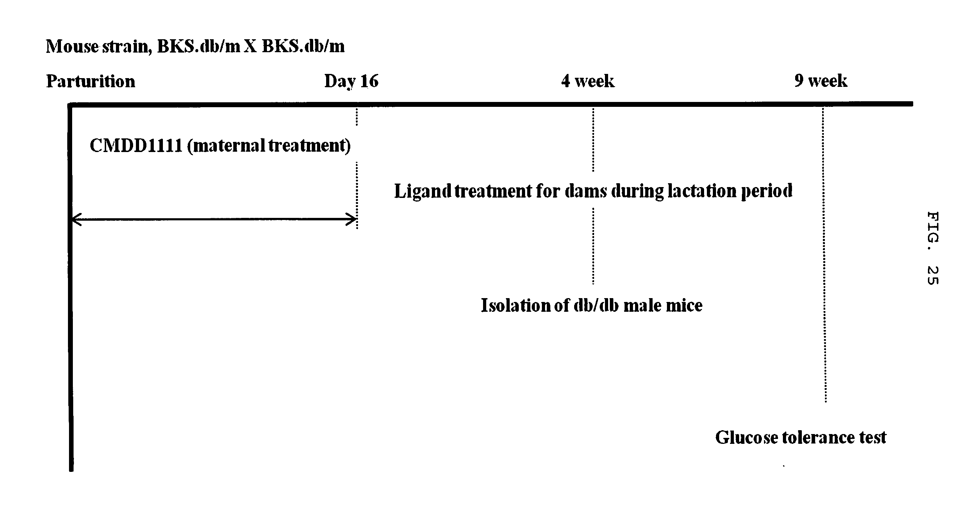

FIG. 25 is an experiment procedure schematic diagram using Type II diabetes model.

FIG. 26 shows the results of a glucose tolerance test of type II diabetes mouse model in the fetal reprogramming experiment conducted by the method of FIG. 25 (*, P < 0.05,n = 6 - 9).

### [Best Mode]

Hereinafter, the present invention will be described in detail by the examples.

The following examples are for merely exemplifying the present invention, and therefore, the scope of the present invention is not limited to the following examples.

**1. Fetal Reprogramming Effect by PPAR δ Agonist**

The structures of PPAR δ agonists used in the following examples are as follows:

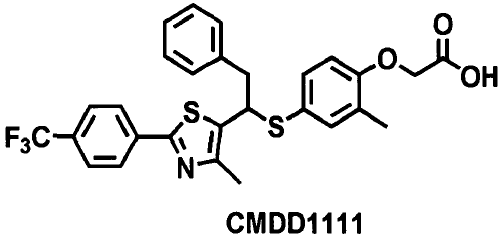

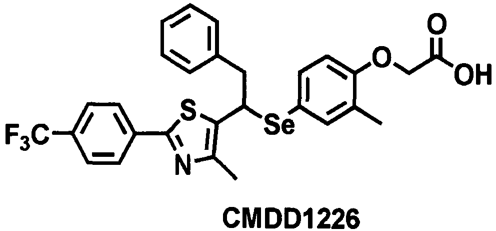

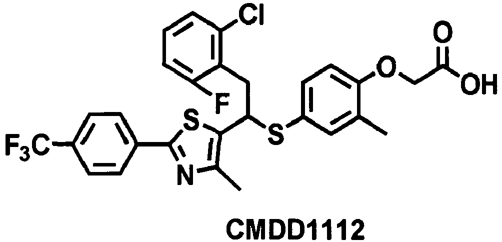

<Example 1> Mouse Fetal Reprogramming using PPAR δ agonist

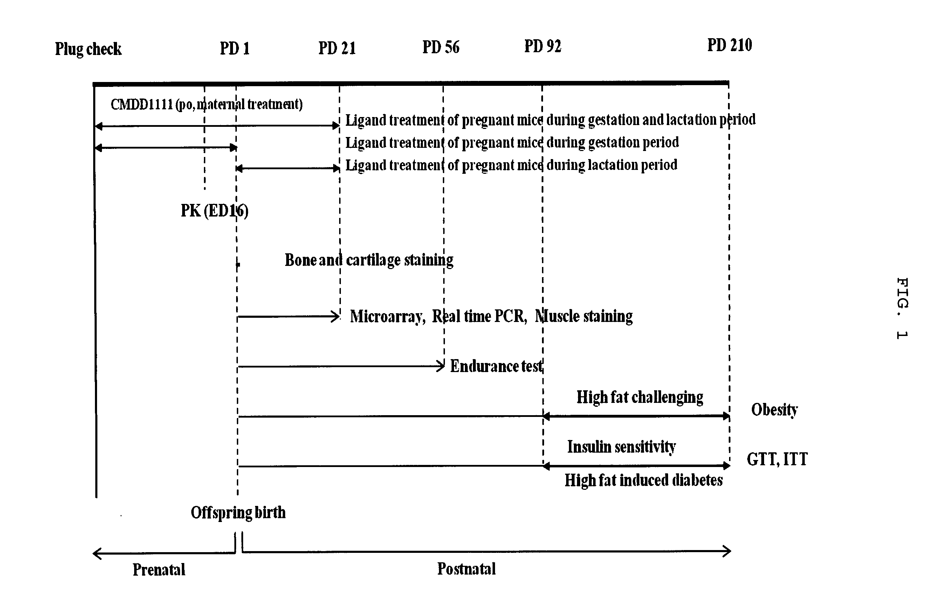

The present inventors used a method of administering a PPAR δ agonist to a pregnant mother (maternal treatment) in order to investigate the role of fetal reprogramming of PPAR δ during mammalian development. For the fetal reprogramming, the PPAR δ agonist was orally administered to pregnant mice at a concentration of 10 mg/kg every day, and a specific experiment method was summarized in FIG. 1.

(1) Confirmation on Whether or Not PPAR δ Agonist Passes Through Placenta

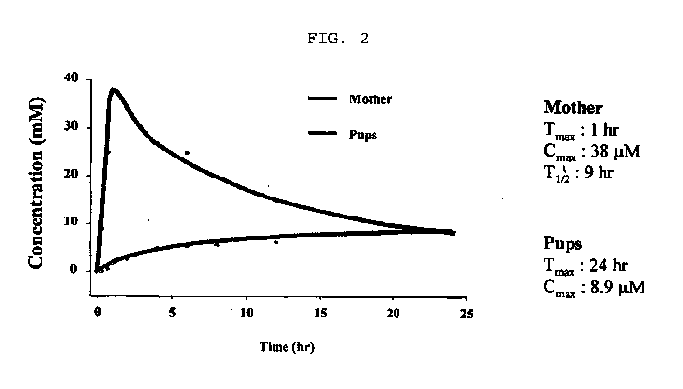

Pharmacokinetic experiment was conducted in order to confirm whether or not a PPAR δ agonist used in the present study, CMDD1111, passes through the placenta. CMDD 1111 was administered to 16-day pregnant mice at a concentration of 10 mg/kg, and then concentrations of the compound distributed in the bloods of mother animals and fetuses were analyzed for 24 hours. As a result, in the case of the mother animals, the maximum blood concentration (Cₘₐₓ) thereof was 38 µM, the time for reaching the maximum blood concentration (Tₘₐₓ) thereof was 1 hour, and the half-time (t_{1/2}) thereof was 9 hours. In the case of the fetuses, the blood concentration thereof tended to increase with the time, unlike the mother animals, and the maximum blood concentration thereof was 8.9 µM. This concentration enables PPAR δ to sufficiently activate in the fetuses, and thus it was confirmed that the compound used in the present example passed through the placenta (FIG. 2).

(2) Reprogramming of Fetal Mouse Through Administration of PPAR δ Agonist to Pregnant Mother

### (Maternal Treatment)

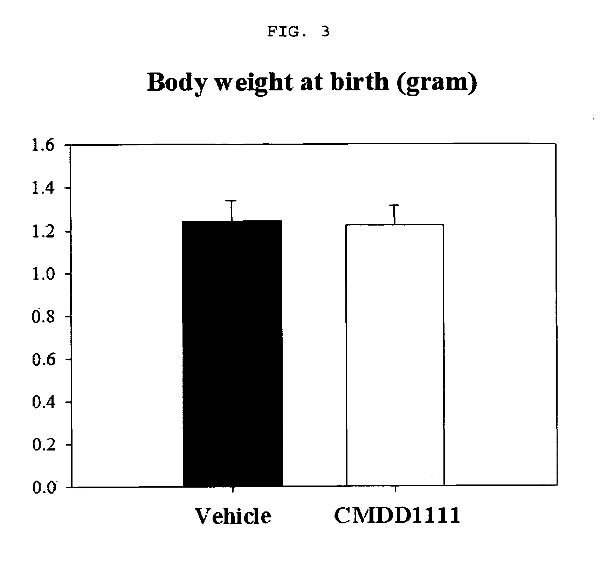

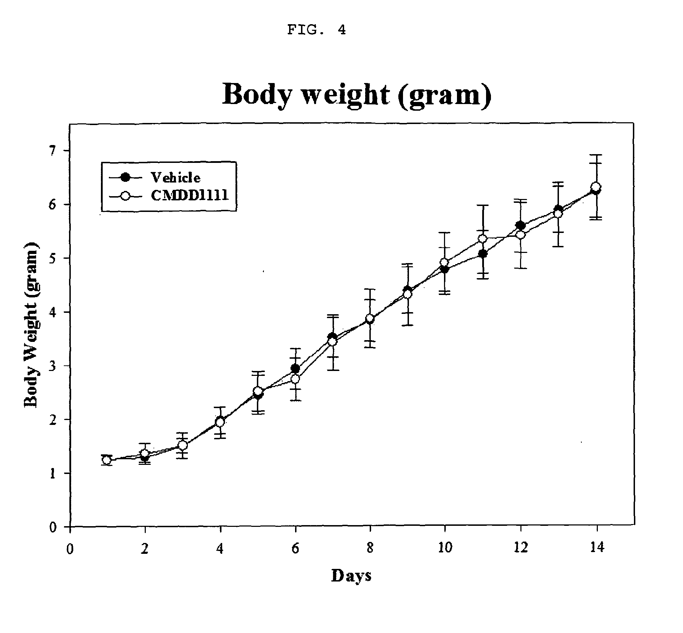

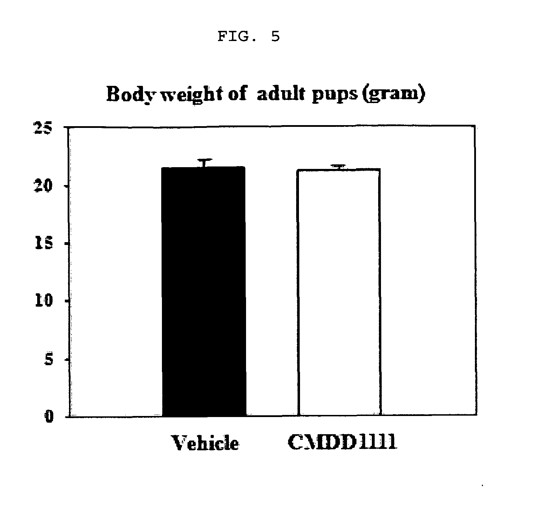

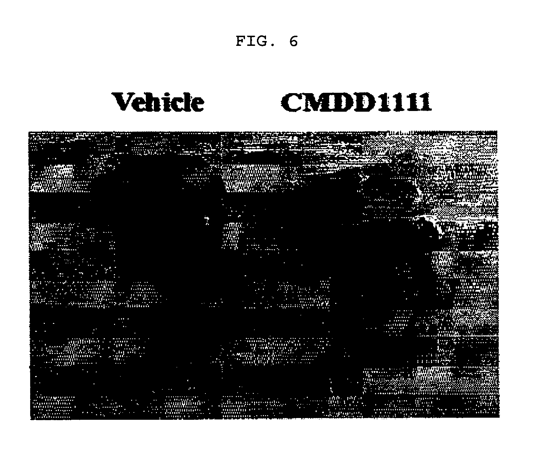

Fertile female mice and male mice were bred, and then pregnancy or not was checked by plug confirmation. CMDD1111 was orally administered to pregnant female mice at a concentration of 10 mg/kg once per day every day for six weeks (gestation and lactation periods). Side effects due to the administered material were not observed during the gestation period. The weight of a fetus group administered with vehicle during the gestation period was 1.33 ± 0.11 g, and the weight of a fetus group administered with CMDD1111 was 1.37 ± 0.09 g. The weight difference between the two groups was not shown (FIG. 3). In addition, growth inhibition or promotion due to the administered material was not observed in the comparison results of growth rate of fetus for 3 weeks (FIG. 4). There was no difference in body weight between the two groups as adults (FIG. 5). Deformity due to the administered material was not observed in the skeleton staining results using Alizaren red S and Alician blue dye (FIG. 6).

<Example 2> Verification on Slow Muscle Fiber Increase Effect in Mice Subjected to Fetal Reprogramming Through PPAR δ Activation

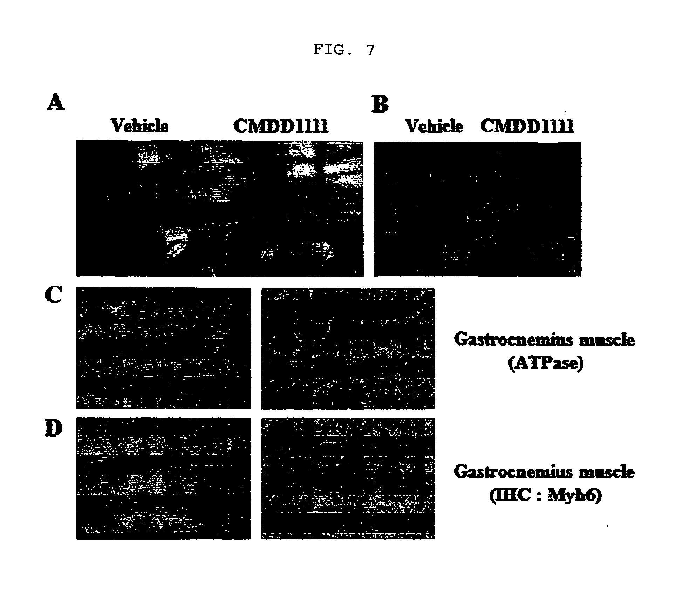

The slow muscle fiber has a lot of myoglobin, which is oxygen transfer protein, and thus appears redder than the fast muscle. When the skin of the fetus was removed and the muscle thereof was observed, it may be seen that administered mice group had redder muscle (FIGS. 7A and 7B). In order to confirm whether or not slow muscle fiber generation was increased due to the administration material (CMDD1111), hind legs thereof were resected and then frozen in OCT. Frozen muscle tissues were cut into 8 mm-thick sections by using a freezing cryotome, and mounted on slides, which was then subjected to metachromatic staining. The slow muscle fiber was stained darker blue than the fast muscle by the metachromatic staining. As can be confirmed in FIG. 7C, dark blue muscle fibers were increasingly generated in the gastrocnemius muscle by the administration material (CMDD1111). Muscle fibers may be discernable even by immunostaining using an isoform antibody of the myosin heavy chain (MHC). The present inventors conducted immunostaining using the slow type MHC in order to confirm whether or not slow muscle fibers were increased by the administration material (CMDD1111). As a result, as can be seen from FIG. 7D, the muscle fibers responsive to the slow type MHC were increased in the gastrocnemius muscle of the administration group. This result was the same as the metachromatic staining result, and these two experiment results confirmed that the slow muscle fibers were increasingly generated by the administration material (CMDD1111).

<Example 3> Verification on Slow Muscle Fiber Increase Effect Through Adjustment of Calcium Ions

The present invention conducted the gene expression analysis to establish the cause of the increase in slow muscle fiber generation in the mice subjected to fetal reprogramming by PPAR δ Activation.

(1) Gene Expression Analysis Using Microarray

As for an animal, the gastrocnemius muscle of 3-week old mice fetal-reprogrammed by PPAR δ agonist (CMDD1111) was resected, and then total RNA was extracted by using the Trizol reagent (Gibco). The isolated RNA was subjected to quantitative and purity verification processes, and then used for gene expression analysis using a Mouse 430 2.0 (Affimatrix) chip.

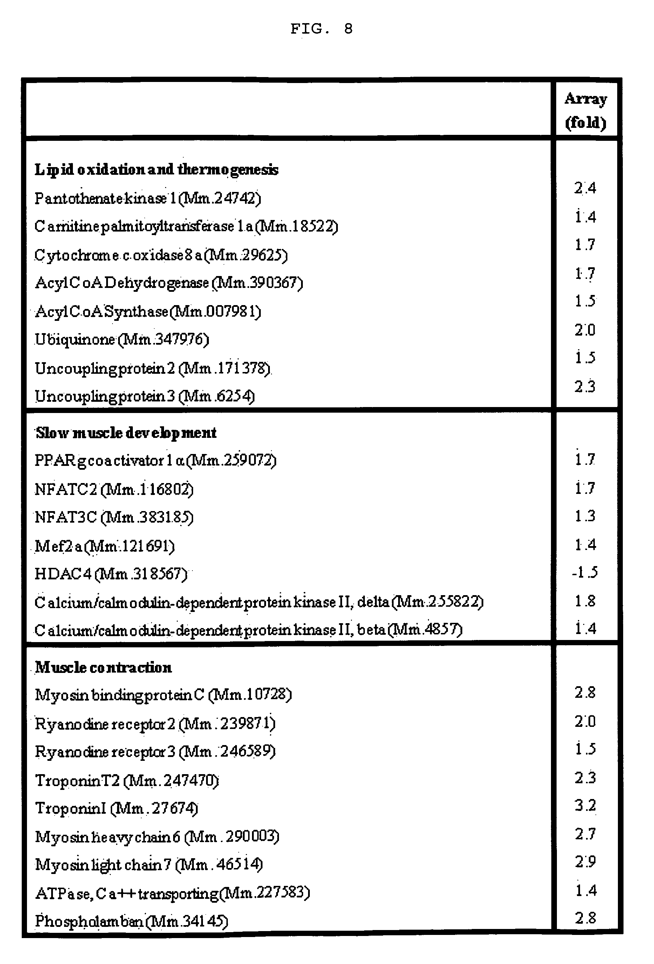

Generally, the slow muscle fiber has more mitochondria than the fast muscle fiber, increased fatty acid oxidation, and a low contraction rate. It was confirmed from analysis results that all of the gene expressions associated with the above three kinds of regulations were increased (FIG. 8).

There were increases in expressions of enzymes necessary for fatty acid oxidation and mitochondrial respiration, such as pantothenate kinase 1 (PANK1: 2.4 fold), carnitine palmitoyl transferase 1a (CPT-1a: 1.4 fold), acyl CoA dehydrogenase (ACAD: 1.7 fold), acyl CoA synthetase (ACSL: 1.5 fold), cytochrome 8a (COX8A: 1.7 fold), ubiquinone (2.0 fold), and uncoupling protein 2, 3 (UCP2, 3: 1.5 fold, 2.3 fold).

There were increases in expressions of PPAR 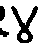 coactivator 1a (1.7 folds), CamIIb (1.4 fold), CamIId (1.8 fold), and Mef2a (1.4 fold), which have been known to increase generation of slow muscle fibers, and there was a decrease in expression of HDAC4 (-1.5 fold) known as a negative regulator.

The slow muscle fiber has more calcium than the fast muscle fiber, and slow subtype calcium regulating proteins. Interestingly, as for the mice with increased slow muscle fibers according to the present invention, there were increases in expression of slow type calcium regulating proteins such as myosin binding protein C3 (MYBPC3: 2.8 fold), Troponin T2 (2.3 fold), Troponin I (3.2 fold), myosin heavy chain 6 (Myh6: 2.7 fold), and myosin light chain 7 (Myl7: 2.9 fold), and there were increases in expression cytosolic calcium concentration regulators, such as ryanodine receptor 2, 3 (RYR2, 3: 2.8 fold, 2.0 fold), ATPase, Ca2+ transporting (SERCA: 1.4 fold), and phospholamban (PLN: 2.8 fold).

(2) Gene Expression Analysis Using Real-Time PCR

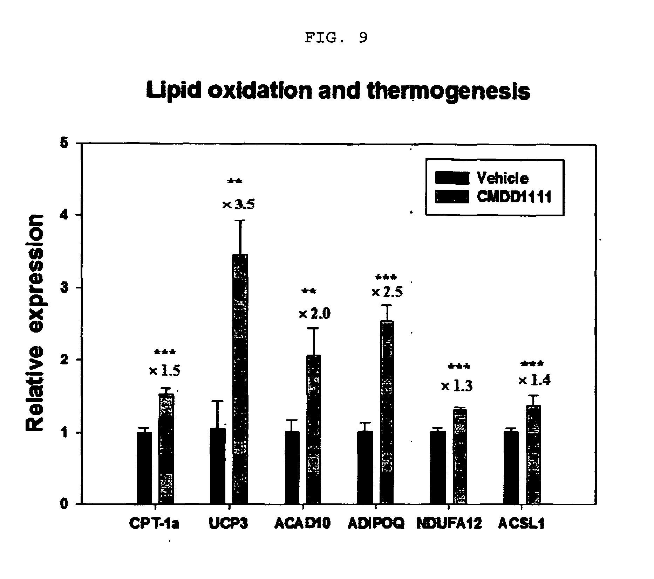

A gene expression analysis experiment using the real-time PCR was conducted to further verify Miroarray analysis results. As for mouse group subjected to fetal reprogramming by administration of PPAR δ agonist (CMDD1111) during a muscular development period, there were increases in expression of core regulators for fatty acid oxidation and thermogenesis, such as carnitine palmitoyl transferase 1a (CPT-1a: 1.5 fold), uncoupling protein 3 (UCP3: 4.2 fold), acyl Co-A synthetase (ACSL: 1.4 fold), acyl Co-A dehydrogenase (ACAD10: 2.0 fold), and NDUFA12 (1.3 fold). In addition, there was a remarkable increase in expression of Adiponectin (ADIPOQ: 2.5 fold), which are commonly expressed in the slow muscle fiber and has been known to increase fatty acid oxidation to improve diabetes (FIG. 9).

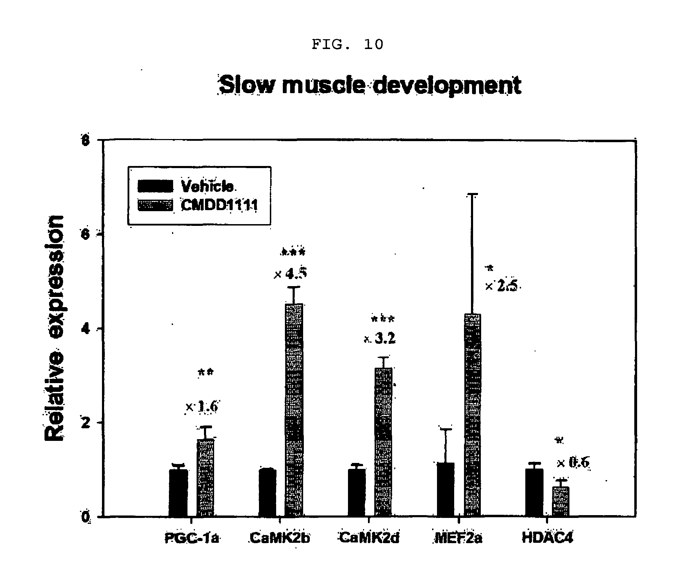

PGC-1α is a gene that is turned out to be a coactivator of fatty acid oxidation as well as a core regulator to increase mitochondria synthesis and slow muscle fiber generation. Expression of this gene was increased by 1.6 fold due to PPAR δ agonist (CMDD1111). In addition, expressions of CaMKII B and D, which have been known to increase fast-to-slow muscle fiber transformation during a muscular remodeling procedure, were also increased by 4.5 fold and 3.2 fold, respectively. Whereas, expression of HDAC4, previously known to suppress the slow muscle fiber generation through the loss of function study, was decreased by 0.6 fold (FIG. 10).

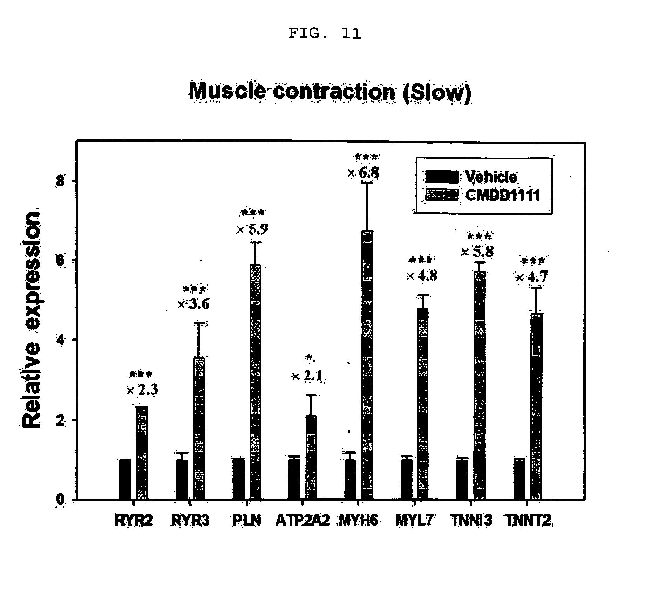

As for the skeleton muscle, the concentration of cytosolic calcium is necessary for excitation-contraction coupling, and the concentration thereof itself is an important regulator of regulating muscle plasticity. In the present invention, the mice with increased slow muscle fibers had increased expressions of factors, known to regulate the cytosolic calcium in the myocytes. RYR2 and RYR3 are genes regulating calcium efflux in the sarcoplasmic membrane, and expressions thereof were increased by 2.2 fold and 3.6 fold, respectively, as compared with the control group. ATP2A2 is gene regulating calcium influx from the cytoplasm to the sarcoplasmic reticulum, and expression thereof was increased by 2.2 fold. Expression of phospholamban, which is a gene regulating activity of ATP2A2 and specific to slow muscle, was increased by 3.2 fold. Also, there were increases in expressions of TNNI3 (5.8 fold), MYH6 (6.8 fold), and Myl7 (4.8 fold), which are combined with calcium to thereby be involved in muscle contraction (FIG. 11).

(3) Primary Cell Culture and Gene Expression Analysis

It was found from the microarray analysis and real-time PCR analysis that expressions of RYR3, ATP2A2, and PLN, which are core genes for regulating concentration of cytosolic calcium, were significantly increased for the mice subjected to fetal reprogramming by the PPAR δ agonist (CMDD1111). However, it has not been reported that the PPARs response element (PPRE) is present at promoters of these three genes. Therefore, the present inventors resected the muscle of the mouse with the removal of PPAR δ gene and conducted primary culture, in order to verify whether or not the increases in expressions of the above three genes are PPAR δ-dependent. After hind legs of a five-day-old fetus was resected, primary culture was conducted by the same method as developed by Rando (Rando et al, J. Cell. Biol., 1994, 125, 1275-1287). Myoblast, after being cultured, was transferred to a 6-well plate, and then differentiated into myotube while being treated with 300 nM of PPAR δ agonist (CMDD1111). The differentiated myotube was further treated with 300 nM of PPAR δ agonist (CMDD1111) for 2 days, and then total RNA was extracted by using a TRIzol reagent (Gibco). The extracted RNA was subjected to reverse transcription, to synthesize cDNA, which was then used as a template for real-time PCR analysis.

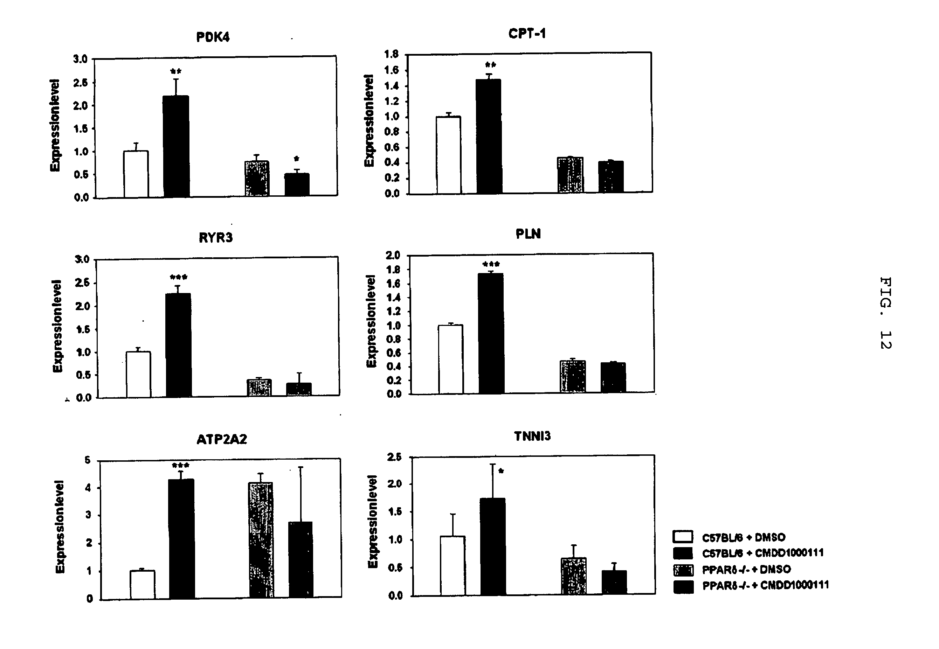

As the result of real-time PCR analysis, expressions of PDK4 and CPT-1, known to be directly regulated by PPAR δ, were increased by 2.2 fold and 1.5 fold, respectively. However, there was no change in PPAR 5-deficient cells. This means that expressions of PDK4 and CPT-1 are PPAR δ-dependently regulated. This tendency was also shown in the same manner for RYR (2.2 fold), PLN (1.7 fold), TNNI3 (1.7 fold), and ATP2A2 (4.3 fold), as can be seen in FIG. 12. Therefore, PPAR δ directly regulates expressions of genes regulating concentration of cytosolic calcium.

(4) Verification on Cardiac Muscle Cell Regeneration Effect

As for the mouse group subjected to fetal reprogramming by PPAR δ agonist (CMDD1111), it was confirmed that expressions of genes such as RYR2, ATP2A2, and PLN, known to be commonly expressed in the heart muscle, were remarkably increased.

(5) Analysis of Cytosolic Calcium

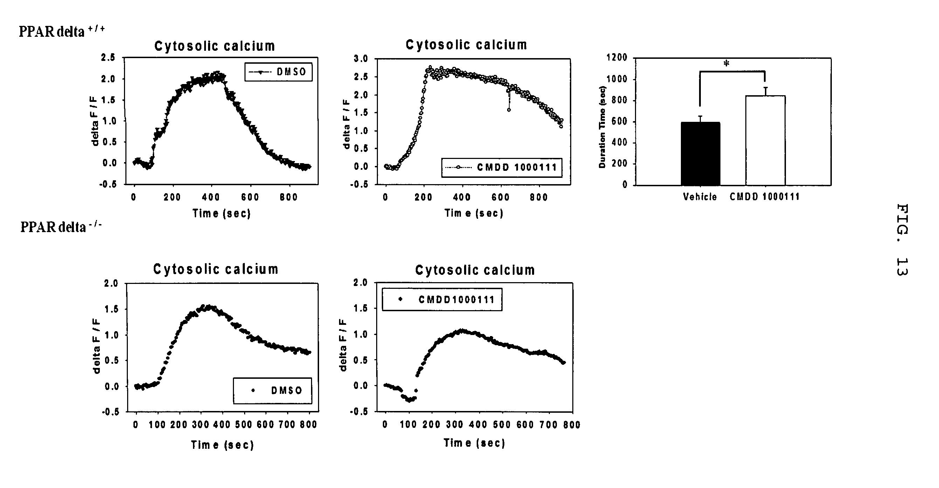

An experiment using a calcium-specific dye reagent, Flou4(Molecular probe), was conducted in order to confirm the effect on concentration of cytosolic calcium by the changes of RYR, SERCA, and PLN. The experiment method is as follows. The isolated muscle cells were grown on a culture plate with an attached glass slide through primary culture, and then differentiated into myotube while being treated with 300 nM of PPAR δ agonist (CMDD1111). Flou4 (final concentration: 5µM) dye reagent was put into the differentiated muscle cells, which were then cultured for 30 minutes. The calcium-free Kreb solution was used as a culture medium to measure the change of cytosolic calcium, and treatment with 10 mM of caffeine was conducted to activate calcium channels. Intracellular calcium ion change was measured by using a confocal microscope. As the measurement result, the retention time of cytosolic calcium was increased by 43% (592 ± 60 versus 847 ± 77 sec) in the muscle cell to which PPAR δ agonist (CMDD1111) was added (FIG. 13). The reason was that expressions of RYR regulating calcium efflux and PLN suppressing calcium influx in the sarcoplasmic membrane were increased by PPAR δ. It has been previously found that the concentration of cytosolic calcium ions has effects of generating force or suppressing fatigue in the muscle cell and, particularly, promotes generation of slow muscle during the muscular remodeling procedure. Therefore, it seems that the mice subjected to fetal reprogramming through PPAR δ activation according to the present invention has an increase in slow muscle fiber generation due to the influence of the increase in cytosolic calcium ions.

<Example 4> Verification on Endurance Increase Effect of Mice Subjected to Fetal Reprogramming Through PPAR δ Activation

The slow muscle fiber has great lipolytic capacity and has tolerance to fatigue due to exercise, and thus is appropriate in the exercise needing endurance. The present inventors conducted an experiment using a treadmill in order to measure an endurance increase effect of mice subjected to fetal reprogramming by PPAR δ agonist.

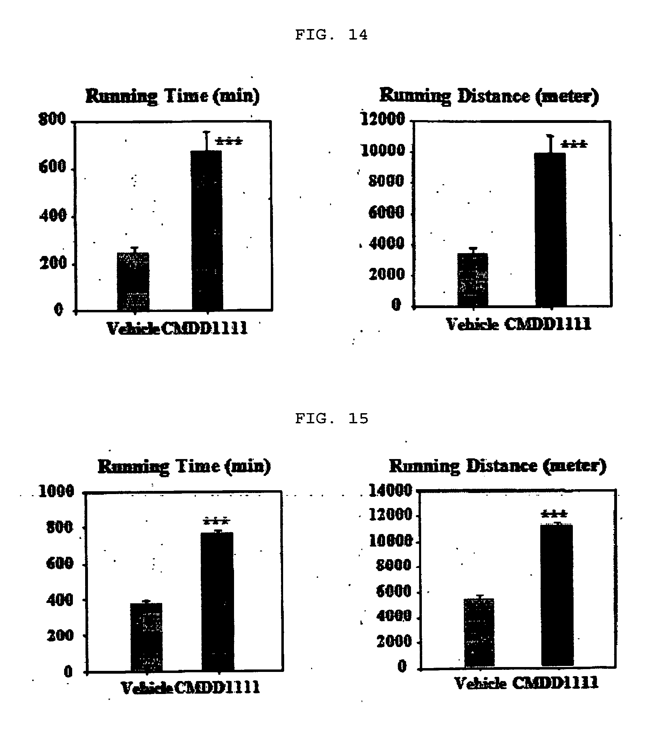

Before the experiment, mice were exercised for 5 minutes at a speed of 7 meters/min for two days so as to be adapted to exercising equipment. On day 3, the main experiment was conducted. As for the method for experiment, the mice were made to run at a speed of 10 meter/min for 60 minutes, and after that, the speed was increased at 1 meter/min once every 15 minutes until the maximum speed reaches 15 meters/min. As for the male mice, the running time was increased by 2.9 fold (235 ± 26 versus 677 ± 78 min, P < 0.0001, n = 6) and the running distance was increased by 3.0 fold (9927 ± 1168 versus 3303 ± 394 m, P < 0.0001) in the PPAR δ agonist (CMDD1111)-administered mouse group than the control mouse group. As for the female mice, the running time was increased by 2.0 fold (376 ± 19 versus 761 ± 17 min, P < 0.0001, n = 4) and the running distance was also increased by 2.1 fold (5419 ± 291 versus 11194 ± 261 m, P < 0.0001), which showed the same tendency as the results for the male mice (FIG. 14 and 15).

<Example 5> Measurement of Respiratory Exchange Ratio (RER) of Mice Subjected to Fetal Reprogramming Through PPAR δ Activation

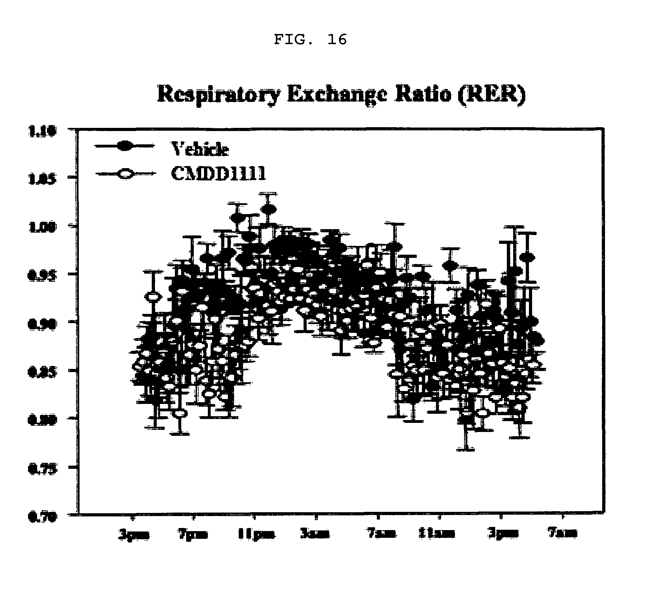

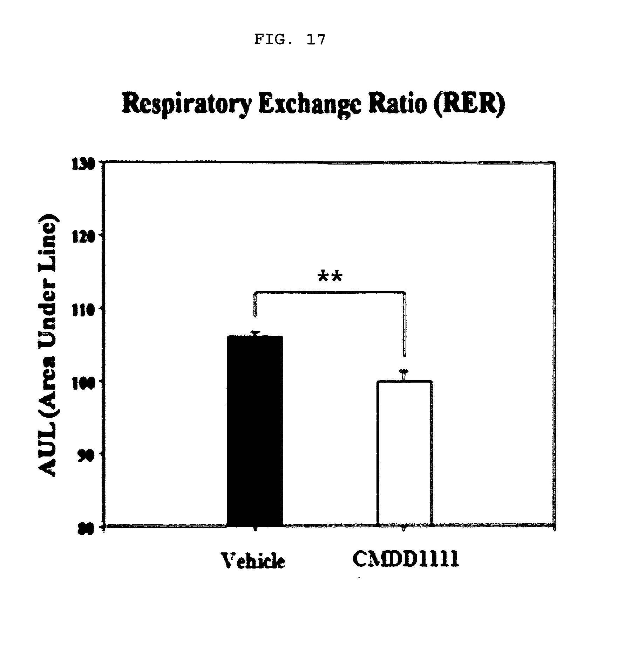

RER is a ratio of the amount of CO₂ exhaled to O₂ inhaled in one breath. The RER is 1.0 when carbohydrate is mainly used for energy production, while the RER is 0.7 when fat is used. In the present invention, the slow muscle fibers of the mice subjected to fetal reprogramming was increasingly generated. The RER experiment using Oximax (Columbus) was conducted in order to evaluate whether or not this increase in slow muscle fiber generation actually influenced mouse energy metabolism. As a result, it may be seen that, the vehicle-administered mouse group had a smaller RER value than the PPAR δ agonist (CMDD1111)-administered mouse group, and thus used more fat than carbohydrate (FIG. 16). Since the RER has a periodic value depending on the time, the RER difference between the two groups was more clearly compared by using AUL, in order to check the RER difference between the two groups. The average AUL value of the control group was 107.4 ± 1.4. Whereas, the average AUL value of the mouse group subjected to fetal reprogramming was 102.2 ± 2.8, which was lower than that of the mouse control group (FIG. 17).

The present inventors checked a difference in lipid oxidation capacity between the two groups by using the following equations.

Carbohydrate oxidation = (4.51.RER - 3.18).VO₂

Lipid oxidation = 1.67.(1-RER).VO₂

As the calculation result, it may be seen that the mouse group to which PPAR δ agonist (CMDD1111) was administered during the fetal programming period used more lipid by average 44% (6.6 mg/kgₘᵢₙ vs. 9.5 mg/kgₘᵢₙ) but less carbohydrate by 20% (48 mg/kgₘᵢₙ vs. 38 mg/kgₘᵢₙ), as compared with the control group to which vehicle was administered. Therefore, it may be seen that the mice in which PPAR δ was activated during the fetal programming period had increased slow muscle fiber generation and thus increased the lipometabolism.

<Example 6> Verification on Obesity and Diabetes Prevention/Suppression Effect of Mice Subjected to Fetal Reprogramming Through PPAR δ Activation

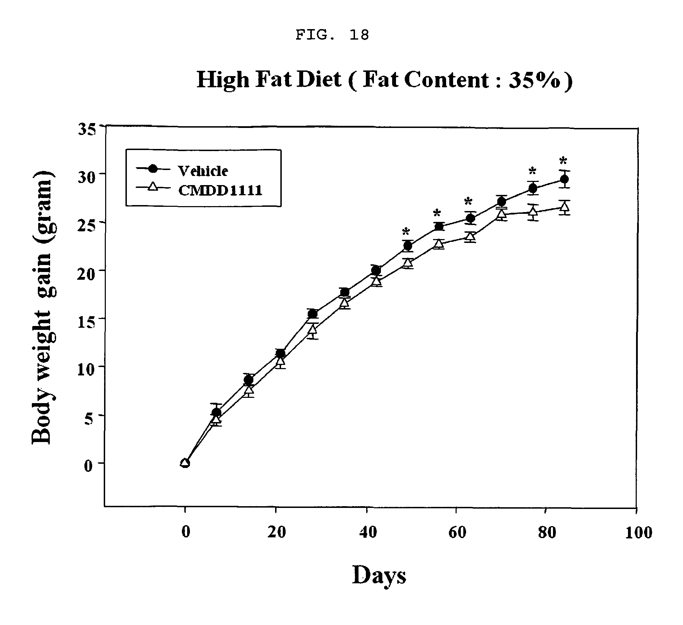

Even though the mice subjected to fetal reprogramming by PPAR δ according to the present invention were administered with the PPAR δ agonist compound only during the gestation and lactation periods, lipometabolitic capacity thereof was continuously increased even as adults. Lipometabolism has deep correlation with the occurrence of obesity and diabetes. Therefore, the present inventors checked the fetal reprogramming effect of PPAR δ on the occurrence of metabolic syndrome. The control group and administration group of 13-week old mice were fed with high-fat feedstuff (fat content: 35%) over a total of 84 days while being weighed once per week. As a result, it may be seen that, the body weight gain was suppressed by 8% in the mice subjected to fetal reprogramming by PPAR δ agonist compound (CMDD1111) as compared with the control group, as shown in FIG. 18.

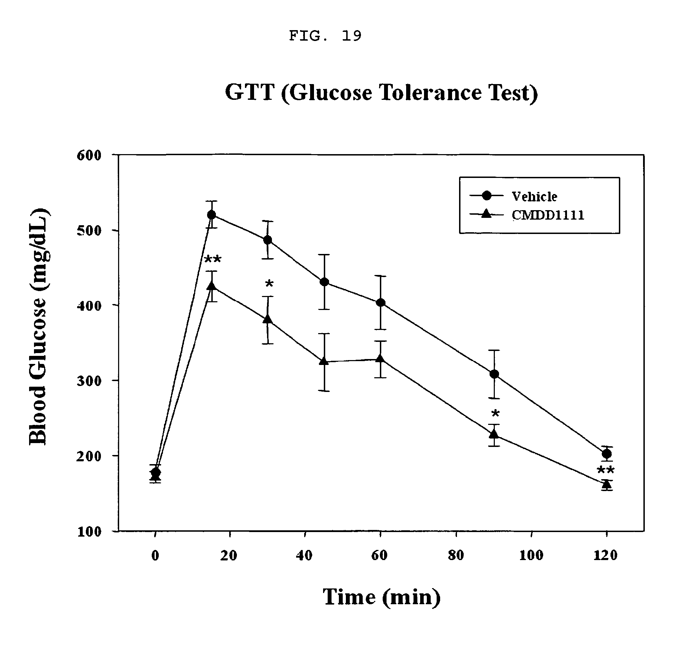

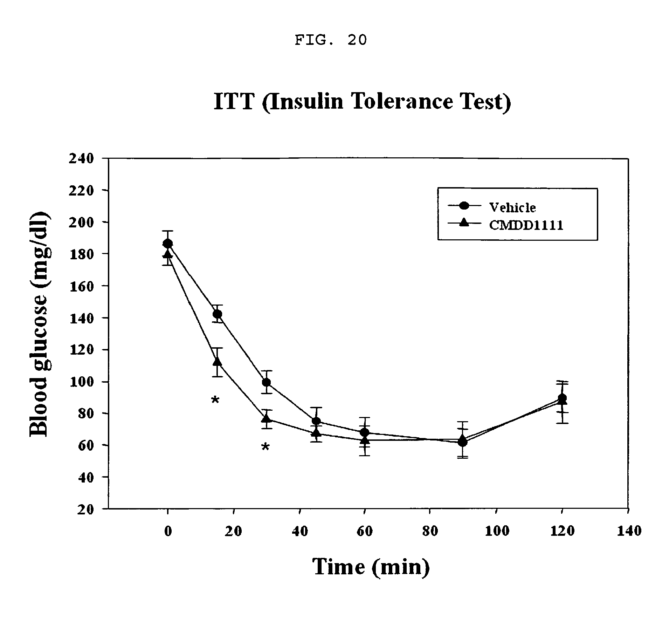

The present researchers conducted the glucose tolerance test (GTT) after the mice were fed with high-fat feedstuff for 17 weeks to induce diabetes. The experiment was conducted following the method proposed by Kelly, and challenged with over-night fasting in order to confirm the role thereof in the muscle. Glucose (1 mg/kg) was administered by using intraperitoneal injection, and then the blood glucose level change was measured for 2 hours. As a result, the fasting blood glucose level was lower, the maximum blood glucose concentration was lower, and the glucose clearance time was shorter in the mice of the administration group as compared with the mice of the control group (FIG. 19). At the same time, the insulin tolerance test was conducted (FIG. 20). To sum the above results up, it may be seen that animals subjected to fetal reprogramming by PPAR δ activation enhanced endurance due to an increase in slow muscle fibers and prevented and suppressed the occurrence of obesity and diabetes due to high-fat diet.

<Example 7> Verification on Memory Improvement Effect of Mice Subjected to Fetal Reprogramming Through PPAR δ Activation

An animal experiment was conducted in order to verify a memory improvement effect of the mice subjected to fetal reprogramming by PPAR δ agonist. Pregnant mice were orally administered with PPAR δ agonist, CMDD1111, at a concentration of 10 mg/kg/day during the gestation and lactation periods in order to verify effects of the invented material at the brain formation stage. When the fetus became an 8-week old adult, the Morris water maze test was conducted to compare memory between the control group and the administration group. This test can check special learning and memory, and is a task that is mainly dependent on the hippocampus in the brain. As the experiment result, the average times required to find the flatform were 26 seconds and 22 seconds (male and female, respectively) for the control group and were 9 seconds and 6 seconds (male and female, respectively) for the administration group. Therefore, it was verified that the PPAR δ agonist improved memory during the fetal programming period, and this effect was maintained until the adulthood.

**[Table 1]**

| Water Maze Test Results | | |
|---|---|---|
| Experiment Group | | Morris water maze test results |
| Vehicle | Male | 26 seconds |
| | Female | 22 seconds |
| CMDD1111 | Male | 9 seconds |
| | Female | 6 seconds |

<Example 8> Verification on Fetal Programming Effects of PPAR δ Agonists Having Different Structures

In order to complete the present invention, the fetal programming effects of PPAR δ agonists having different chemical structures from the above-mentioned CMDD111 was checked.

(1) Verification on Fetal Reprogramming Effect Using PPAR δ Agonist, CMDD1226

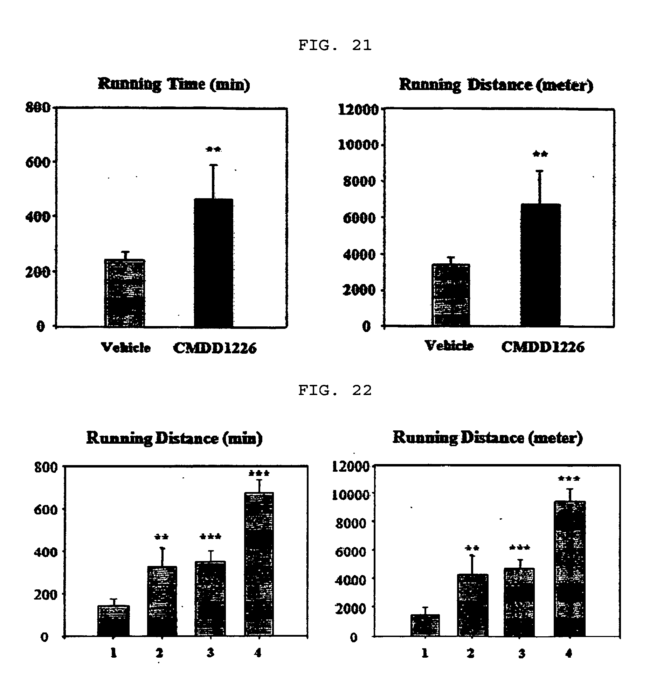

The Experiment using pregnant C57BL/6 mice was conducted in order to verify an endurance enhancement effect by fetal reprogramming of CMDD1226. The pregnant mice were administered with CMDD1226 at a concentration of 10 mg/kg/day every day during the gestation and lactation periods, and the endurances of the fetuses when being 7-week old adults were measured by using a treadmill. As a result, the group subjected to fetal reprogramming had a 1.9-fold increase in the running time and a 2.0-fold increase in the running distance as compared with the control group, and the endurance increase effect by the PPAR δ agonist was verified (FIG. 21).

(2) Fetal Programming Using GW501516 (verification of effect and determination of optimum administration period)

The experiment for verifying the fetal reprogramming effect of GW501516 and determining the optimum administration period thereof was conducted. The experiment method was shown in FIG. 1, and the experiment was conducted with respect to three kinds of administration periods (gestation period, lactation period, and both of gestation and lactation periods). As the experiment result, the group administered with PPAR δ agonist only during the gestation period had 2.6-fold and 3.2-fold increases in the running time and the running distance, respectively, and the group administered with PPAR δ agonist only during the lactation period had 2.4-fold and 3.0-fold increases in the running time and the running distance, respectively, as compared with the control group. The group administered with PPAR δ agonist during both the gestation and lactation periods had the most obvious increase in endurance, and had 5.1-fold and 6.6-fold increases as compared with the control group (FIG. 22).

(3) Fetal Programming Using PPAR δ Agonist, CMDD1112 (verification of effect and determination of optimum administration concentration)

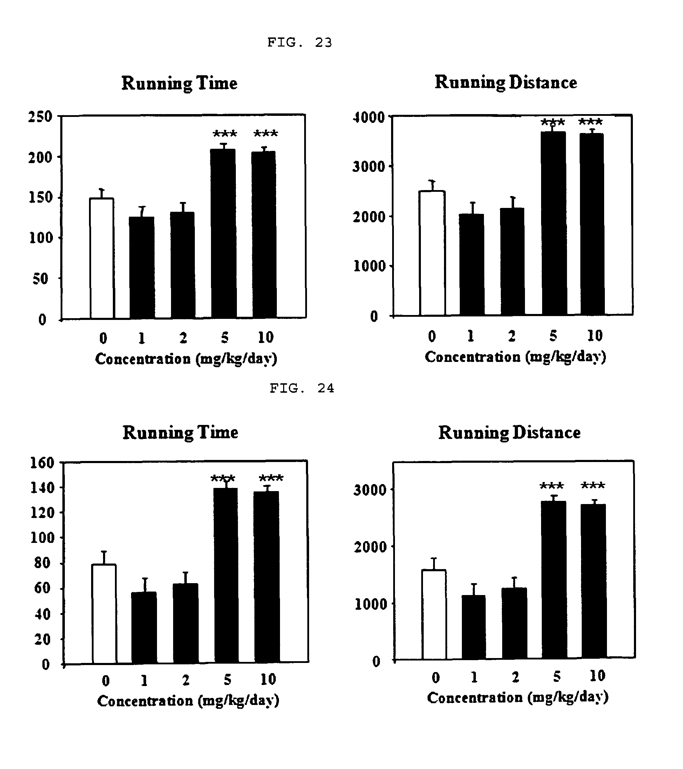

In order to verify a fetal reprogramming effect of CMDD1112 having superior selectivity to PPAR δ as compared with GW501516 and determine the optimum administration concentration of the PPAR δ agonist, the experiment was conducted by using four kinds of concentrations (1, 2, 5, 10 mg/kg/day). As the experiment result, it was confirmed that the endurance enhancement effect was not exhibited at the concentrations of 1 and 2 mg/kg/day and the endurance was enhanced (running time: 1.4 fold, running distance: 1.5 fold) only at the concentration of 5 mg/kg/day (FIGS. 23 and 24).

In the present invention, as the result of animal fetal reprogramming by PPAR δ agonist, the experiment was conducted for three kinds of materials having different structures in order to check the endurance enhancement effect. The experiment results showed that all of the three kinds of PPAR δ agonists exhibited an endurance enhancement effect, and thus, the PPAR δ agonists can have effects of increasing slow muscle generation, enhancing endurance, preventing metabolic syndromes such as obesity, diabetes, arteriosclerosis, and fatty liver, and improving memory. As the experiment results using GW501516, superior effects may be confirmed when PPAR δ agonist was administered during both of the gestation and lactation periods. In addition, as the experiment results using CMDD1112, the effect was observed only at the concentration of 5 mg/kg/day or more in the procedure of fetal reprogramming. However, since all the agonists have different activation concentrations, materials having different structures can have lower activation concentrations.

<Example 9> Verification on Diabetes Disease Prevention/Suppression Effect by Fetal Reprogramming in Type II Diabetes Mouse Model

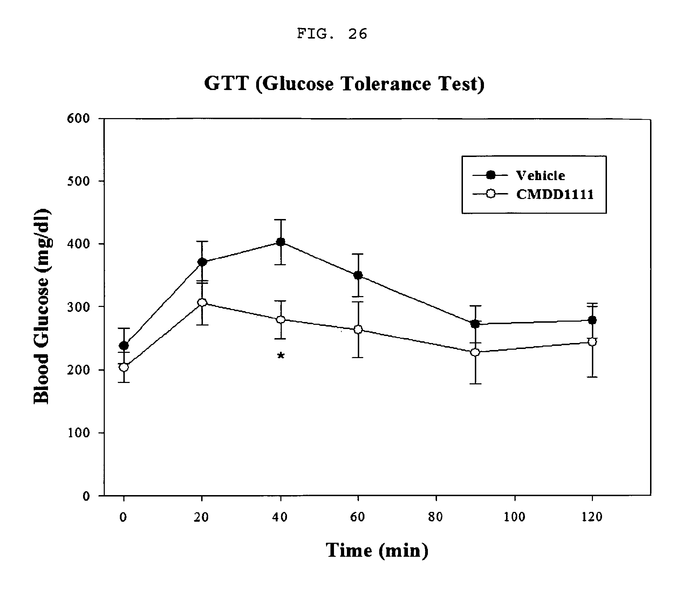

It was confirmed that the fetal reprogramming method of PPAR δ developed by the present invention prevented/suppressed the occurrence of diabetes even in db/db mice having congenital diabetes due to genetic defects. The present researchers have checked the fetal reprogramming effect of PPAR δ by using a leptin receptor-deficient mouse (db/db). After hetero type female and male mice were bred, the PPAR δ agonist (CMDD1111) was administered to the female mice only during the lactation period. The glucose tolerance test (GTT) was conducted using only homo type male fetuses having induced diabetes through gene analysis and blood glucose analysis. As the result, the glucose tolerance was improved by fetal reprogramming of PPAR δ as shown in FIG. 26.

<Example 10> Development of Novel PPAR δ Agonist for Fetal Reprogramming

As described above, the PPAR δ agonist can increase slow muscle fiber generation of the fetus, enhance endurance, in advance prevent metabolic diseases such as obesity, diabetes, arteriosclerosis, and fatty liver, and improve memory, by fetal reprogramming mammals including human beings.

In addition, the present invention developed a new PPAR δ agonist for mammal fetal reprogramming, and verified *in* vitro and *in vivo* effects of this material.

**2. Development of PPAR δ Agonist**

In the present invention, a novel PPAR δ agonist represented by Chemical Formula V below was developed in order to more efficiently achieve fetal reprogramming.

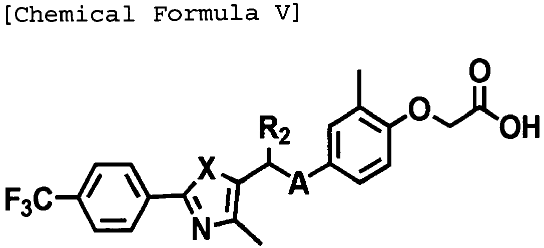

[In Chemical Formula V, A is independently sulfur (S) or selenium (Se);

R₂ is selected from the following structures:

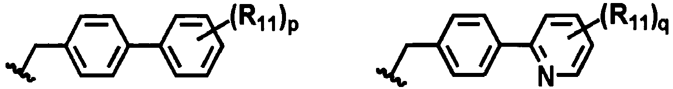

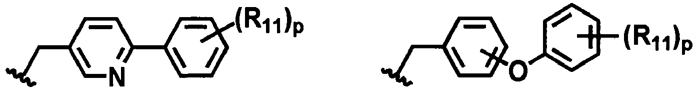

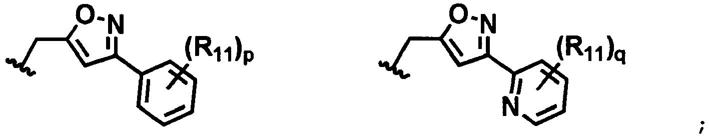

X is sulfur (S) or selenium (Se);

R₁₁ is hydrogen, halogen, C₁-C₅ alkyl, or C₁-C₅ alkoxy;

p is an integer of 1∼5;

q is an integer of 1∼4; and

the alkyl and alkoxy of R₁₁ may be further substituted with at least one halogen or C₁-C₅ alkylamine.]

Hereinafter, the present invention will be described in detail by examples.

However, the following examples are for merely exemplifying the present invention, and therefore, the scope of the present invention is not limited to the following examples.

<Example 11> Preparation of Compound 1

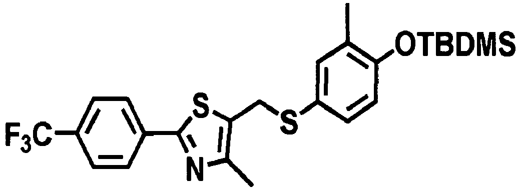

The compound 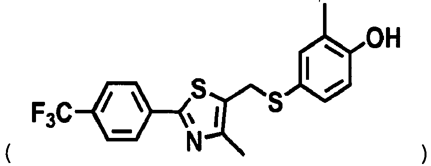 500 mg (1.26 mmol), obtained by using the existing method (WO2006/091047), and imidazole 172 mg (2.0 equivalent) were completely dissolved in 20 ml of dimethyl formamide. *Tert-*butyldimethylsilylchloride 210 mg (1.1 equivalent) was slowly added, and stirred at room temperature for 4 hours. After completion of the reaction, extraction was carried out using an aqueous ammonium chloride solution and ethylacetate, and moisture was removed from the organic layer over magnesium sulfate. After purification using a silica gel column, the solvent was distilled under reduced pressure, to obtain a title compound.

<Example 12> Preparation of Compound 2

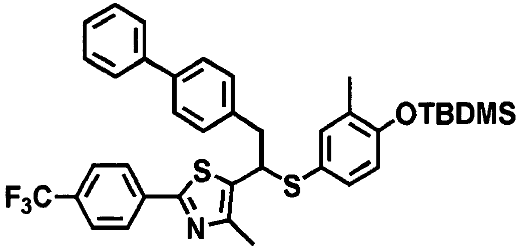

Compound 1 500 mg (0.98 mmol), prepared from Example 11, was dissolved in 20 ml of anhydrous tetrahydrofuran, and the temperature was decreased to -78°C. Lithium diisopropyl amide (LDA) 1.1 mg (1.8M, 2.0 equivalent) was slowly added thereto. After that, 4-phenylbenzyl chloride 199 mg (0.98 mmol) was added to the reaction solution, and the reaction temperature was slowly increased to room temperature. After the reaction for further 30 minutes, the reaction was terminated by an aqueous ammonium chloride solution. The organic solvent was extracted by using ethylacetate and a salt solution, and moisture was removed from the organic layer over magnesium sulfate. After the filtration, the solvent was distilled under reduced pressure, and the residual was purified by silica gel column chromatography, to obtain a title compound.

¹H NMR (300 MHz, CDCl₃) δ 7.98 (t, 2H), 7.65 (t, 2H), 7.52-7.23 (m, 6H), 7.14 (t, 2H), 7.05 (t, 2H), 6.63 (t, 1H), 4.54 (m, 1H), 3.40 (m, 1H), 3.14 (m, 1H), 2.11 (s, 3H), 1.89 (s, 3H), 0.99 (s, 9H), 0.18 (s, 6H)

<Example 13> Preparation of Compound 3

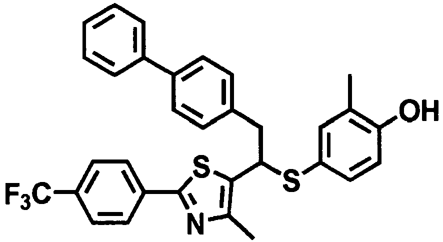

Compound 2 500 mg (0.74 mmol), prepared from Example 12, was completely dissolved in 10 ml of tetrahydrofuran. Tetrabutylammonium fluoride (TBAF) 1.8 ml (a 1M-tetrahydrofuran solution, 2.5 equivalent) was slowly added at room temperature. After the reaction for 30 minutes, extraction was carried out using an aqueous ammonium chloride solution and ethylacetate, and moisture was removed from the organic layer over magnesium sulfate. After the filtration, the solvent was distilled under reduced pressure, and the residual was purified by silica gel column chromatography, to obtain a title compound.

¹H NMR (300 MHz, CDCl₃) δ 7.98 (t, 2H), 7.65 (t, 2H), 7.54∼7.31 (m, 6H), 7.17 (t, 3H), 7.07 (t, 1H), 6.61 (t, 1H), 4.95 (s, 1H), 4.54 (m, 1H), 3.40 (m, 1H), 3.13 (m, 1H), 2.17 (s, 3H), 1.89 (s, 3H)

<Example 14> Preparation of Compound 4

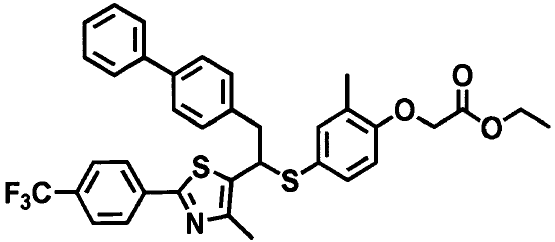

Compound 3 300mg (0.53 mmol), prepared from Example 13, 10 ml of acetone containing 5% water, and potassium carbonate 185mg (0.53 mmol, 2.5 equivalent) were well mixed at room temperature. 71 *µ*ℓ of bromoacetic acid ethyl ester (0.64 mmol, 1.2 equivalent) was added thereto, and then strongly stirred for 4 hours. After termination of the reaction, extraction was carried out by using a salt solution and ethyl acetate, and then moisture was removed over magnesium sulfate. After the filtration, the solvent was distilled under reduced pressure, and the residual was purified by silica gel column chromatography, to obtain a title compound.

¹H NMR (300 MHz, CDCl₃) δ 7.98 (d, 2H), 7.65 (d, 2H), 7.54-7.23 (m, 6H), 7.18 (t, 2H), 7.05 (t, 2H), 6.62 (t, 1H), 4.60 (s, 2H), 4.24 (q, 2H), 4.12 (q, 1H), 3.43 (d, 1H), 3.14 (d, 1H), 2.21 (s, 3H), 2.06 (s, 3H), 1.25 (t, 3H)

<Example 15> Preparation of Compound 5

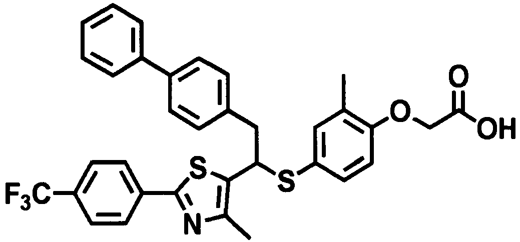

Compound 4 300 mg (0.46 mmol), prepared from Example 14, was well mixed with 15 ml of THF and 10 ml of water, and then further stirred at 0°C for 60 minutes. After termination of the reaction, 2.5 ml of 0.5 M NaHSO₄ was added thereto. Extraction was carried out by using a salt solution and ethyl acetate, followed by filtration. The solvent was distilled under reduced pressure, followed by purification with LH-20 column chromatography, to obtain a title compound.

¹H NMR (300 MHz, CDCl₃) δ 7.95(d, 2H), 7.66(d, 2H), 7.53(d, 2H), 7.46(d, 2H), 7.41(t, 2H), 7.39(t, 1H), 7.26(d, 3H), 7.05(d, 1H), 6.52(d, 1H), 4.56(m, 3H), 3.40(q, 1H), 3.12(q, 1H), 2.15(s, 3H), 1.79(s, 3H)

<Example 16> Preparation of Compound 6

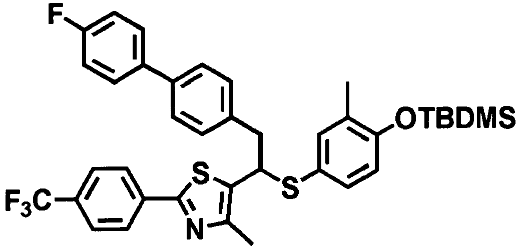

Compound 1 500 mg (0.98 mmol), prepared from Example 11, was dissolved in 20 ml of anhydrous tetrahydrofuran, and the temperature was decreased to -78°C. Lithium diisopropyl amide (LDA) 1.1 mg (1.8M, 2.0 equivalent) was slowly added thereto. After that, 4-(4-fluorophenyl)-benzylchloride 216 mg (0.98 mmol) was added to the reaction solution, and the reaction temperature was slowly increased to room temperature. After the reaction for further 30 minutes, the reaction was terminated by an aqueous ammonium chloride solution. The organic solvent was extracted by using ethylacetate and a salt solution, and moisture was removed from the organic layer over magnesium sulfate. After the filtration, the solvent was distilled under reduced pressure, and the residual was purified by silica gel column chromatography, to obtain a title compound.

¹H NMR (300 MHz, CDCl₃) δ 7.96 (d, 2H), 7.66 (d, 2H), 7.45 (m, 2H), 7.38 (d, 2H), 7.13-7.06 (m, 6H), 6.64 (t, 1H), 4.54 (m, 1H), 3.42 (m, 1H), 3.13 (m, 1H), 2.15 (s, 3H), 1.87 (s, 3H), 0.97 (s, 9H), 0.16 (s, 6H)

<Example 17> Preparation of Compound 7

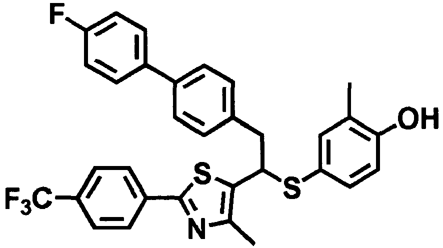

Compound 6 500 mg (0.72 mmol), prepared from Example 16, was completely dissolved in 10 ml of tetrahydrofuran. Tetrabutylammonium fluoride (TBAF) 1.8 ml (a 1M-tetrahydrofuran solution, 2.5 equivalent) was slowly added at room temperature. After the reaction for 30 minutes, extraction was carried out using an aqueous ammonium chloride solution and ethylacetate, and moisture was removed from the organic layer over magnesium sulfate. After the filtration, the solvent was distilled under reduced pressure, and the residual was purified by silica gel column chromatography, to obtain a title compound.

¹H NMR (300 MHz, CDCl₃) δ 7.80 (d, 2H), 7.67 (d, 2H), 7.50 (m, 2H), 7.41 (d, 2H), 7.17~7.05 (m, 6H), 6.63 (t, 1H), 4.95 (s, 1H), 4.54 (m, 1H), 3.40 (m, 1H), 3.13 (m, 1H), 2.17 (s, 3H), 1.89 (s, 3H)

<Example 18> Preparation of Compound 8

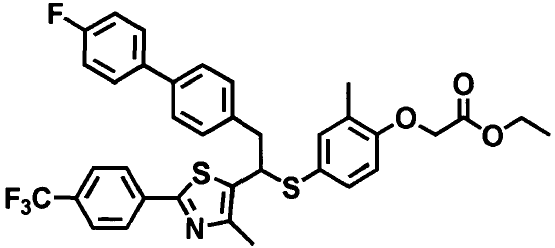

Compound 7 300mg (0.52 mmol), prepared from Example 17, 10 ml of acetone containing 5% water, and potassium carbonate 179 mg (1.29 mmol, 2.5 equivalent) were well stirred at room temperature. 69 *µ*ℓ of bromoacetic acid ethyl ester (0.62 mmol, 1.2 equivalent) was added thereto, and then strongly stirred for 4 hours. After termination of the reaction, extraction was carried out by using a salt solution and ethyl acetate, and then moisture was removed over magnesium sulfate. After the filtration, the solvent was distilled under reduced pressure, and the residual was purified by silica gel column chromatography, to obtain a title compound.

¹H NMR (300 MHz, CDCl₃) δ 7.80 (d, 2H), 7.67 (d, 2H), 7.50 (m, 2H), 7.41 (d, 2H), 7.17-7.05 (m, 6H), 6.63 (t, 1H), 4.59 (s, 2H), 4.55 (q, 1H), 4.23 (q, 2H), 3.40 (q, 1H), 3.11 (q, 1H), 2.20 (s, 3H), 1.87 (s, 3H), 1.26 (t, 3H)

<Example 19> Preparation of Compound 9

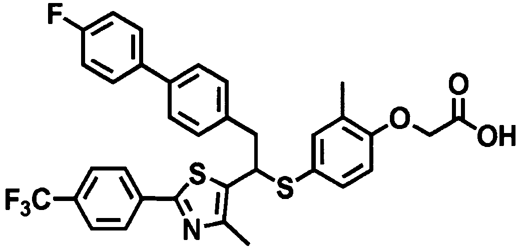

Compound 8 300 mg (0.45 mmol), prepared from Example 18, was well mixed with 15 ml of THF and 10 ml of water, and then further stirred at 0°C for 60 minutes. After termination of the reaction, 2.5 ml of 0.5 M NaHSO₄ was added thereto. Extraction was carried out by using a salt solution and ethyl acetate, followed by filtration. The solvent was distilled under reduced pressure, followed by purification with LH-20 column chromatography, to obtain a title compound.

¹H NMR (300 MHz, CDCl₃) δ 7.76 (d, 2H), 7.46 (d, 2H), 7.37 (m, 2H), 7.31 (d, 2H), 7.04~6.88 (m, 6H), 6.35 (t, 1H), 4.50 (t, 1H), 3.91 (S, 2H), 3.30 (q, 1H), 3.01 (q, 1H), 1.84 (S, 3H), 1.70 (S, 3H)

<Example 20> Preparation of Compound 10

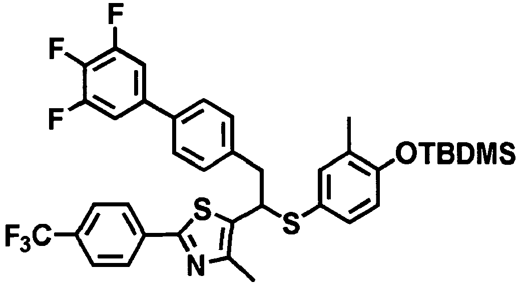

Compound 1 500 mg (0.98 mmol), prepared from Example 11, was dissolved in 20 ml of anhydrous tetrahydrofuran, and the temperature was decreased to -78°C. Lithium diisopropyl amide (LDA) 1.1 ml (1.8M, 2.0 equivalent) was slowly added thereto. After that, 4-(3,4,5-trifluorophenyl)benzylchloride 252 mg (0.98 mmol) was added to the reaction solution, and the reaction temperature was slowly increased to room temperature. After the reaction for further 30 minutes, the reaction was terminated by an aqueous ammonium chloride solution. The organic solvent was extracted by using ethylacetate and a salt solution, and moisture was removed from the organic layer over magnesium sulfate. After the filtration, the solvent was distilled under reduced pressure, and the residual was purified by silica gel column chromatography, to obtain a title compound.

¹H NMR (300 MHz, CDCl₃) δ 7.98 (d, 2H), 7.66 (d, 2H), 7.38 (d, 2H), 7.18~7.04 (m, 6H), 6.63 (t, 1H), 4.54 (m, 1H), 3.41 (q, 1H), 3.13 (q, 1H), 2.11 (s, 3H), 1.88 (s, 3H), 0.99 (s, 9H), 0.18 (s, 6H)

<Example 21> Preparation of Compound 11

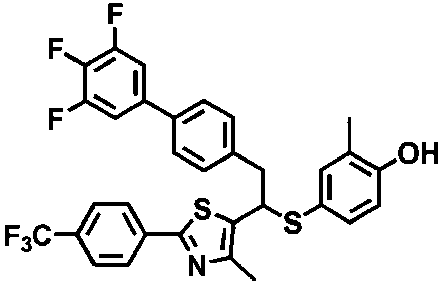

Compound 10 500 mg (0.69 mmol), prepared from Example 20, was completely dissolved in 10 ml of tetrahydrofuran. Tetrabutylammonium fluoride (TBAF) 1.7 ml (a 1M-tetrahydrofuran solution, 2.5 equivalent) was slowly added at room temperature. After the reaction for 30 minutes, extraction was carried out using an aqueous ammonium chloride solution and ethylacetate, and moisture was removed from the organic layer over magnesium sulfate. After the filtration, the solvent was distilled under reduced pressure, and the residual was purified by silica gel column chromatography, to obtain a title compound.

¹H NMR (300 MHz, CDCl₃) δ 7.98 (d, 2H), 7.66 (d, 2H), 7.38 (d, 2H), 7.18~7.04 (m, 6H), 6.63 (t, 1H), 5.01 (s, 1H), 4.53 (m, 1H), 3.41 (q, 1H), 3.13 (q, 1H), 2.15 (s, 3H), 1.86 (s, 3H)

<Example 22> Preparation of Compound 12

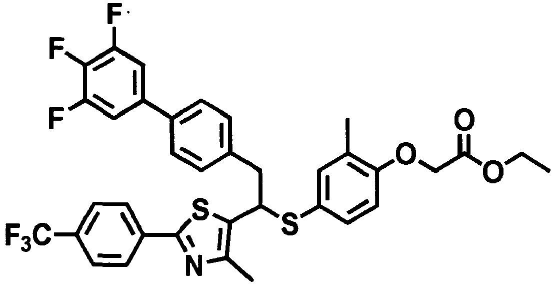

Compound 11 300mg (0.49 mmol), prepared from Example 21, 10 ml of acetone containing 5% water, and potassium carbonate 146 mg (1.29 mmol, 2.5 equivalent) were well stirred at room temperature. 65 *µ*ℓ of bromoacetic acid ethyl ester (0.58 mmol, 1.2 equivalent) was added thereto, and then strongly stirred for 4 hours. After termination of the reaction, extraction was carried out by using a salt solution and ethyl acetate, and then moisture was removed over magnesium sulfate. After the filtration, the solvent was distilled under reduced pressure, and the residual was purified by silica gel column chromatography, to obtain a title compound.

¹H NMR (300 MHz, CDCl₃) δ 7.98 (d, 2H), 7.66 (d, 2H), 7.38 (d, 2H), 7.17-7.06 (m, 6H), 6.55 (t, 1H), 4.57 (s, 2H), 4.53 (q, 1H), 4.23 (q, 2H), 3.41 (q, 1H), 3.13 (q, 1H), 2.20 (s, 3H), 1.88 (s, 3H), 1.25 (t, 3H)

<Example 23> Preparation of Compound 13

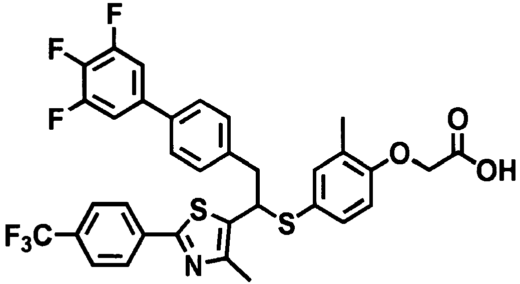

Compound 12 300 mg (0.43 mmol), prepared from Example 22, was well mixed with 15 ml of THF and 10 ml of water, and then further stirred at 0°C for 60 minutes. After termination of the reaction, 2.5 ml of 0.5 M NaHSO₄ was added thereto. Extraction was carried out by using a salt solution and ethyl acetate, followed by filtration. The solvent was distilled under reduced pressure, followed by purification with LH-20 column chromatography, to obtain a title compound.

¹H NMR (300 MHz, CDCl₃) δ 7.98 (d, 2H), 7.66 (d, 2H), 7.38 (d, 2H), 7.17-7.06 (m, 6H), 6.55 (t, 1H), 4.57 (s, 2H), 4.51 (t, 1H), 4.20 (s, 2H), 3.33 (q, 1H), 3.06 (q, 1H), 1.96 (s, 3H), 1.72 (s, 3H)

<Example 24> Preparation of Compound 14

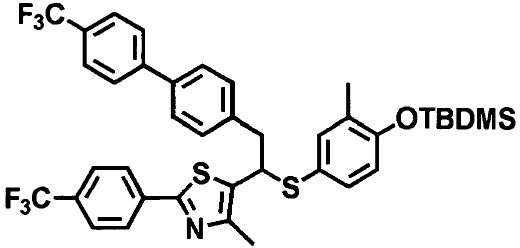

Compound 1 500 mg (0.98 mmol), prepared from Example 11, was dissolved in 20 ml of anhydrous tetrahydrofuran, and the temperature was decreased to -78°C. Lithium diisopropyl amide (LDA) 1.1 ml (1.8M, 2.0 equivalent) was slowly added thereto. After that, 4-(4-trifluorophenyl)phenylbenzylchloride 265 mg (0.98 mmol) was added to the reaction solution, and the reaction temperature was slowly increased to room temperature. After the reaction for further 30 minutes, the reaction was terminated by an aqueous ammonium chloride solution. The organic solvent was extracted by using ethylacetate and a salt solution, and moisture was removed from the organic layer over magnesium sulfate. After the filtration, the solvent was distilled under reduced pressure, and the residual was purified by silica gel column chromatography, to obtain a title compound.

<Example 25> Preparation of Compound 15

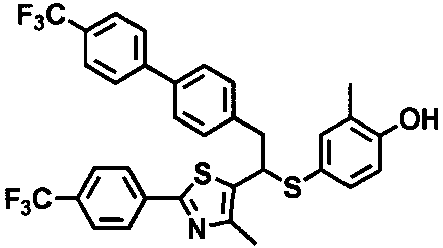

Compound 14 500 mg (0.67 mmol), prepared from Example 24, was completely dissolved in 10 ml of tetrahydrofuran. Tetrabutylammonium fluoride (TBAF) 1.68 ml (a 1M-tetrahydrofuran solution, 2.5 equivalent) was slowly added at room temperature. After the reaction for 30 minutes, extraction was carried out using an aqueous ammonium chloride solution and ethylacetate, and moisture was removed from the organic layer over magnesium sulfate. After the filtration, the solvent was distilled under reduced pressure, and the residual was purified by silica gel column chromatography, to obtain a title compound.

<Example 26> Preparation of Compound 16

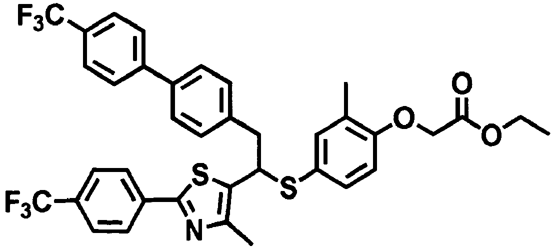

Compound 15 300mg (0.48 mmol), prepared from Example 25, 10 ml of acetone containing 5% water, and potassium carbonate 164 mg (1.19 mmol, 2.5 equivalent) were well stirred at room temperature. 63 *µ*ℓ of bromoacetic acid ethyl ester (0.57 mmol, 1.2 equivalent) was added thereto, and then strongly stirred for 4 hours. After termination of the reaction, extraction was carried out by using a salt solution and ethyl acetate, and then moisture was removed over magnesium sulfate. After the filtration, the solvent was distilled under reduced pressure, and the residual was purified by silica gel column chromatography, to obtain a title compound.

<Example 27> Preparation of Compound 17

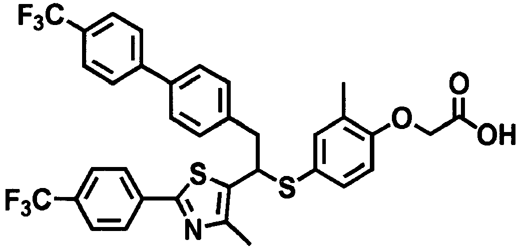

Compound 16 300 mg (0.42 mmol), prepared from Example 26, was well mixed with 15 ml of THF and 10 ml of water, and then further stirred at 0°C for 60 minutes. After termination of the reaction, 2.5 ml of 0.5 M NaHSO₄ was added thereto. Extraction was carried out by using a salt solution and ethyl acetate, followed by filtration. The solvent was distilled under reduced pressure, followed by purification with LH-20 column chromatography, to obtain a title compound.

¹H NMR (300 MHz, CDCl₃) δ 7.77 (d, 2H), 7.59 (d, 2H), 7.50 (m, 4H), 7.38 (d, 2H), 7.10 (d, 2H), 6.90 (m, 2H), 6.38 (t, 1H), 4.50 (t, 1H), 4.07 (s, 2H), 3.33 (q, 1H), 3.06 (q, 2H), 1.69 (s, 3H), 1.24 (s, 3H)

<Example 28> Preparation of Compound 18

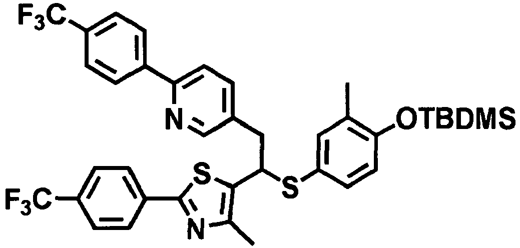

Compound 1 500 mg (0.98 mmol), prepared from Example 11, was dissolved in 20 ml of anhydrous tetrahydrofuran, and the temperature was decreased to -78°C. Lithium diisopropyl amide (LDA) 1.1 ml (1.8M, 2.0 equivalent) was slowly added thereto. After that, 5-(chloromethyl)-2-(4-(trifluoromethyl)phenyl)pyridine 266 mg (0.98 mmol) was added to the reaction solution, and the reaction temperature was slowly increased to room temperature. After the reaction for further 30 minutes, the reaction was terminated by an aqueous ammonium chloride solution. The organic solvent was extracted by using ethylacetate and a salt solution, and moisture was removed from the organic layer over magnesium sulfate. After the filtration, the solvent was distilled under reduced pressure, and the residual was purified by silica gel column chromatography, to obtain a title compound.

<Example 29> Preparation of Compound 19

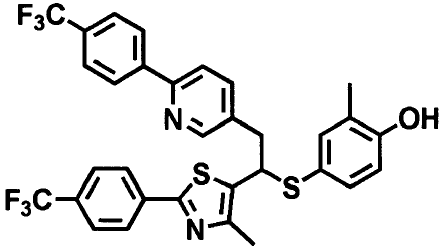

Compound 18 500 mg (0.67 mmol), prepared from Example 28, was completely dissolved in 10 ml of tetrahydrofuran. Tetrabutylammonium fluoride (TBAF) 1.68 ml (a 1M-tetrahydrofuran solution, 2.5 equivalent) was slowly added at room temperature. After the reaction for 30 minutes, extraction was carried out using an aqueous ammonium chloride solution and ethylacetate, and moisture was removed from the organic layer over magnesium sulfate. After the filtration, the solvent was distilled under reduced pressure, and the residual was purified by silica gel column chromatography, to obtain a title compound.

<Example 30> Preparation of Compound 20

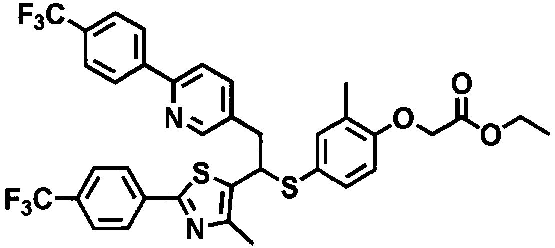

Compound 19 300mg (0.48 mmol), prepared from Example 29, 10 ml of acetone containing 5% water, and potassium carbonate 164 mg (1.19 mmol, 2.5 equivalent) were well stirred at room temperature. 63 *µ*ℓ of bromoacetic acid ethyl ester (0.57 mmol, 1.2 equivalent) was added thereto, and then strongly stirred for 4 hours. After termination of the reaction, extraction was carried out by using a salt solution and ethyl acetate, and then moisture was removed over magnesium sulfate. After the filtration, the solvent was distilled under reduced pressure, and the residual was purified by silica gel column chromatography, to obtain a title compound.

<Example 31> Preparation of Compound 21

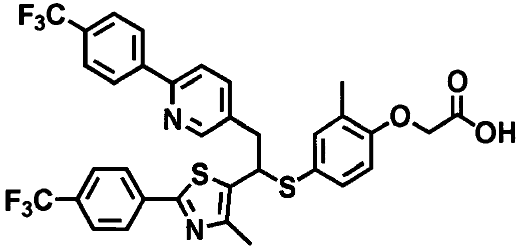

Compound 20 300 mg (0.42 mmol), prepared from Example 30, was well mixed with 15 ml of THF and 10 ml of water, and then further stirred at 0°C for 60 minutes. After termination of the reaction, 2.5 ml of 0.5 M NaHSO₄ was added thereto. Extraction was carried out by using a salt solution and ethyl acetate, followed by filtration. The solvent was distilled under reduced pressure, followed by purification with LH-20 column chromatography, to obtain a title compound.

¹H NMR (300 MHz, CDCl₃) δ 8.38 (s, 1H), 7.94 (d, 2H), 7.78 (d, 2H), 7.61~7.47 (m, 5H), 7.33 (d, 1H), 6.89 (d, 2H), 6.38 (t, 1H), 4.46 (t, 1H), 4.04 (s, 2H), 3.27 (q, 1H), 3.02 (q, 2H), 1.84 (s, 3H), 1.75 (s, 3H)

<Example 32> Preparation of Compound 22

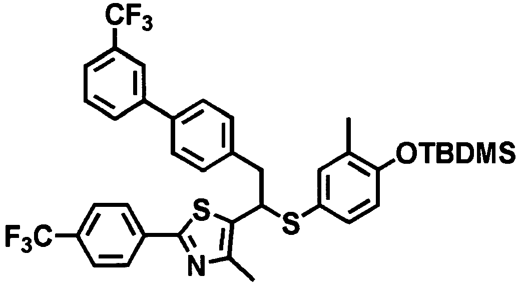

Compound 1 500 mg (0.98 mmol), prepared from Example 11, was dissolved in 20 ml of anhydrous tetrahydrofuran, and the temperature was decreased to -78°C. Lithium diisopropyl amide (LDA) 1.1 mg (1.8M, 2.0 equivalent) was slowly added thereto. After that, 4-(3-trifluorophenyl)phenylbenzylchloride 266 mg (0.98 mmol) was added to the reaction solution, and the reaction temperature was slowly increased to room temperature. After the reaction for further 30 minutes, the reaction was terminated by an aqueous ammonium chloride solution. The organic solvent was extracted by using ethylacetate and a salt solution, and moisture was removed from the organic layer over magnesium sulfate. After the filtration, the solvent was distilled under reduced pressure, and the residual was purified by silica gel column chromatography, to obtain a title compound.

<Example 33> Preparation of Compound 23

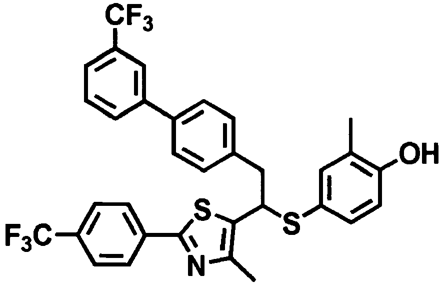

Compound 22 500 mg (0.67 mmol), prepared from Example 32, was completely dissolved in 10 ml of tetrahydrofuran. Tetrabutylammonium fluoride (TBAF) 1.68 ml (a 1M-tetrahydrofuran solution, 2.5 equivalent) was slowly added at room temperature. After the reaction for 30 minutes, extraction was carried out using an aqueous ammonium chloride solution and ethylacetate, and moisture was removed from the organic layer over magnesium sulfate. After the filtration, the solvent was distilled under reduced pressure, and the residual was purified by silica gel column chromatography, to obtain a title compound.

<Example 34> Preparation of Compound 24

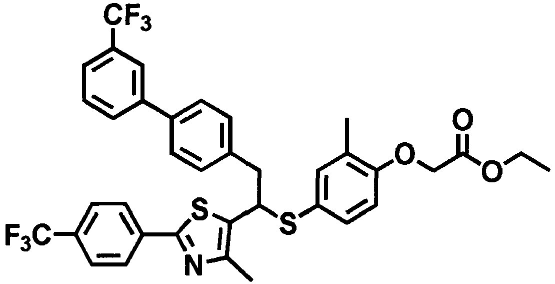

Compound 23 300mg (0.48 mmol), prepared from Example 33, 10 ml of acetone containing 5% water, and potassium carbonate 164 mg (1.19 mmol, 2.5 equivalent) were well stirred at room temperature. 63 *µ*ℓ of bromoacetic acid ethyl ester (0.57 mmol, 1.2 equivalent) was added thereto, and then strongly stirred for 4 hours. After termination of the reaction, extraction was carried out by using a salt solution and ethyl acetate, and then moisture was removed over magnesium sulfate. After the filtration, the solvent was distilled under reduced pressure, and the residual was purified by silica gel column chromatography, to obtain a title compound.

<Example 35> Preparation of Compound 25

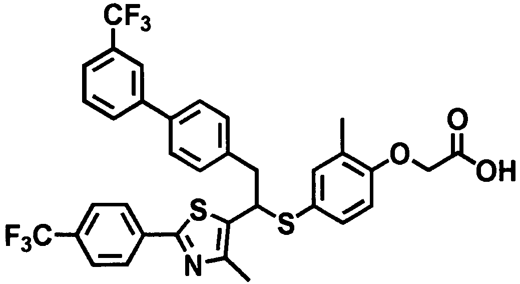

Compound 24 300 mg (0.42 mmol), prepared from Example 34, was well mixed with 15 ml of THF and 10 ml of water, and then further stirred at 0°C for 60 minutes. After termination of the reaction, 2.5 ml of 0.5 M NaHSO₄ was added thereto. Extraction was carried out by using a salt solution and ethyl acetate, followed by filtration. The solvent was distilled under reduced pressure, followed by purification with LH-20 column chromatography, to obtain a title compound.

¹H NMR (300 MHz, CDCl₃) δ 7.96 (d, 2H), 7.78 (s, 1H), 7.68 (m, 3H), 7.58~7.45 (m, 4H), 7.20 (d, 3H), 7.07 (t, 1H), 6.57 (t, 1H), 4.70 (s, 2H), 4.57 (t, 1H), 3.43 (q, 1H), 3.15 (q, 1H), 2.18 (s, 3H), 1.81 (s, 3H)

<Example 36> Preparation of Compound 26

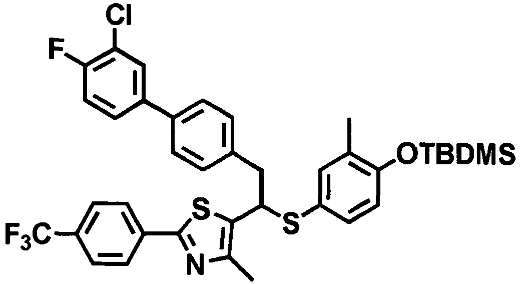

Compound 1 500 mg (0.98 mmol), prepared from Example 11, was dissolved in 20 ml of anhydrous tetrahydrofuran, and the temperature was decreased to -78°C. Lithium diisopropyl amide (LDA) 1.1 ml (1.8M, 2.0 equivalent) was slowly added thereto. After that, 4-(3-chloro-4-trifluoromethyl)phenylbenzylchloride 250 mg (0.98 mmol) was added to the reaction solution, and the reaction temperature was slowly increased to room temperature. After the reaction for further 30 minutes, the reaction was terminated by an aqueous ammonium chloride solution. The organic solvent was extracted by using ethylacetate and a salt solution, and moisture was removed from the organic layer over magnesium sulfate. After the filtration, the solvent was distilled under reduced pressure, and the residual was purified by silica gel column chromatography, to obtain a title compound.

<Example 37> Preparation of Compound 27

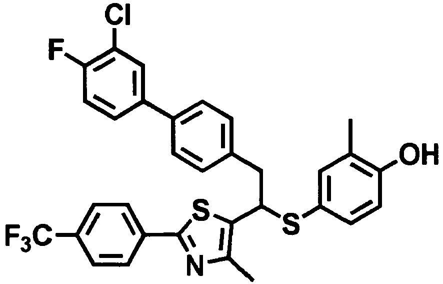

Compound 26 500 mg (0.69 mmol), prepared from Example 36, was completely dissolved in 10 ml of tetrahydrofuran. Tetrabutylammonium fluoride (TBAF) 1.72 ml (a 1M-tetrahydrofuran solution, 2.5 equivalent) was slowly added at room temperature. After the reaction for 30 minutes, extraction was carried out using an aqueous ammonium chloride solution and ethylacetate, and moisture was removed from the organic layer over magnesium sulfate. After the filtration, the solvent was distilled under reduced pressure, and the residual was purified by silica gel column chromatography, to obtain a title compound.

¹H NMR (300 MHz, CDCl₃) δ 7.96 (d, 2H), 7.67 (d, 2H), 7.56 (q, 1H), 7.37 (m, 3H), 7.22~7.05 (m, 5H), 6.58 (t, 1H), 5.17 (s, 1H), 4.54 (q, 1H), 3.42 (q, 1H), 3.13 (q, 1H), 2.17 (s, 3H), 1.87 (s, 3H)

<Example 38> Preparation of Compound 28

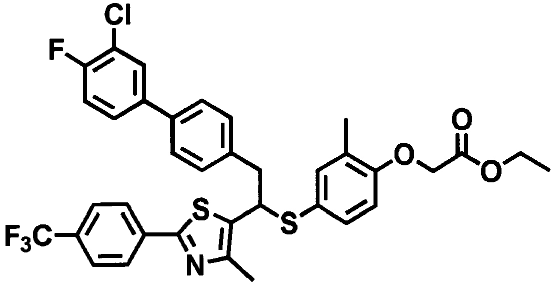

Compound 27 300mg (0.49 mmol), prepared from Example 37, 10 ml of acetone containing 5% water, and potassium carbonate 169 mg (1.19 mmol, 2.5 equivalent) were well stirred at room temperature. 65 *µ*ℓ of bromoacetic acid ethyl ester (0.59 mmol, 1.2 equivalent) was added thereto, and then strongly stirred for 4 hours. After termination of the reaction, extraction was carried out by using a salt solution and ethyl acetate, and then moisture was removed over magnesium sulfate. After the filtration, the solvent was distilled under reduced pressure, and the residual was purified by silica gel column chromatography, to obtain a title compound.

<Example 39> Preparation of Compound 29

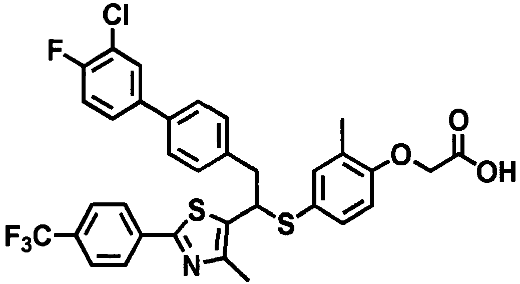

Compound 28 300 mg (0.43 mmol), prepared from Example 38, was well mixed with 15 ml of THF and 10 ml of water, and then further stirred at 0°C for 60 minutes. After termination of the reaction, 2.5 ml of 0.5 M NaHSO₄ was added thereto. Extraction was carried out by using a salt solution and ethyl acetate, followed by filtration. The solvent was distilled under reduced pressure, followed by purification with LH-20 column chromatography, to obtain a title compound.

¹H NMR (300 MHz, CDCl₃) δ 7.96 (d, 2H), 7.67 (d, 2H), 7.56 (q, 1H), 7.37 (m, 3H), 7.22~7.05 (m, 5H), 6.58 (t, 1H), 4.62 (s, 2H), 4.56 (q, 1H), 3.42 (q, 1H), 3.14 (q, 1H), 2.19 (s, 3H), 1.81 (s, 3H)

<Example 40> Preparation of Compound 30

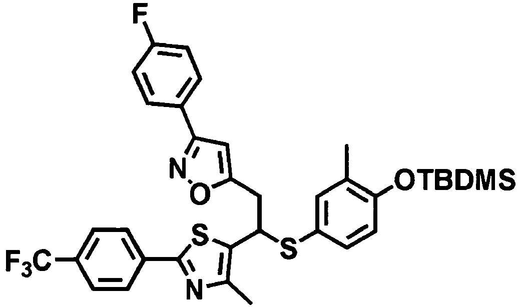

Compound 1 500 mg (0.98 mmol), prepared from Example 11, was dissolved in 20 ml of anhydrous tetrahydrofuran, and the temperature was decreased to -78°C. Lithium diisopropyl amide (LDA) 1.1 ml (1.8M, 2.0 equivalent) was slowly added thereto. After that, 5-(chloromethyl)-3-(4-(trifluoromethyl)phenyl)isoxazole 257 mg (0.98 mmol) was added to the reaction solution, and the reaction temperature was slowly increased to room temperature. After the reaction for further 30 minutes, the reaction was terminated by an aqueous ammonium chloride solution. The organic solvent was extracted by using ethylacetate and a salt solution, and moisture was removed from the organic layer over magnesium sulfate. After the filtration, the solvent was distilled under reduced pressure, and the residual was purified by silica gel column chromatography, to obtain a title compound.

<Example 41> Preparation of Compound 31

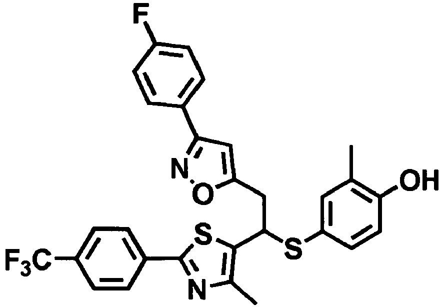

Compound 30 500 mg (0.72 mmol), prepared from Example 40, was completely dissolved in 10 ml of tetrahydrofuran. Tetrabutylammonium fluoride (TBAF) 1.80 ml (a 1M-tetrahydrofuran solution, 2.5 equivalent) was slowly added at room temperature. After the reaction for 30 minutes, extraction was carried out using an aqueous ammonium chloride solution and ethylacetate, and moisture was removed from the organic layer over magnesium sulfate. After the filtration, the solvent was distilled under reduced pressure, and the residual was purified by silica gel column chromatography, to obtain a title compound.

<Example 42> Preparation of Compound 32

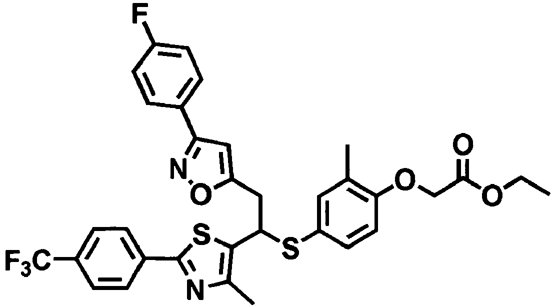

Compound 31 300mg (0.53 mmol), prepared from Example 41, 10 ml of acetone containing 5% water, and potassium carbonate 182 mg (1.19 mmol, 2.5 equivalent) were well stirred at room temperature. 70 *µ*ℓ of bromoacetic acid ethyl ester (0.63 mmol, 1.2 equivalent) was added thereto, and then strongly stirred for 4 hours. After termination of the reaction, extraction was carried out by using a salt solution and ethyl acetate, and then moisture was removed over magnesium sulfate. After the filtration, the solvent was distilled under reduced pressure, and the residual was purified by silica gel column chromatography, to obtain a title compound.

¹H NMR (300 MHz, CDCl₃) δ 7.96 (d, 2H), 7.66 (m, 2H), 7.20~7.08 (m, 4H), 6.58 (t, 1H), 6.22 (s, 1H), 4.61 (s, 2H), 4.57 (t, 1H), 4.25 (m, 2H), 3.42 (q, 1H), 3.14 (q, 1H), 1.88 (s, 3H), 1.57 (s, 3H), 1.27 (t, 3H)

<Example 43> Preparation of Compound 33

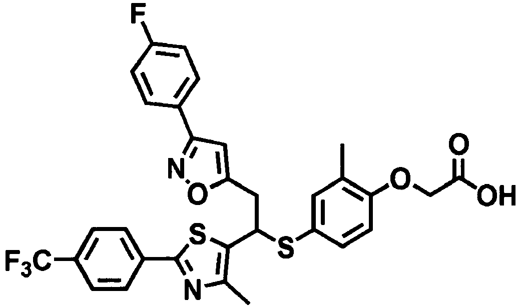

Compound 32 300 mg (0.46 mmol), prepared from Example 34, was well mixed with 15 ml of THF and 10 ml of water, and then further stirred at 0°C for 60 minutes. After termination of the reaction, 2.5 ml of 0.5 M NaHSO₄ was added thereto. Extraction was carried out by using a salt solution and ethyl acetate, followed by filtration. The solvent was distilled under reduced pressure, followed by purification with LH-20 column chromatography, to obtain a title compound.

¹H NMR (300 MHz, CDCl₃) δ 7.96 (d, 2H), 7.66 (m, 2H), 7.20~7.08 (m, 4H), 6.58 (t, 1H), 6.22 (s, 1H), 4.70 (q, 1H), 4.63 (s, 2H), 3.44 (q, 1H), 3.25 (q, 1H), 2.21 (s, 3H), 2.02 (s, 3H)

<Example 44> Verification on in vitro Activity of Developed Material (Transfection assay)

This assay was conducted by using CV-1 cells. The cells were cultured on a 96-well plate with a DMEM medium containing 10 % FBS, DBS (delipidated), and 1 % penicillin/streptomycin in 37 °C of an incubator 5 % of carbon dioxide. Test was conducted in four stages of cell inoculation, transfection, treatment with the developed material, and result confirmation. The CV-1 cells were inoculated on the 96-well plate at a cell density at 5,000 cells/well, and after 24 hours, transfection was performed. PPARs plasmid DNA, reporter DNA, and β-galactosidase DNA were mixed with a transfection reagent, and then used to treat the cells. The developed material was dissolved in dimethylsulfoxide (DMSO), and then was used to treat the cells by using media at various concentrations. After 24 hours of culturing in an incubator, the cells were lysed with a lysis buffer and luciferase activity and β-galactosidase activity were measured using a luminometer and a microplate reader. The measured luciferase value was calibrated with the β-galactosidase value, and this value was depicted on a graph and EC₅₀ was determined.

**[Table 2]**

| EC₅₀ Data | | | |
|---|---|---|---|
| Compound No. | hPPAR δ (nM) | hPPARa | hPPARg |
| 5 | 3.3 | ia | ia |
| 9 | 3.9 | ia | ia |
| 13 | 8.8 | ia | ia |
| 17 | 6.4 | ia | ia |
| 21 | 10.6 | ia | ia |
| 25 | 6.1 | ia | ia |
| 28 | 4.6 | ia | ia |
| 33 | 41.4 | ia | ia |

As can be seen in Table 2, the compound according to the present invention was highly selective for PPAR δ, and has activity of 2-200 nM for PPAR δ.

<Example 45> Verification on *in vivo* Activity of Developed Material

Animal test was conducted in order to confirm the *in vivo* effect of Compounds 5 and 9 exhibiting superior PPAR δ activity from the *In vitro* test results.

(1) Verification of Obesity Suppression Effect

Test using mice was conducted in order to confirm the *in vivo* effect of the material according to the present invention. 8-week old C57BL/6 mice were used, and feedstuff containing 35% fat was used to induce obesity. A vehicle and Compounds 5 and 9 (10 mg/kg/day) were orally administered over a 60-day period of high-fat diet. The test results confirmed that the group administered with Compound 5 had a 35% obesity suppression effect and the group administered with Compound 9 had a 33% obesity suppression effect, as compared with the control group administered with vehicle.

(2) Verification on Diabetes Improvement Effect

The glucose tolerance test (GTT) was conducted in order to verify the diabetes improvement effect of the material according to the present invention. Glucose (1.5 g/kg) was abdominally administered to mice with high-fat-induced diabetes, which was orally administered with medicine over 70 days, and then the blood glucose level change was measured for 2 hours. As the result, the group administered with Compounds 5 and 9 (10mg/Kg/day) showed a rapid decrease in blood glucose level between 20 and 40 minutes as compared with the control group, and showed a complete glucose clearance after 100 minutes. In contrast, the mouse control group administered with the vehicle did not maintain a normal blood glucose level even after 120 minutes. These results verified that the developed materials, Compounds 5 and 9, were effective in improving diabetes.

(3) Hyperlipidemia

High-density lipoprotein cholesterol (HDL) has been known to play an important role in treating lipid metabolic disorders such as arteriosclerosis and hyperlipidemia while lowering the cholesterol level of an epidermal cell and mitigating an inflammatory reaction. Serum was separated from the mice fed over a 6-week period of feedstuff containing 10% fat in order to confirm the high-density cholesterol increase effect of Compounds 5 and 9, which were the developed materials of the present invention. As the result of analyzing the concentration of high-density lipid cholesterol (HDL) in serum, the group administered with GW501516 showed a 29% HDL increase, but the groups administered with Compounds 5 and 9 showed 68% and 62% HDL increases, respectively.

(4) Arteriosclerosis Prevention and Treatment Effect

The *in vivo* test using ApoE-/- mice was conducted in order to verify the arteriosclerosis prevention and treatment effect of Compounds 5 and 9, the developed materials of the present invention. ApoE-/- mice, as an arteriosclerosis disease model, were fed with HDL feedstuff (21% high fat and 1.25% high cholesterol feedstuff) over 14 weeks, to thereby induce arteriosclerosis, and then Compounds 5 and 9 were administered to the mice over the last 4 weeks. The medicine treatment manner was adopted. Arteries of the mice were resected, and then analyzed by the en face method. As the result, it was confirmed that Compounds 5 and 9 had 23% and 18% decreases in occurrence of arteriosclerosis, respectively.

(5) Verification on Muscle endurance Enhancement Effect

The animal test was conducted in order to verify the muscle endurance enhancement and muscular function improvement effects of the material according to the present invention. Compounds 5 and 9 were orally administered at a concentration of 10 mg/kg/day in order to confirm the fetal reprogramming effect. As the result of observing muscle after skin removal, the administration group was redder than the control group. The test using a treadmill was conducted in order to confirm the effect of this muscular fiber change on muscle endurance enhancement and muscular function improvement. As the result, it was confirmed that the developed material significantly increased the running time and the running distance, which verified that the developed material enhanced endurance.

**[Table 3]**

| Endurance Test Results Using Treadmill | | | | | | |
|---|---|---|---|---|---|---|
| Increase Rate (Administration Group/Control Group) | Gestation Period | | Lactation Period | | Gestation+Lactation Periods | |
| | Time | Distance | Time | Distance | Time | Distance |
| Compound 5 | 2.4 fold | 2.5 fold | 2.1 fold | 2.2 fold | 3.6 fold | 3.9 fold |
| Compound 9 | 1.9 fold | 1.9 fold | 1.5 fold | 1.5 fold | 2.8 fold | 3.0 fold |

In addition, it was verified that the developed material was effective in enhancing muscle endurance and improving muscular function. 10-week old C57BL/6 mice were exercised while being orally administered with the developed material at a concentration of 10 mg/kg/day. The mice were exercised using a treadmill for 30 minutes once each day over 30 days. The mice were exercised under exercise conditions of 2 meter/min for 5 minutes, 5 meters/min for 5 minutes, 8 meters/min for 5 minutes, and 20 meters/min for 5 minutes, respectively. By the end of the test, muscle endurance enhancement and muscular function improvement effects were tested by using a treadmill. As the result, the treatment group showed increases in the running time (Compound 5: 1.7 fold, Compound 9: 1.5 fold) and the running distance (Compound 5: 1.8 fold, Compound 9: 1.5 fold) as compared with the control group.

(6) Verification on Memory Improvement Effect

The animal test was conducted in order to verify the memory improvement effect of the material according to the present invention. Compounds 5 and 9, which are the developed materials, were orally administered to the mother at a concentration of 10 mg/kg/day during the fetal reprogramming period. The Morris water maze test was used to confirm the difference in brain function between the control group and the administered group. As the result, the memories of the groups administered with the developed materials were significantly increased (Compound 5: 8.2 seconds, Compound 9: 9.7 seconds), as compared with the control group (Vehicle: 28 seconds).

<Example 46> Preparation of Formulation Containing PPAR δ Agonist as Active Component

The PPAR δ agonist of the present invention may be orally or non-orally administered to mammals including human beings. The composition containing the PPAR δ agonist as an effective component is not limited to a particular formulation, and may be formulated into oral administration, drip liquid, tablet, trituration, liquid suppository, external application, patch, intravenous injection, powder, granule, sugar-coated tablet, capsule, pill, suspension, liquid, ampoule, injection, or the like, and may be applied to any other shape of medicine.

Preparative Example 1: Preparation of Tablet

The tablet was prepared by the conventional method while mixing the following components.

Compound of Chemical Formula 1 (ex.: Compound 5 or 9) 15 mg

Lactose 95 mg

Hydroxypropylcellulose 3 mg

Calcium carboxymethylcellulose 8 mg

Magnesium stearate 0.5 mg

Preparative Example 2: Preparation of Powder

The powder was prepared by the conventional method while mixing the following components.

Compound of Chemical Formula 1 (ex.: Compound 5 or 9) 30 mg

Lactose 20 mg

Starch 15 mg

Magnesium stearate: suitable amount

Preparative Example 3: Preparation of Capsule

The hard capsule was prepared by the conventional method while mixing the following components.

Compound of Chemical Formula 1 (ex.: Compound 5 or 9) 80 mg

Lactose 30 mg

Starch 25 mg

Talc 2 mg

Magnesium stearate: suitable amount

Preparative Example 4: Preparation of Soft Capsule

Contents of the soft capsule were prepared by the conventional method while mixing the following components. The soft capsule was prepared by the conventional method using, per capsule, gelatin 132 mg, concentrated glycerin 50 mg, 70% D-sorbitol 6 mg, appropriate amount of ethylvanillin as fragrance ingredient, and Carnauba Wax as a coating agent.

Compound of Chemical Formula 1 (ex.: Compound 5 or 9) 80 mg

Polyethyleneglycol 400 400mg

Concentrated glycerin 50 mg

Purified water 35 mg

Preparative Example 5: Preparation of Suspension

The suspension was prepared by the conventional method while mixing the following components.

Compound of Chemical Formula 1 (ex.: Compound 5 or 9) 50 mg

Isomerized sugar 10 g

Sugar 25 mg

Calcium carboxymethylcellulose 50 mg

Lemony flavor, appropriate amount

Total preparation amount was adjusted to 100 ml by using purified water.

Preparative Example 6: Preparation of Injection

Each ampoule (2 ml) containing contents of the following components was prepared by the conventional method while mixing the following components.

Compound of Chemical Formula 1 (ex.: Compound 5 or 9) 20 mg

Mannitol 180 mg

Sterile distilled water for injection 2974 mg

Disodium phosphate 26 mg

Preparative Example 7: Preparation of Ointment

Ointments (ointment examples 1 to 3) were prepared by the conventional method while mixing the following components.

[Ointment Example 1]

Compound of Chemical Formula 1 (ex.: Compound 5 or 9) 0.5 wt%

White Petrolatum 70 wt%

Mineral oil 5 wt%

Polyoxylstearyl ether 5 wt%

Polyethylene glycol (ex.: PEG 400 or USP) 19.2 wt%

Butylhydroxyanisol 0.3 wt%

[Ointment Example 2]

Compound of Chemical Formula 1 (ex.: Compound 5 or 9) 0.5 wt%

White Petrolatum 70 wt%

Mineral oil 5 wt%

Polyoxylstearyl ether 5 wt%

Polyethylene glycol (ex.: PEG 400 or USP) 19.2 wt%

Butylhydroxyanisol 0.2 wt%

Paraben (ex.: methylparaben or propylparaben) 0.1 wt%

[Ointment Example 3]

Compound of Chemical Formula 1 (ex.: Compound 5 or 9) 0.01 to 2 wt%

White Petrolatum 30 to 75 wt%

Mineral oil 2 to 10 wt%

Polyoxylstearyl ether 0.3 to 10 wt%

Polyethylene glycol (ex.: PEG 400 or USP) 2 to 45 wt%

Butylhydroxyanisol 0 wt% or 0.002 to 2.5 wt%

Paraben (ex.: methylparaben or propylparaben) 0 wt% or 0.01 to 1.5 wt%

Preparative Example 8: Preparation of Cream

Creams for external local use (cream examples 1 and 2) were prepared by the conventional method while mixing the following components.

[Cream Example 1]

Compound of Chemical Formula 1 (ex.: Compound 5 or 9) 0.1 wt%

White Petrolatum 40 wt%

Mineral oil 11 wt%

Polyoxylstearyl ether 8.5 wt%

Propyleneglycol 18 wt%

Butylhydroxyanisol 0.02 wt%

Purified water, appropriate amount (22.38 wt%)

[Cream Example 2]

Compound of Chemical Formula 1 (ex.: Compound 5 or 9) 0.01 to 3 wt%

White Petrolatum 30 to 75 wt%

Mineral oil 2 to 10 wt%

Polyoxylstearyl ether 0.3 to 10 wt%

Propyleneglycol 0.3 to 45 wt%

Butylhydroxyanisol 0.002 to 2.5 wt%

Paraben (ex.: Methylparaben or propylparaben) 0.01 to 3.5 wt%

Purified water, appropriate amount (2 to 30 wt%)

Preparative Example 9: Preparation of Beverage

The beverage was prepared to have a total volume of 100 mL by the conventional method while mixing purified water with the following components.

Compound of Chemical Formula 1 (ex.: Compound 5 or 9) 2 mg

Citric acid 9 g

White sugar 9 g

Glucose 2.8 g

DL-malic acid 0.25 g

Purified water, appropriate amount

Preparative Example 9: Preparation of Food

9-1. Manufacture of Seasoning for Cooking

Seasoning for cooling for health promotion was manufactured by using 0.001-0.2 parts by weight of Compound of Chemical Formula 1 (ex.: Compound 5 or 9).

9-2. Manufacture of Flour Based Food

Food for health promotion was manufactured by using the flour added with 0.001-0.2 parts by weight of Compound of Chemical Formula 1 (ex.: Compound 5 or 9) to make bread, cakes, cookies, crackers, and noodles.

9-3. Manufacture of Dairy Products

Various dairy products such as butter, ice cream, milk powder, and baby food, were manufactured by using milk added with 0.001-0.2 parts by weight of Compound of Chemical Formula 1 (ex.: Compound 5 or 9).

9-4. Manufacture of Juice

Juice for health promotion was manufactured by adding 0.001-0.2 parts by weight of Compound of Chemical Formula 1 (ex.: Compound 5 or 9) to 1,000 mℓ of juice of vegetable such as tomato or carrot or fruit such as apple, grape, orange, or pineapple.

Preparative Example 10: Preparation of Feedstuff for Animal

1 kg of feedstuff for animal was prepared by the conventional method while mixing the following components.

Compound of Chemical Formula 1 (ex.: Compound 5 or 9) 0.1 g

Casein 200 g

Corn starch 400 g

Dyestrose 130 g

Sugar 100 g

Cellulose 50 g

Soybean oil 70 g

Antioxidant (ex.: t-butylhydroquinone) 0.02 g

Inorganic substance (ex.: salt mix) 35 g

Vitamin (ex.: vitamin mix) 10 g

L-cystine 3 g

Choline bitartrate 2 g

### [Industrial Applicability]

According to the present invention, the PPAR δ agonist adjusts calcium ion during embryo genesis and an early fetal development period to increase slow muscle fiber and thus improve muscle endurance, thereby improving lipid and glucose metabolism and reprogramming the metabolism of the entire body, thus preventing/inhibiting the occurrence of metabolic diseases, such as obesity and diabetes in an adult or adult body caused by a high-fat diet and a lack of exercise, and improving memory. Therefore, the PPAR δ agonist can be used for a medicine composition, a nutritional supplement for pregnant women, a medicine composition for animals, an endurance enhancer for animals, a composition for dry milk and baby food, and a composition for animal feedstuff, for enhancing endurance of human beings and animals through fetal reprogramming, preventing/inhibiting metabolic diseases such as obesity, diabetes, arteriosclerosis and fatty liver, and improving memory.

## Claims

1. A composition for fetal reprogramming of a mammal, the composition containing a peroxisome proliferator activated receptor δ (PPAR δ) agonist as an effective component.

2. The composition of claim 1, wherein the composition enhances muscle endurance, prevents metabolic diseases, or improves memory of an object born or produced by a fetal reprogramming method.

3. The composition of claim 2, wherein the metabolic diseases are obesity, diabetes, hyperlipidemia, arteriosclerosis, or fatty acid.

4. The composition of claim 1, wherein the composition increases a birthrate of descendants or offspring, improves tolerance to illness, or lengthens lifespan.

5. The composition of claim 1, wherein the mammal is a human being.

6. The composition of claim 1, wherein the mammal excludes a human being.

7. The composition of claim 1, wherein the composition is administered to a mother body, or descendants or offspring thereof during a gestation period, a lactation period, or gestation and lactation periods.

8. The composition of any one of claims 1 to 7, wherein the PPAR δ agonist is represented by Chemical Formula I: 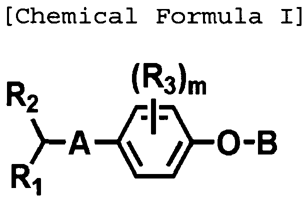 [In Chemical Formula I, A is oxygen (O), nitrogen (NH),
sulfur (S) or selenium (Se); B is 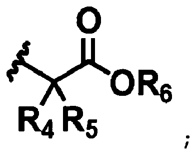 R₁ is selected from the following structures; 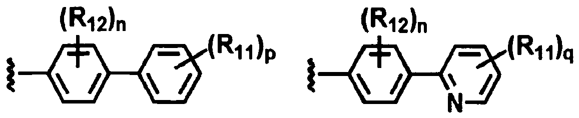 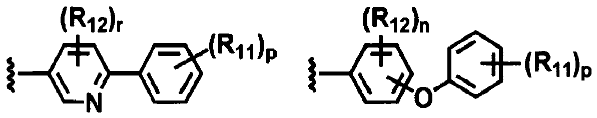 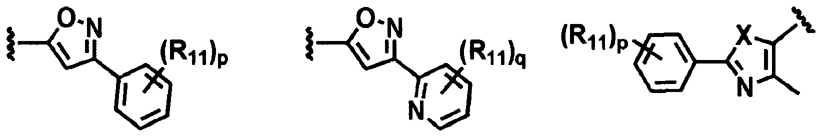 R₂ is selected from hydrogen, C₁-C₅ alkyl, 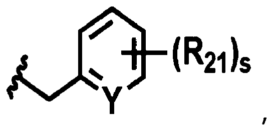 or the following structures; 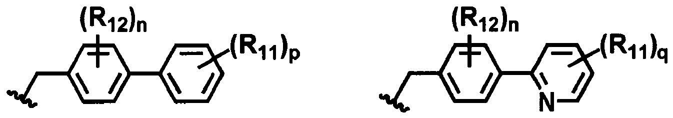 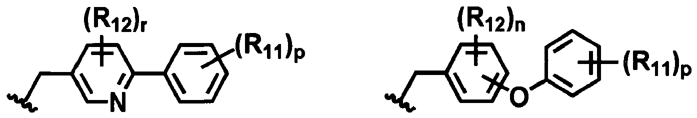 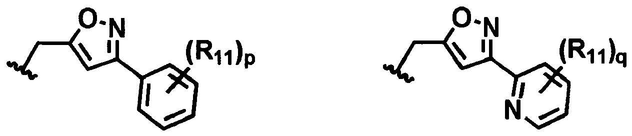 X is sulfur (S) or selenium (Se);
Y is carbon (CH) or nitrogen (N);
R₃ is hydrogen, C₁-C₈ alkyl, or halogen;
R₄ and R₅ each are independently hydrogen, halogen, or C₁-C₈ alkyl;
R₆ is hydrogen, C₁-C₈ alkyl, C₂-C₇ alkenyl, alkali metal, alkali earth metal, or organic acid;
R₁₁ and R₁₂ each are independently hydrogen, halogen, C₁-C₈ alkyl, or C₁-C₈ alkoxy;
R₂₁ is hydrogen, halogen, C₁-C₇ alkyl, a heterocyclic group, C₁-C₇ alkoxy, C₁-C₅ alkyl substituted with halogen, or phenyl substituted with halogen;
m and n each are independently an integer of 1~4;
p is an integer of 1~5;
q is an integer of 1~4;
r is an integer of 1~3;
s is an integer of 1~5; and
the alkyl and alkoxy of R₂, R₃, R₄, R₅, R₆, R₁₁, R₁₂ and R₂₁ may be further substituted with at least one halogen or C₁-C₅ alkylamine.]

9. The composition of claim 8, wherein the PPAR δ agonist is represented by Chemical Formula II: 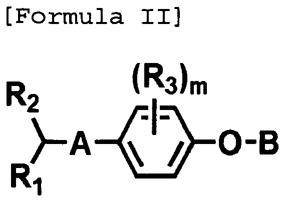 [In Chemical Formula I, A is sulfur (S) or selenium (Se); B is 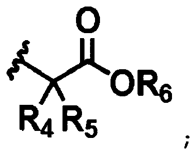 R₁ is selected from the following structures; 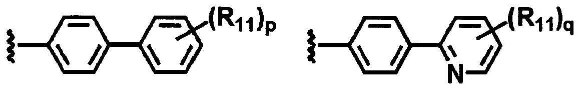 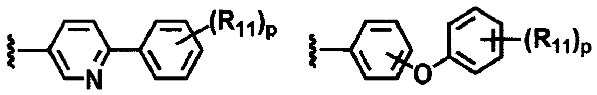 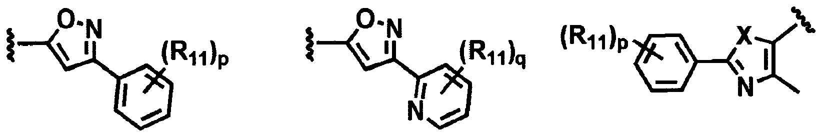 R₂ is selected from hydrogen, C₁-C₅ alkyl, 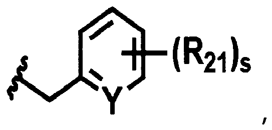 or the following structures; 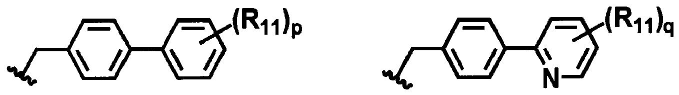 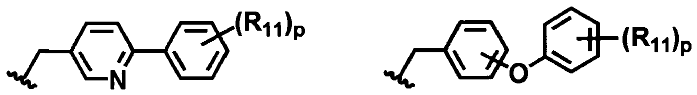 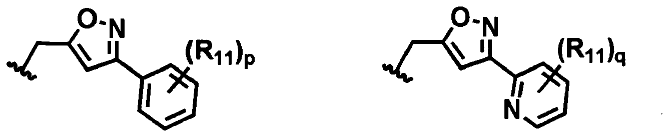 X is sulfur (S) or selenium (Se);
Y is carbon (CH) or nitrogen (N);
R₃ is hydrogen, C₁-C₅ alkyl, or halogen;
R₄ and R₅ each are independently hydrogen or C₁-C₅ alkyl;
R₆ is hydrogen, C₁-C₅ alkyl, C₂-C₅ alkenyl, alkali metal, or alkali earth metal;
R₁₁ is hydrogen, halogen, C₁-C₅ alkyl, or C₁-C₅ alkoxy;
R₂₁ is hydrogen, halogen, C₁-C₅ alkyl, a heterocyclic group, C₁-C₅ alkoxy, C₁-C₅ alkyl substituted with halogen, or phenyl substituted with halogen;
m is an integer of 1~4;
p is an integer of 1~5;
q is an integer of 1~4;
s is an integer of 1~5; and
the alkyl and alkoxy of R₂, R₃, R₄, R₅, R₆, R₁₁, and R₂₁ may be further substituted with at least one halogen or C₁-C₅ alkylamine.]

10. The composition of claim 8, wherein the PPAR δ agonist is represented by Chemical Formula III: 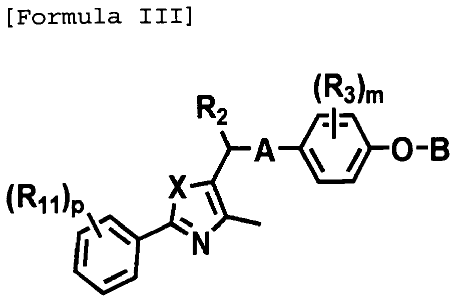 [In Chemical Formula III, A is independently sulfur (S) or
selenium (Se); B is 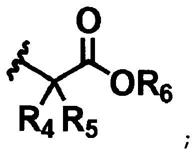 R₂ is selected from hydrogen, C₁-C₅ alkyl, 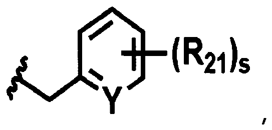 or the following structures; 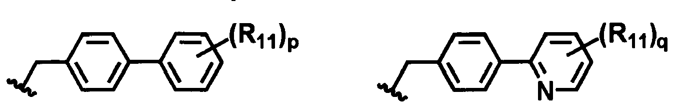 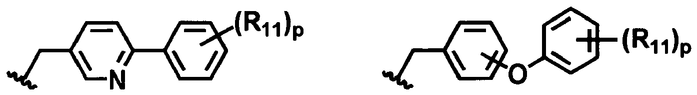 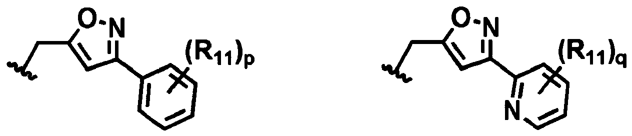 X is sulfur (S) or selenium (Se);
Y is carbon (CH) or nitrogen (N);
R₃ is hydrogen, C₁-C₅ alkyl, or halogen;
R₄ and R₅ each are independently hydrogen or C₁-C₅ alkyl;
R₆ is hydrogen, C₁-C₅ alkyl, C₂-C₅ alkenyl, alkali metal, or alkali earth metal;
R₁₁ is hydrogen, halogen, C₁-C₅ alkyl, or C₁-C₅ alkoxy;
R₂₁ is hydrogen, halogen, C₁-C₅ alkyl, a heterocyclic group, C₁-C₅ alkoxy, C₁-C₅ alkyl substituted with halogen, or phenyl substituted with halogen;
m is an integer of 1~4;
p is an integer of 1~5;
q is an integer of 1~4;
s is an integer of 1~5; and
the alkyl and alkoxy of R₂, R₃, R₄, R₅, R₆, R₁₁, and R₂₁ may be further substituted with at least one halogen or C₁-C₅ alkylamine.]

11. The composition of claim 8, wherein the PPAR δ agonist is represented by Chemical Formula IV: 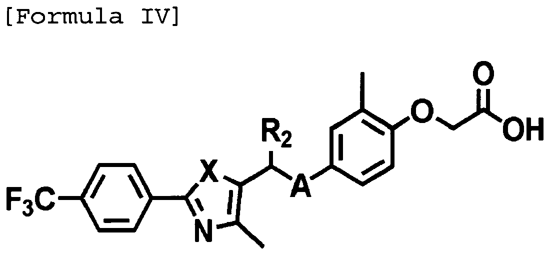 [In Chemical Formula IV, A is independently sulfur (S) or selenium (Se);
R₂ is selected from hydrogen, C₁-C₅ alkyl, 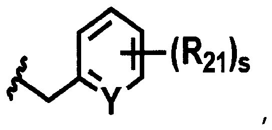 or the following structures; 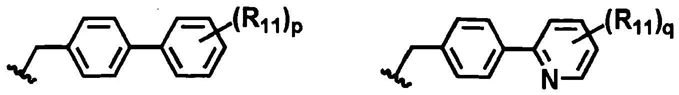 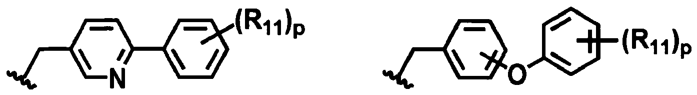 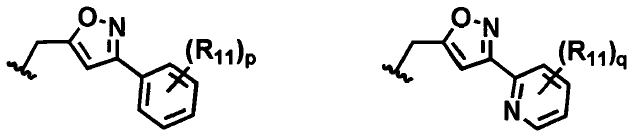 X is sulfur (S) or selenium (Se);
Y is carbon (CH) or nitrogen (N);
R₁₁ is hydrogen, halogen, C₁-C₅ alkyl, or C₁-C₅ alkoxy;
R₂₁ is hydrogen, halogen, C₁-C₅ alkyl, a heterocyclic group,
C₁-C₅ alkoxy, C₁-C₅ alkyl substituted with halogen, or phenyl substituted with halogen;
p is an integer of 1~5;
q is an integer of 1~4;
s is an integer of 1~5; and
the alkyl and alkoxy of R₁₁ and R₂₁ may be further substituted with at least one halogen or C₁-C₅ alkylamine.]

12. The composition of claim 8, wherein the PPAR δ agonist is represented by Chemical Formula V: 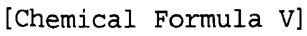 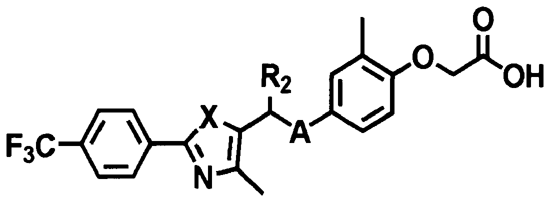 [In Chemical Formula V, A is independently sulfur (S) or selenium (Se);
R₂ is selected from the following structures; 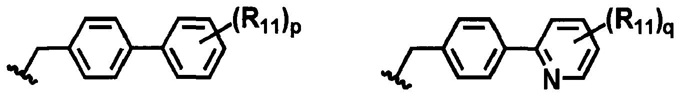 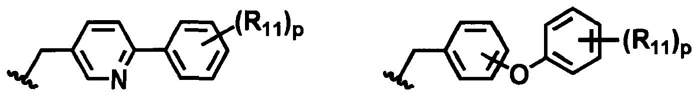 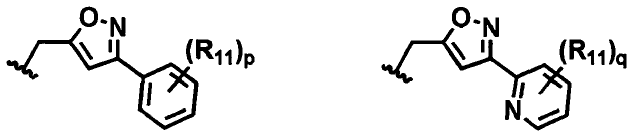 X is sulfur (S) or selenium (Se);
R₁₁ is hydrogen, halogen, C₁-C₅ alkyl, or C₁-C₅ alkoxy;
p is an integer of 1-5;
q is an integer of 1-4; and
the alkyl and alkoxy of R₁₁ may be further substituted with at least one halogen or C₁-C₅ alkylamine.]

13. The composition of claim 8, wherein the PPAR δ agonist is represented by Chemical Formula VI: 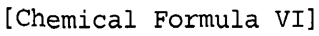 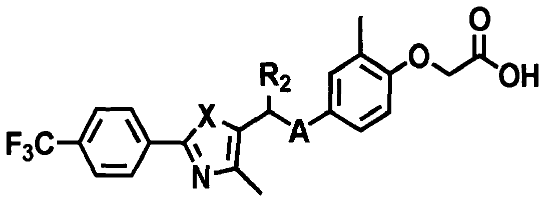 [In Chemical Formula VI, A is independently sulfur (S) or selenium (Se);
R₂ is selected from the following structures; 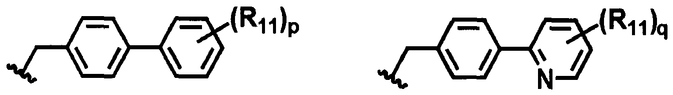 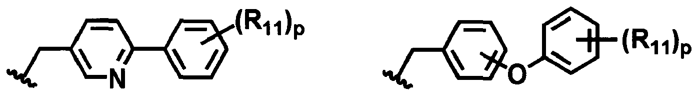 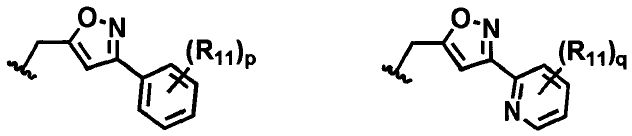 X is sulfur (S) or selenium (Se);
R₁₁ is hydrogen, C₁-C₃ alkyl or halogen;
p is an integer of 1~5;
q is an integer of 1~4; and
the alkyl of R₁₁ may be further substituted with at least one halogen or C₁-C₅ alkylamine.]

14. The composition of claim 8, wherein the PPAR δ agonist is selected from the following compounds:

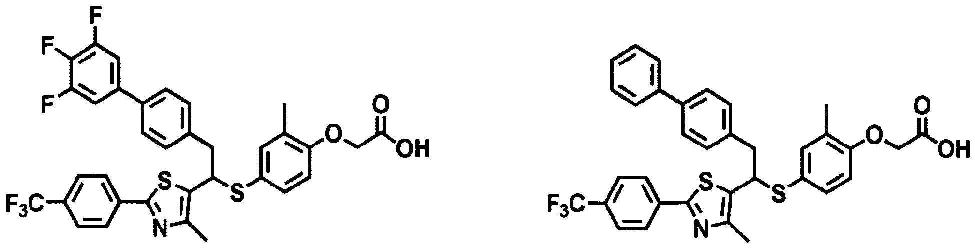

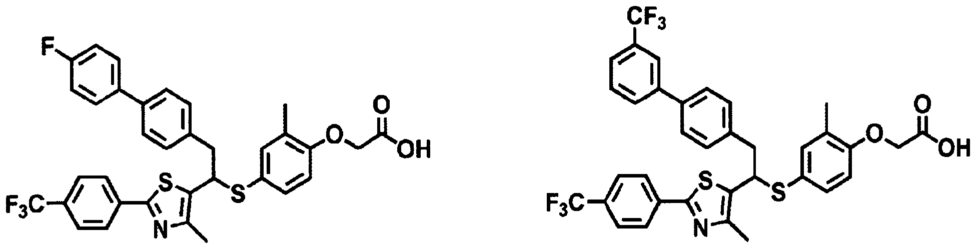

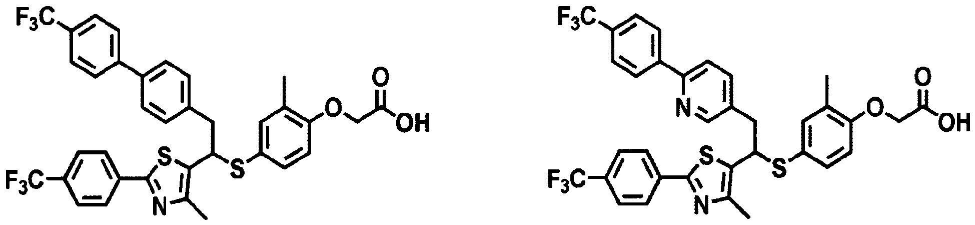

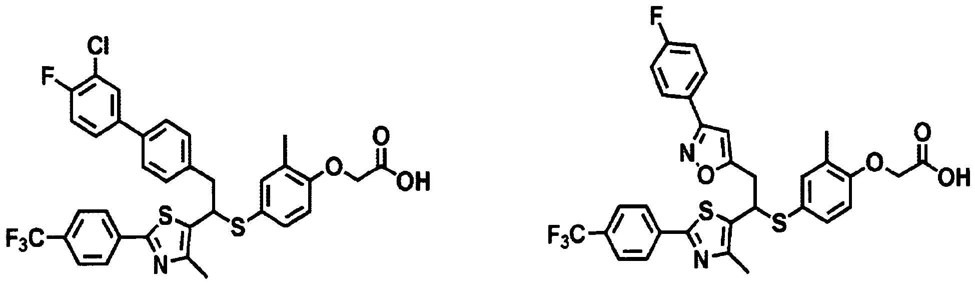

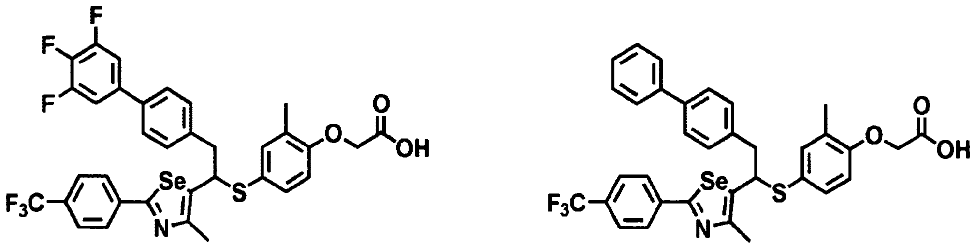

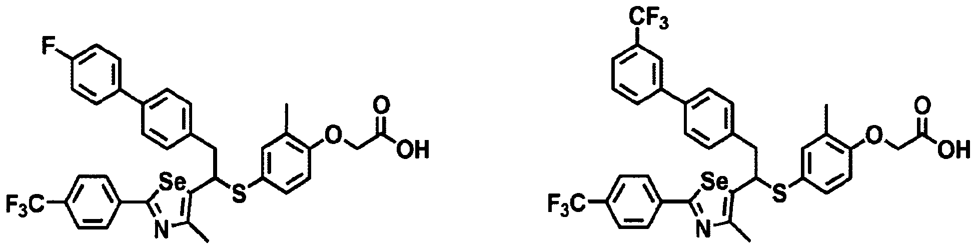

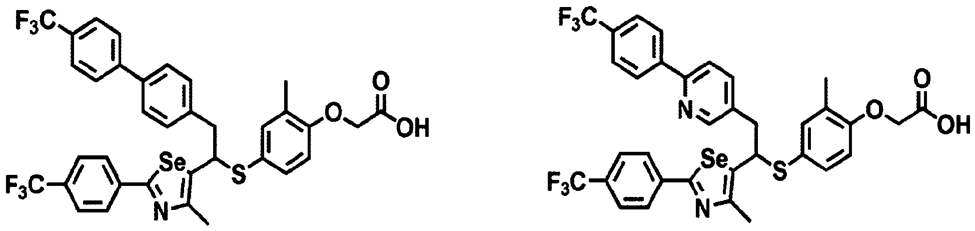

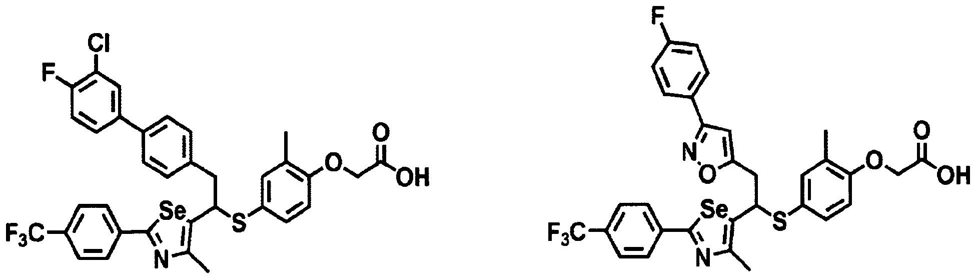

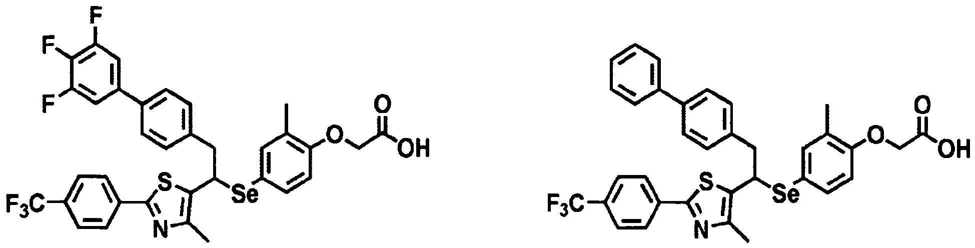

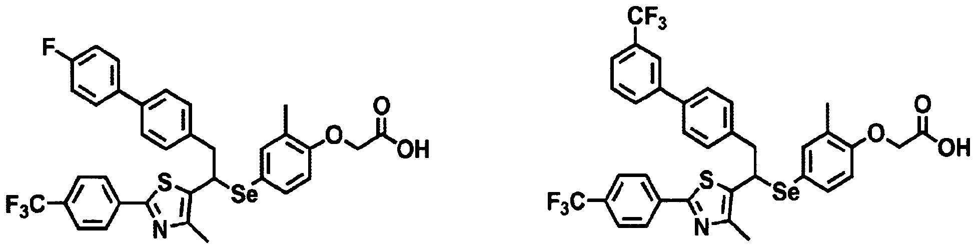

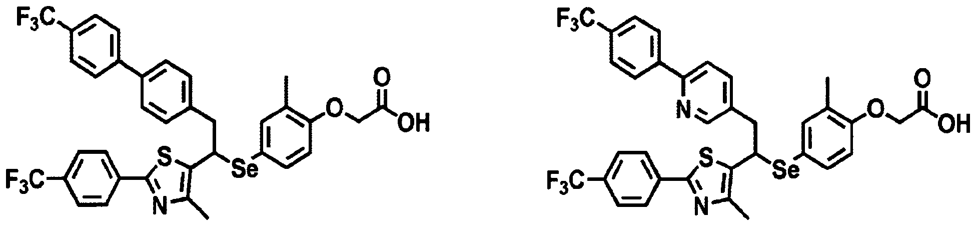

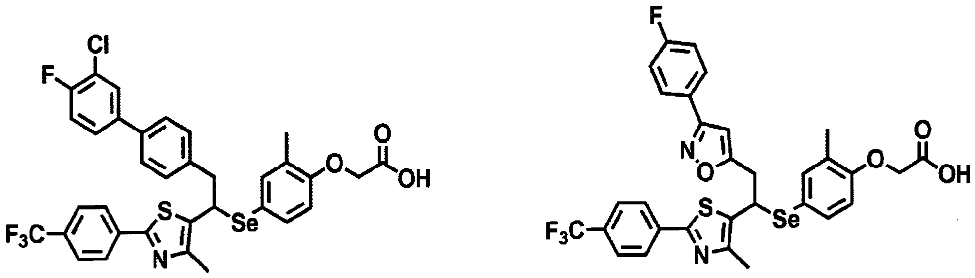

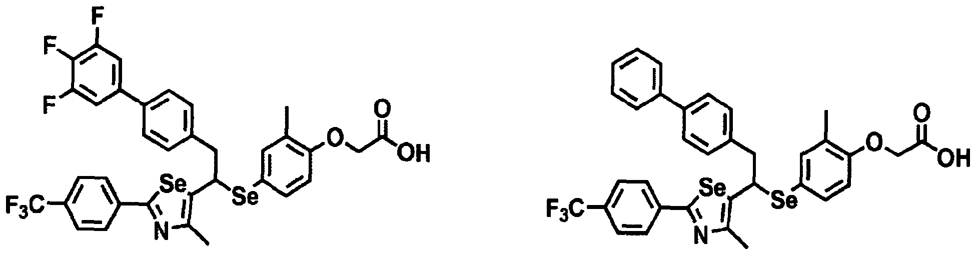

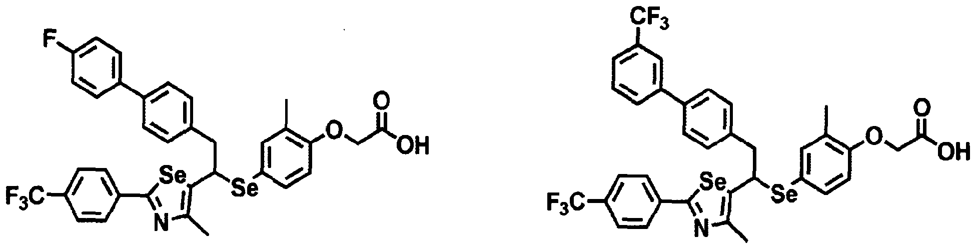

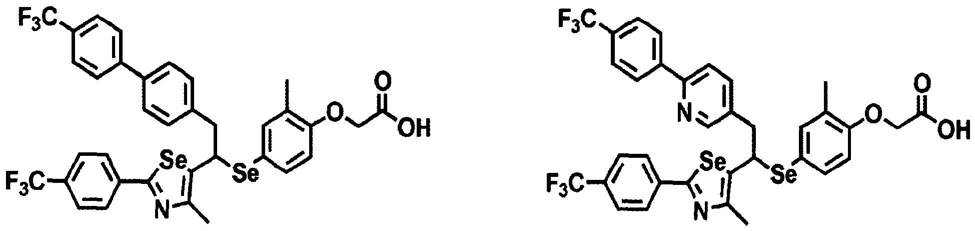

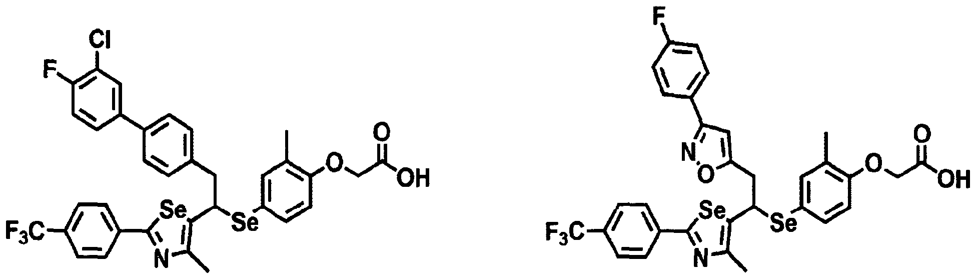

15. A derivative represented by Chemical Formula V: 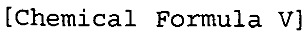 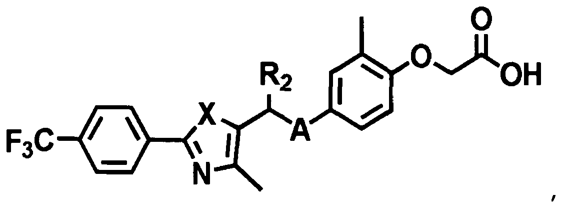 or hydrate, solvate, stereoisomer, or pharmaceutically acceptable salt thereof,
[In Chemical Formula V, A is independently sulfur (S) or selenium (Se);
R₂ is selected from the following structures; 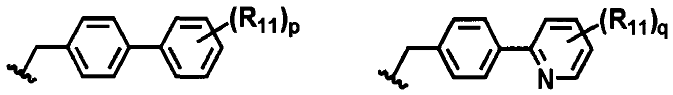 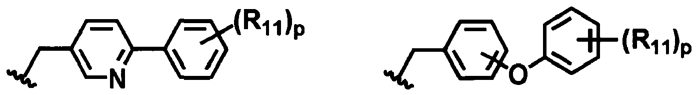 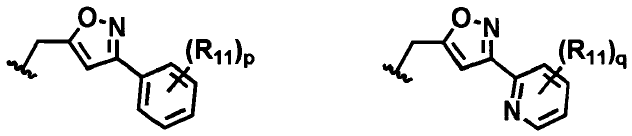 X is sulfur (S) or selenium (Se);
R₁₁ is hydrogen, halogen, C₁-C₅ alkyl, or C₁-C₅ alkoxy;
p is an integer of 1~5;
q is an integer of 1~4; and
the alkyl and alkoxy of R₁₁ may be further substituted with at least one halogen or C₁-C₅ alkylamine.]

16. The derivative of claim 15, represented by Chemical Formula VI: 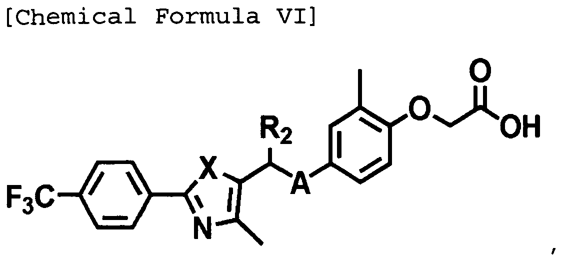 or hydrate, solvate, stereoisomer, or pharmaceutically acceptable salt thereof,
[In Chemical Formula VI, A is independently sulfur (S) or selenium (Se);
R₂ is selected from the following structures; 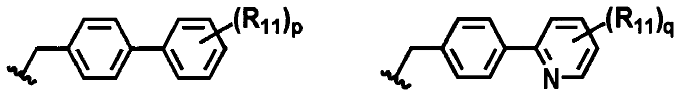 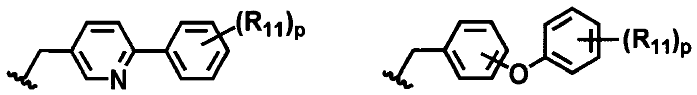 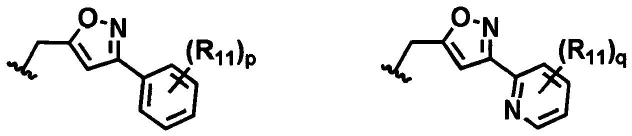 X is sulfur (S) or selenium (Se);
R₁₁ is hydrogen, C₁-C₃ alkyl, or halogen;
p is an integer of 1~5;
q is an integer of 1~4; and
the alkyl of R₁₁ may be further substituted with at least one halogen or C₁-C₅ alkylamine.]

17. The derivative of claim 16, selected from the following compounds: 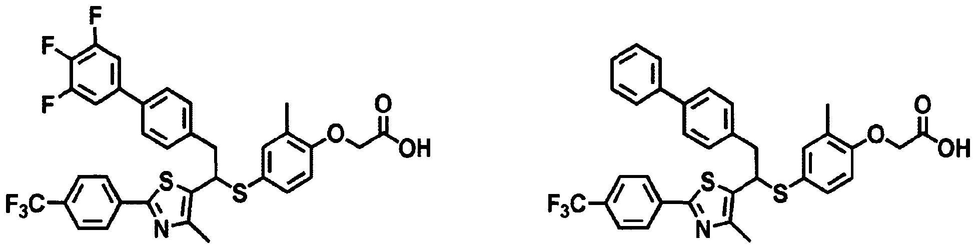 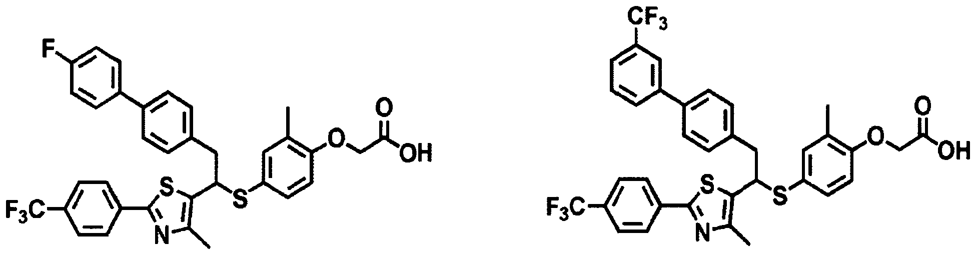 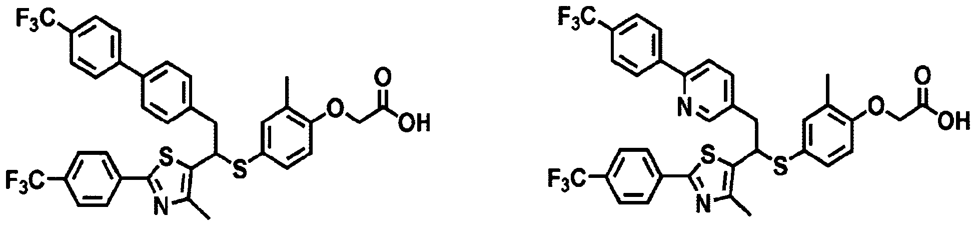 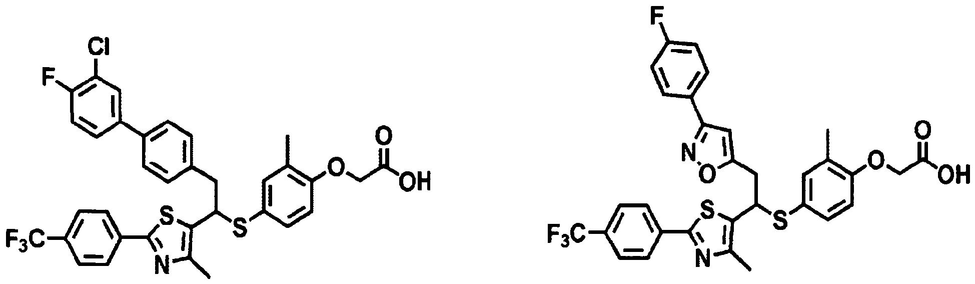 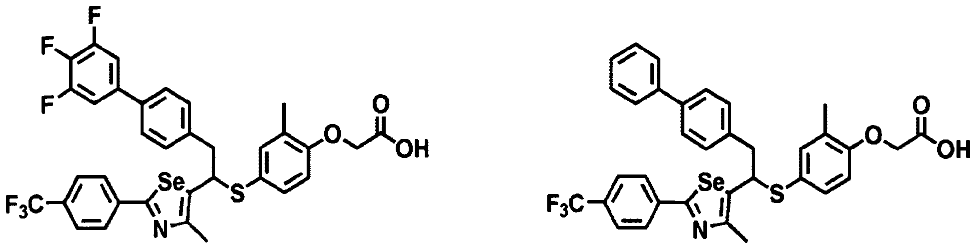 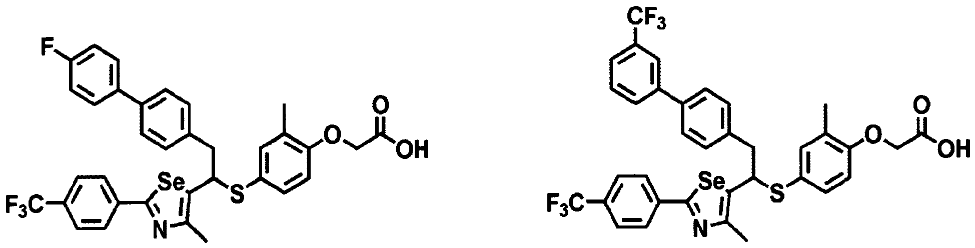 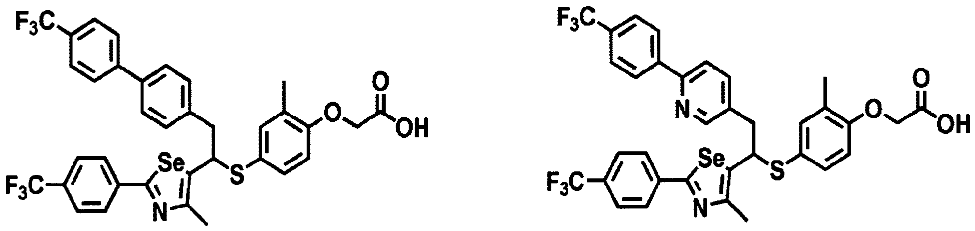 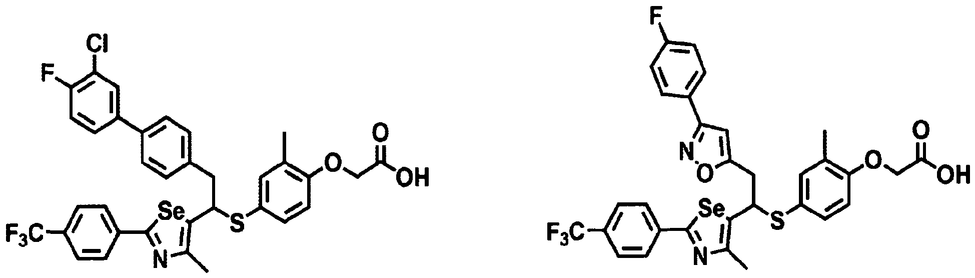 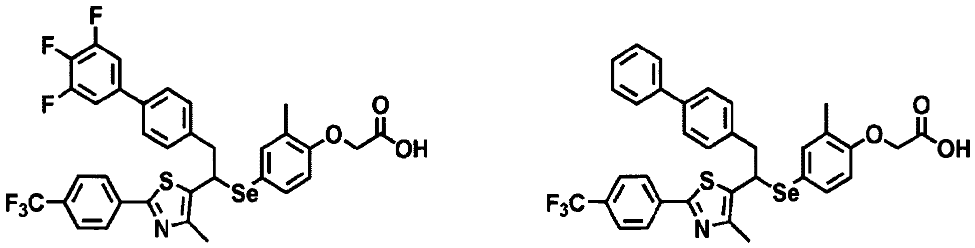 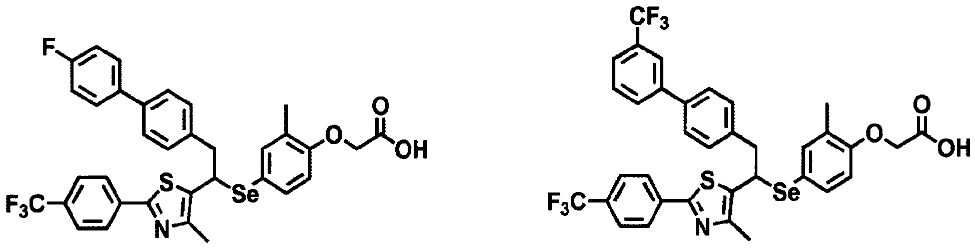 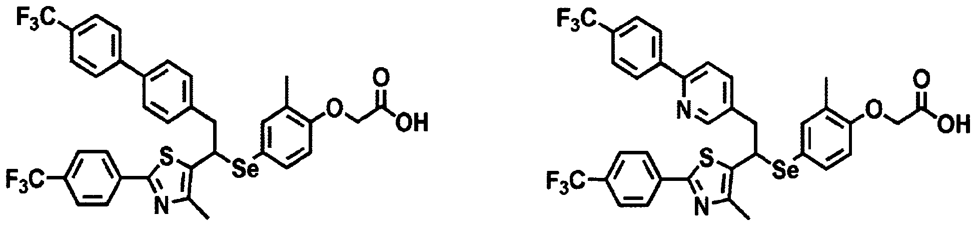 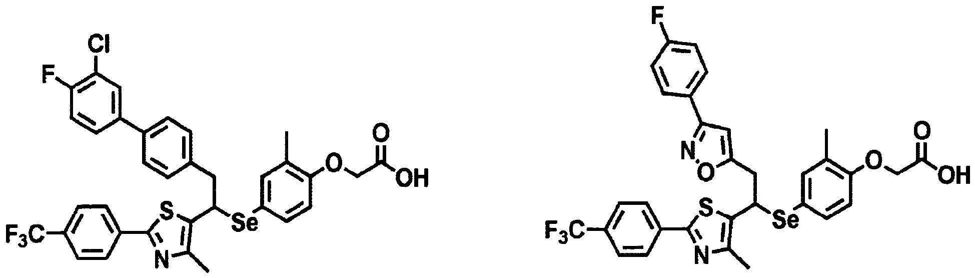 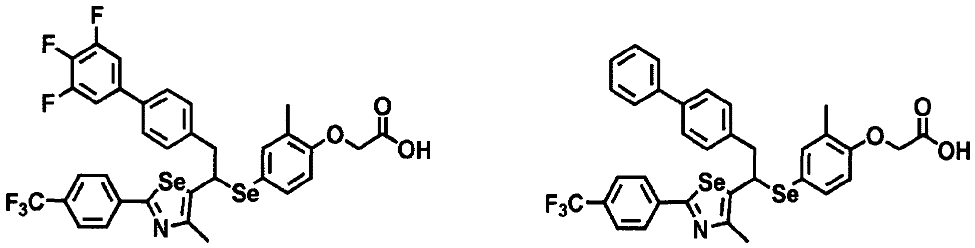 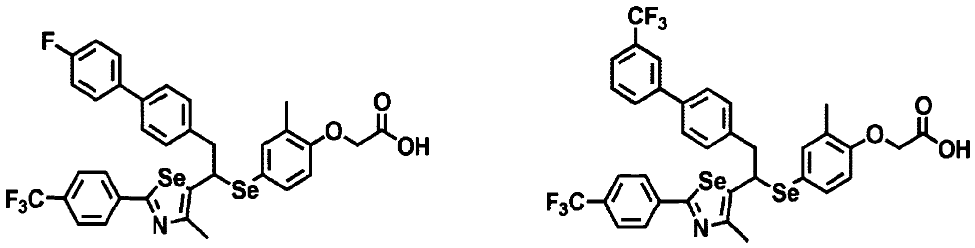 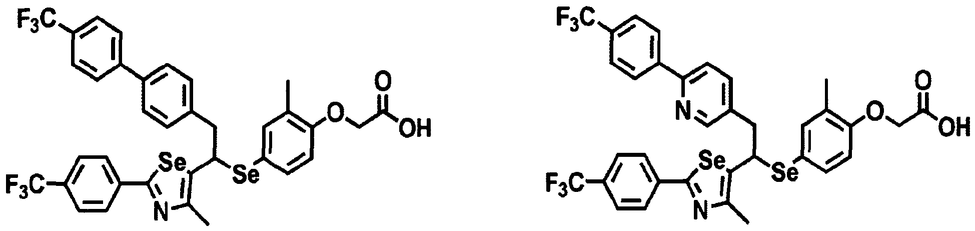 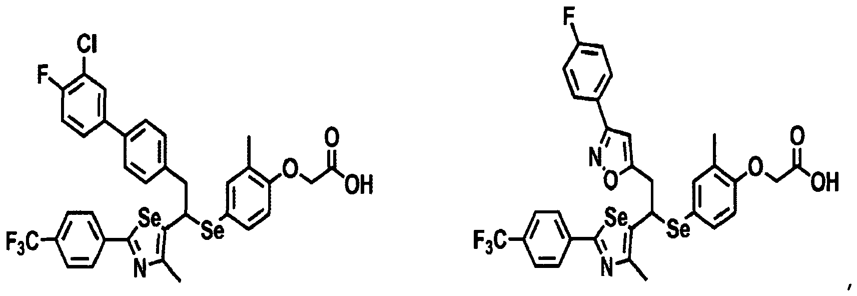 or hydrate, solvate, stereoisomer, or pharmaceutically acceptable salt thereof.

18. A composition for medicine for treating and preventing atherosclerosis or hyperlipidemia, treating and preventing hypercholesterolemia, treating and preventing fatty liver, treating and preventing diabetes, treating and preventing obesity, strengthening muscle, treating and preventing muscular diseases, enhancing endurance, improving memory, or treating and preventing dementia or Parkinson's disease, containing the derivative of Chemical Formula V of claim 15, or hydrate, solvate, stereoisomer, or pharmaceutically acceptable salt thereof, as an effective component.

19. A composition for a functional food supplement, a functional beverage, a food additive, and a feedstuff for animal, containing the derivative of Chemical Formula V of claim 15, or hydrate, solvate, stereoisomer, or pharmaceutically acceptable salt thereof, as an effective component.

20. A composition for functional cosmetics for preventing and improving obesity, preventing and improving fatty liver, strengthening muscle, preventing and improving muscular diseases, and enhancing endurance, containing the derivative of Chemical Formula V of claim 15, or hydrate, solvate, stereoisomer, or pharmaceutically acceptable salt thereof, as an effective component.

21. A fetal reprogramming method of a mammal excluding a human being, the method comprising administering a PPAR δ agonist to the mammal excluding the human being.

22. The method of claim 21, wherein the method enhances muscle endurance, prevents metabolic diseases, or improves memory of an object produced by the fetal reprogramming method.

23. The method of claim 22, wherein the metabolic diseases are obesity, diabetes, hyperlipidemia, arteriosclerosis, or fatty acid.

24. The method of claim 21, wherein the method increases a birthrate of descendants or offspring, improves tolerance to illness, or lengthens lifespan.

25. The method of claim 21, wherein a PPAR δ agonist is administered to mother or offspring thereof during a gestation period, a lactation period, or gestation and lactation periods.

26. The method of any one of claims 21 to 25, wherein the PPAR δ agonist is represented by Chemical Formula 1. 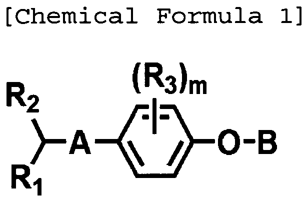 [In Chemical Formula I, A is oxygen (O), nitrogen (NH), sulfur (S) or selenium (Se); B is 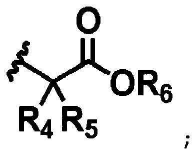 R₁ is selected from the following structures; 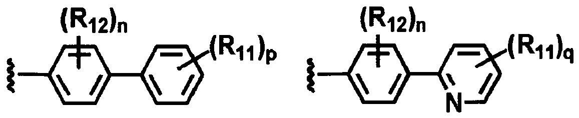 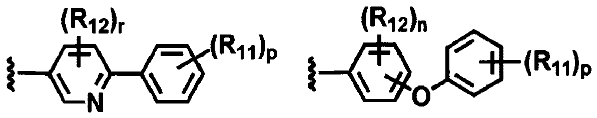 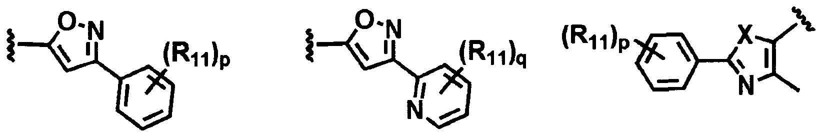 R₂ is selected from hydrogen, C₁-C₅ alkyl, 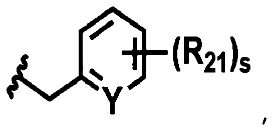 or the following structures: 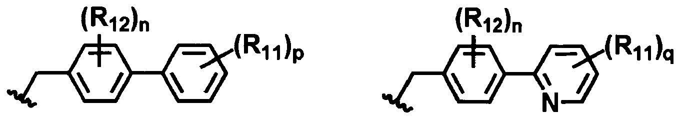 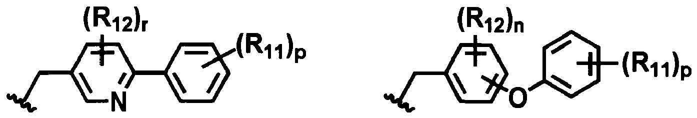 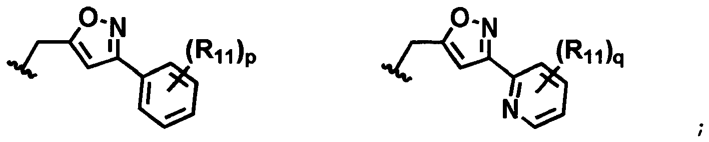 X is sulfur (S) or selenium (Se);
Y is carbon (CH) or nitrogen (N);
R₃ is hydrogen, C₁-C₈ alkyl, or halogen;
R₄ and R₅ each are independently hydrogen, halogen, or C₁-C₈ alkyl;
R₆ is hydrogen, C₁-C₈ alkyl, C₂-C₇ alkenyl, alkali metal, alkali earth metal, or organic acid;
R₁₁ and R₁₂ each are independently hydrogen, halogen, C₁-C₈ alkyl, or C₁-C₈ alkoxy;
R₂₁ is hydrogen, halogen, C₁-C₇ alkyl, a heterocyclic group, C₁-C₇ alkoxy, C₁-C₅ alkyl substituted with halogen, or phenyl substituted with halogen;
m and n each are independently an integer of 1~4;
p is an integer of 1~5;
q is an integer of 1~4;
r is an integer of 1~3;
s is an integer of 1~5; and
the alkyl and alkoxy of R₂, R₃, R₄, R₅, R₆, R₁₁, R₁₂ and R₂₁ may be further substituted with at least one halogen or C₁-C₅ alkylamine.]

27. The method of claim 26, wherein the PPAR δ agonist is represented by Chemical Formula II: 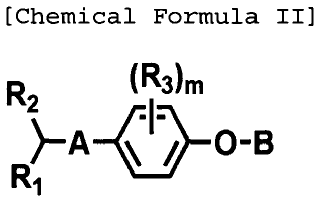 [In Chemical Formula I, A is sulfur (S) or selenium (Se); B is 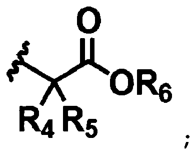 R₁ is selected from the following structures: 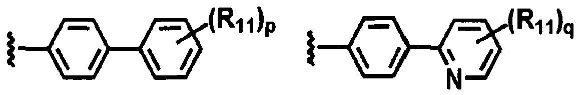 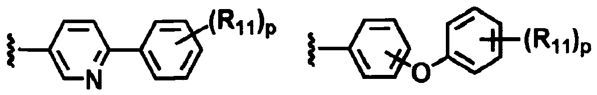 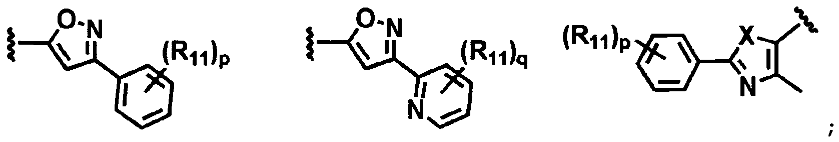 R₂ is selected from hydrogen, C₁-C₅ alkyl, 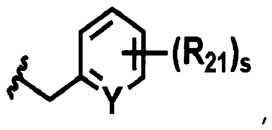 or the following structures: 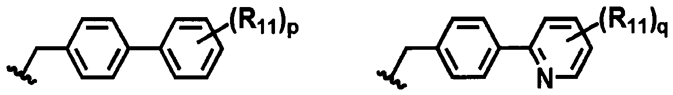 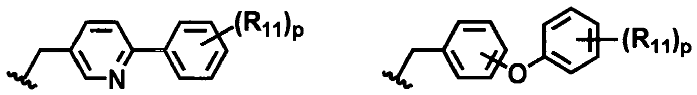 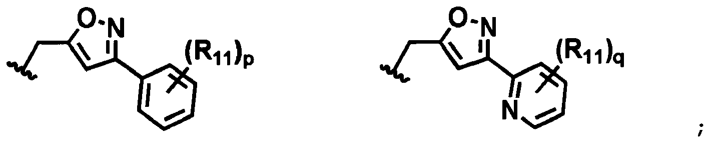 X is sulfur (S) or selenium (Se);
Y is carbon (CH) or nitrogen (N);
R₃ is hydrogen, C₁-C₅ alkyl, or halogen;
R₄ and R₅ each are independently hydrogen or C₁-C₅ alkyl;
R₆ is hydrogen, C₁-C₅ alkyl, C₂-C₅ alkenyl, alkali metal, or alkali earth metal;
R₁₁ is hydrogen, halogen, C₁-C₅ alkyl, or C₁-C₅ alkoxy;
R₂₁ is hydrogen, halogen, C₁-C₅ alkyl, a heterocyclic group, C₁-C₅ alkoxy, C₁-C₅ alkyl substituted with halogen, or phenyl substituted with halogen;
m is an integer of 1~4;
p is an integer of 1~5;
q is an integer of 1~4;
s is an integer of 1~5; and
the alkyl and alkoxy of R₂, R₃, R₄, R₅, R₆, R₁₁, and R₂₁ may be further substituted with at least one halogen or C₁-C₅ alkylamine.]

28. The method of claim 26, wherein the PPAR δ agonist is represented by Chemical Formula III: 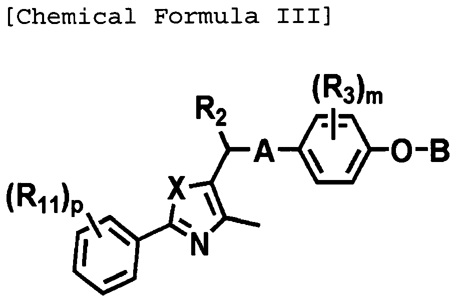 [In Chemical Formula III, A is independently sulfur (S) or
selenium (Se); B is 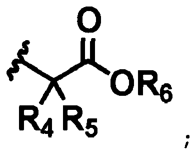 R₂ is selected from hydrogen, C₁-C₅ alkyl, 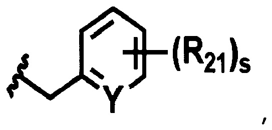 or the following structures: 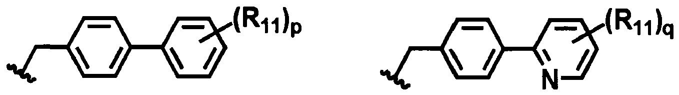 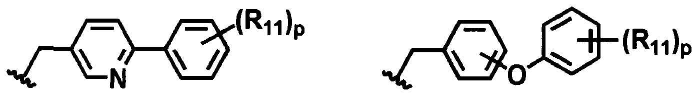 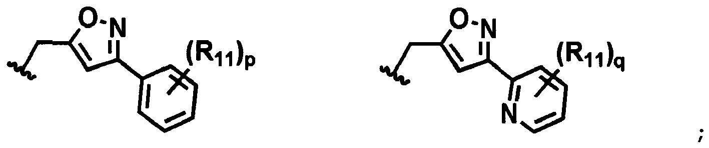 X is sulfur (S) or selenium (Se);
Y is carbon (CH) or nitrogen (N);
R₃ is hydrogen, C₁-C₅ alkyl, or halogen;
R₄ and R₅ each are independently hydrogen or C₁-C₅ alkyl;
R₆ is hydrogen, C₁-C₅ alkyl, C₂-C₅ alkenyl, alkali metal, or alkali earth metal;
R₁₁ is hydrogen, halogen, C₁-C₅ alkyl, or C₁-C₅ alkoxy;
R₂₁ is hydrogen, halogen, C₁-C₅ alkyl, a heterocyclic group, C₁-C₅ alkoxy, C₁-C₅ alkyl substituted with halogen, or phenyl substituted with halogen;
m is an integer of 1~4;
p is an integer of 1~5;
q is an integer of 1~4;
s is an integer of 1~5; and
the alkyl and alkoxy of R₂, R₃, R₄, R₅, R₆, R₁₁, and R₂₁ may be further substituted with at least one halogen or C₁-C₅ alkylamine.]

29. The method of claim 26, wherein the PPAR δ agonist is represented by Chemical Formula IV: 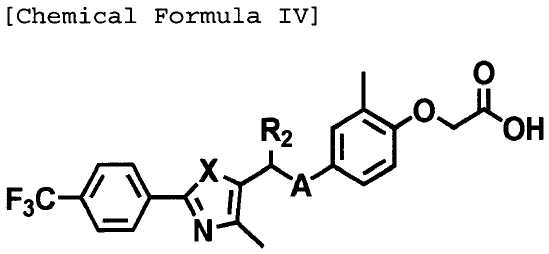 [In Chemical Formula IV, A is independently sulfur (S) or selenium (Se);
R₂ is selected from hydrogen, C₁-C₅ alkyl, 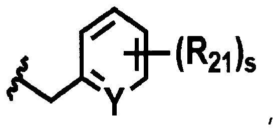 or the following structures; 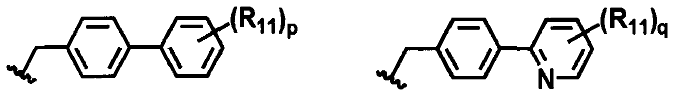 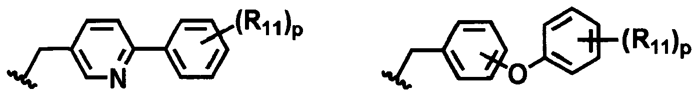 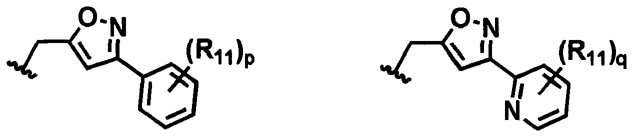 X is sulfur (S) or selenium (Se);
Y is carbon (CH) or nitrogen (N);
R₁₁ is hydrogen, halogen, C₁-C₅ alkyl, or C₁-C₅ alkoxy;
R₂₁ is hydrogen, halogen, C₁-C₅ alkyl, a heterocyclic group, C₁-C₅ alkoxy, C₁-C₅ alkyl substituted with halogen, or phenyl substituted with halogen;
p is an integer of 1~5;
q is an integer of 1~4;
s is an integer of 1~5; and
the alkyl and alkoxy of R₁₁ and R₂₁ may be further substituted with at least one halogen or C₁-C₅ alkylamine.]

30. The method of claim 26, wherein the PPAR δ agonist is represented by Chemical Formula V: 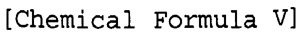 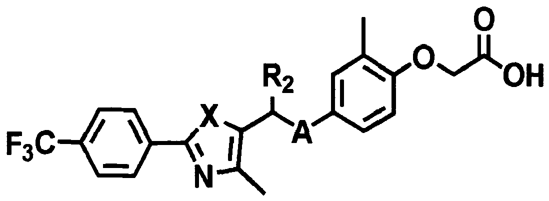 [In Chemical Formula V, A is independently sulfur (S) or selenium (Se);
R₂ is selected from the following structures: 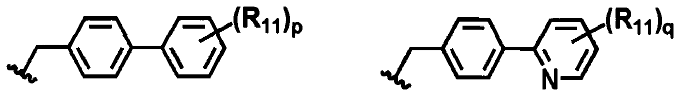 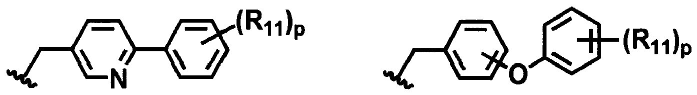 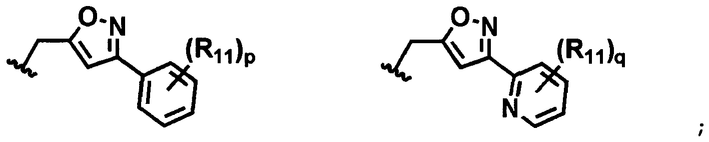 X is sulfur (S) or selenium (Se);
R₁₁ is hydrogen, halogen, C₁-C₅ alkyl, or C₁-C₅ alkoxy;
p is an integer of 1~5;
q is an integer of 1~4;
the alkyl and alkoxy of R₁₁ may be further substituted with at least one halogen or C₁-C₅ alkylamine.]

31. The method of claim 26, wherein the PPAR δ agonist is represented by Chemical Formula VI: 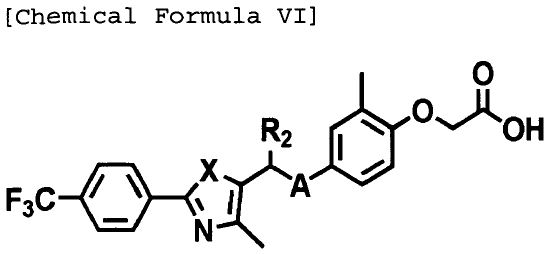 [In Chemical Formula VI, A is independently sulfur (S) or selenium (Se);
R₂ is selected from the following structures: 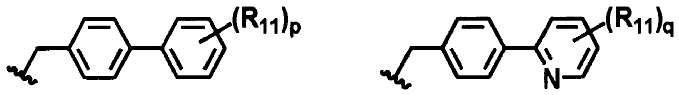 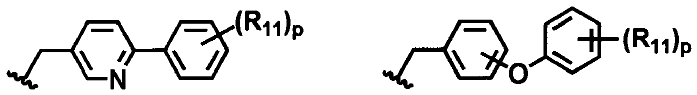 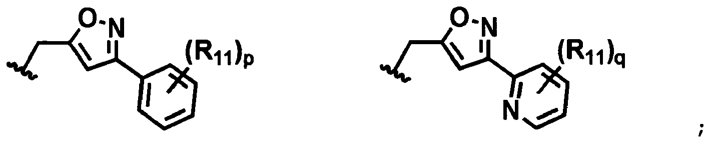 X is sulfur (S) or selenium (Se);
R₁₁ is hydrogen, C₁-C₃ alkyl or halogen;
p is an integer of 1-5;
q is an integer of 1-4; and
the alkyl of R₁₁ may be further substituted with at least one halogen or C₁-C₅ alkylamine.]

32. The method of claim 26, wherein the PPAR δ agonist is selected from the following compounds:

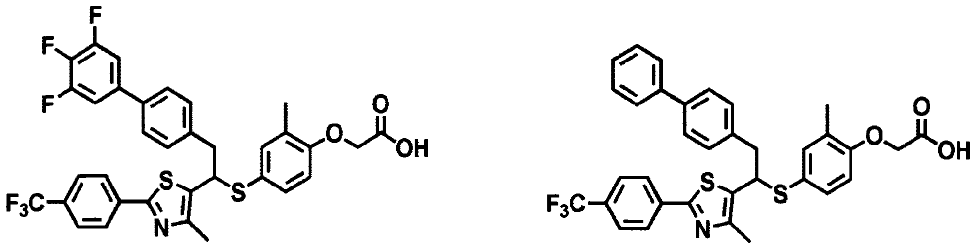

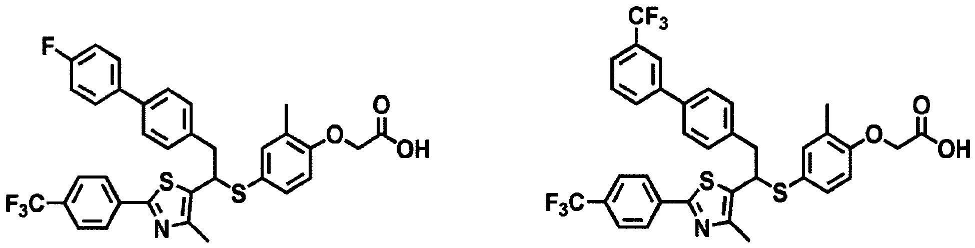

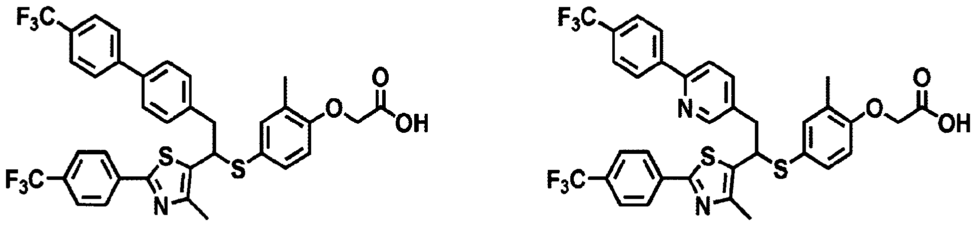

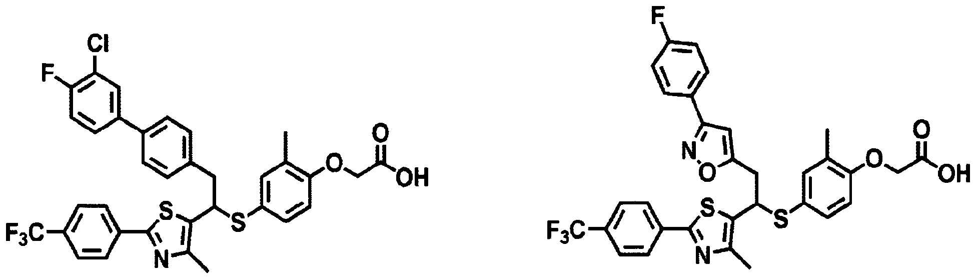

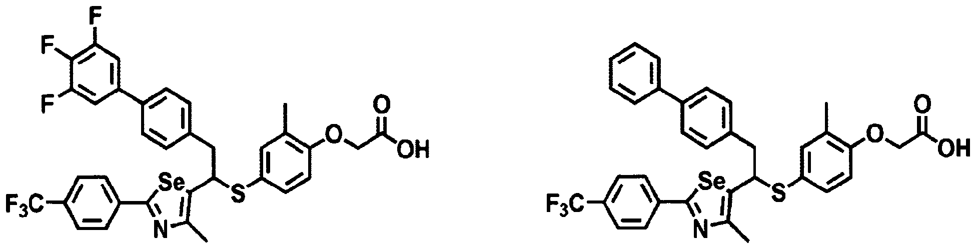

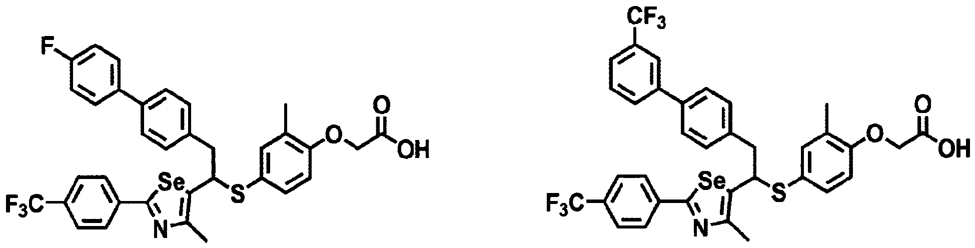

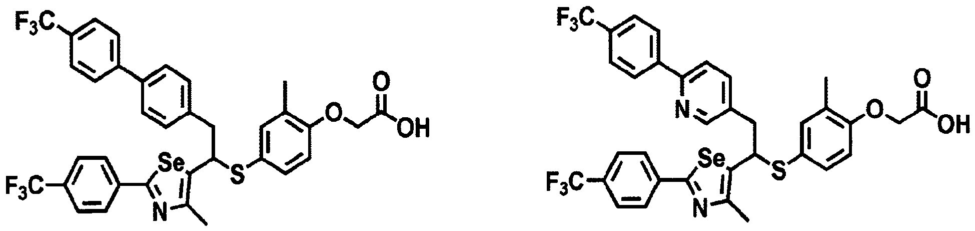

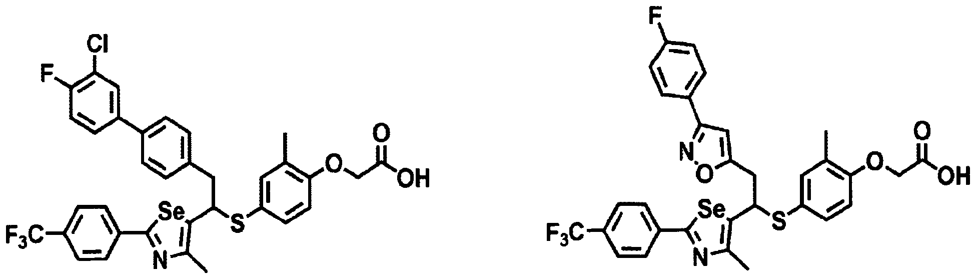

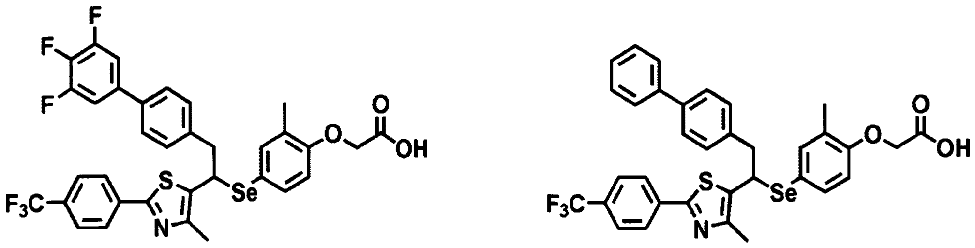

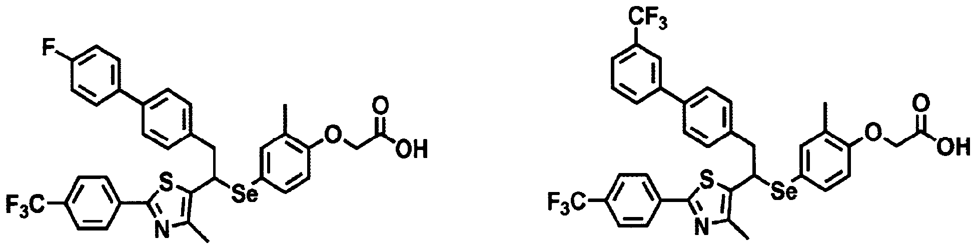

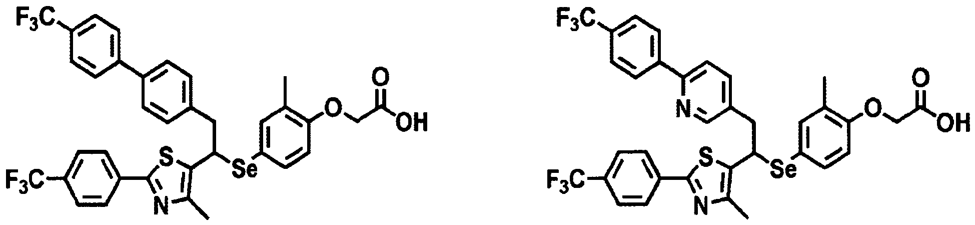

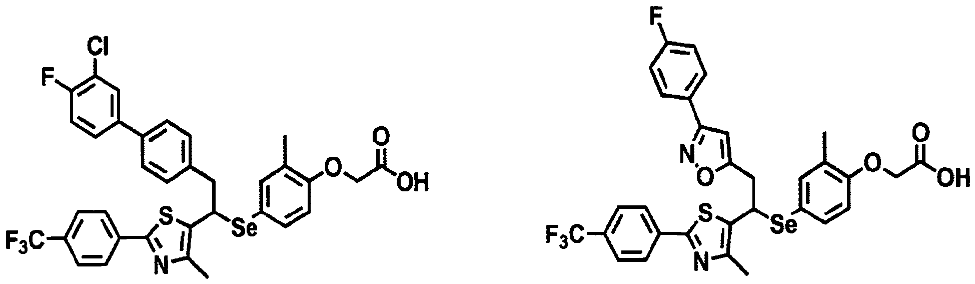

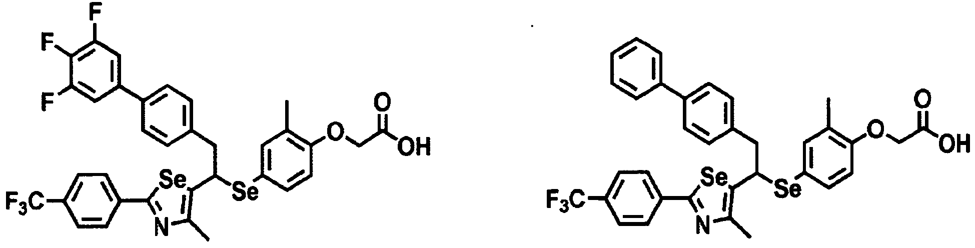

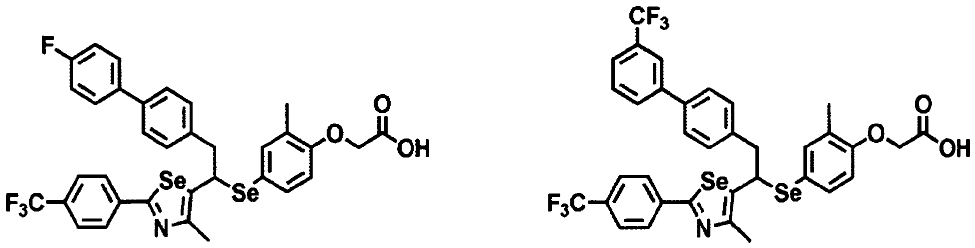

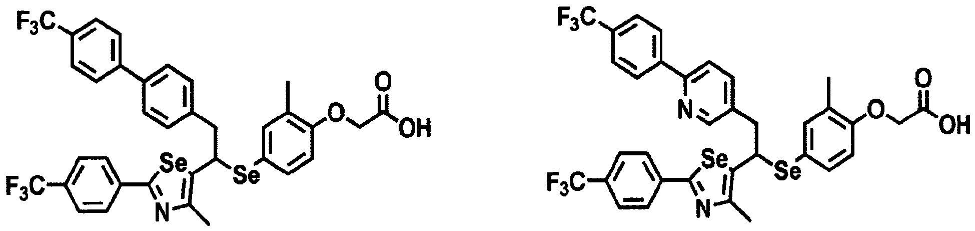

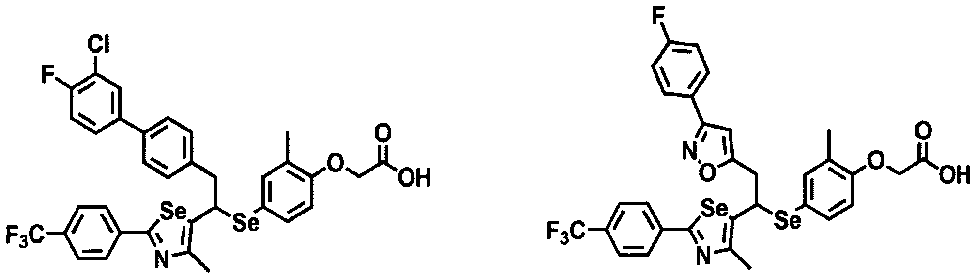

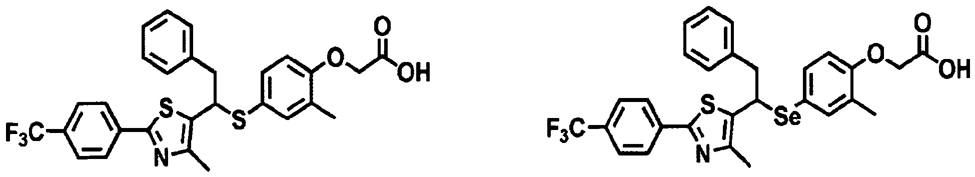

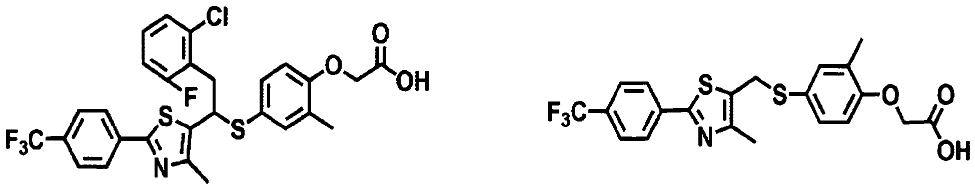

33. A composition for a food additive, a functional food supplement, or a functional beverage, for enhancing muscle endurance, preventing metabolic diseases, or improving memory, of an object produced by a fetal reprogramming method of a mammal including a human being, the composition containing a PPAR δ agonist.

34. The composition of claim 33, wherein the metabolic diseases are obesity, diabetes, hyperlipidemia, arteriosclerosis, or fatty acid.

35. The composition of claim 33, wherein the composition is administered to mammals or descendants or offspring thereof during a gestation period, a lactation period, or gestation and lactation periods.

36. A composition for feedstuff for enhancing muscle endurance, preventing metabolic diseases, or improving memory, of an object produced by a fetal reprogramming method of a mammal, the composition containing a PPAR δ agonist as an additive.

37. The composition of claim 36, wherein the metabolic diseases are obesity, diabetes, hyperlipidemia, arteriosclerosis, or fatty acid.

38. The composition of claim 36, wherein the composition is administered to the mammal or offspring thereof during a gestation period, a lactation period, or gestation and lactation periods.

39. A composition for dry milk or baby food for enhancing muscle endurance, preventing metabolic diseases, or improving memory, of an object produced by a fetal reprogramming method of a mammal, the composition containing a PPAR δ agonist as an additive.

40. The composition of claim 39, wherein the metabolic diseases are obesity, diabetes, hyperlipidemia, arteriosclerosis, or fatty acid.

41. The composition of claim 39, wherein the mammal is a human being.

42. The composition of claim 39, wherein the mammal excludes a human being.

43. An endurance enhancer for an object produced by a fetal reprogramming method of a mammal including a human being, containing a PPAR δ agonist as an effective component.

44. An endurance enhancer of an object produced by a fetal reprogramming method of a mammal excluding a human being, containing a PPAR δ agonist as an effective component.

45. A use of fetal reprogramming of a PPAR δ agonist.

46. A use of a PPAR δ agonist for enhancing muscle endurance, preventing metabolic diseases including obesity, diabetes, hyperlipidemia, arteriosclerosis, and fatty acid, and improving memory, by regulation of fetal programming of a mammal including a human being.

47. A use of a PPAR δ agonist for enhancing muscle endurance, preventing and treating metabolic diseases including obesity, diabetes, hyperlipidemia, arteriosclerosis, and fatty acid, and improving memory, through human fetal reprogramming.

48. A use of a PPAR δ agonist for preventing and treating metabolic diseases including obesity, diabetes, hyperlipidemia, arteriosclerosis, and fatty acid, and improving memory, through animal fetal reprogramming.

49. A use of a PPAR δ agonist for enhancing muscle endurance through animal fetal reprogramming.
